# EUROPÄISCHE PATENTANMELDUNG

(11) **EP 1 127 880 A2**
(43) Veröffentlichungstag der Anmeldung: **29.08.2001**
(21) Anmeldenummer: 01110080.7
(22) Anmeldetag: 13.04.1992
(51) Int. Cl.: C07D 233/68, A61K 31/395, C07D 235/16, C07D 235/08, C07D 233/90, C07D 233/54, C07D 409/06, C07D 471/04, C07D 403/10, C07D 249/18, C07D 231/56, C07D 231/12, C07D 231/14

(54) **Heterocyclisch substituierte Phenylessigsäurederivate und deren Verwendung als A-II-Antagonisten und als Inhibitoren der Proliferation von glatten Muskelzellen**

(30) Priorität: 26.04.1991 DE 4113693; 16.01.1992 DE 4200954
(62) Teilanmeldung aus: 92106345.9
(71) Anmelder: BAYER AG, 51368 Leverkusen (DE)
(72) Erfinder: Müller, Ulrich, Dr., 42111 Wuppertal (DE); Mohrs, Klaus, Dr., 42113 Wuppertal (DE); Dressel, Jürgen, Dr., 42477 Radevormwald (DE); Hanko, Rudolf, Dr., 40239 Düsseldorf (DE); Hübsch, Walter, Dr., 42113 Wuppertal (DE); Matzke, Michael, Dr., 42113 Wuppertal (DE); Niehwöhner, Ulrich, Dr., 42929 Wermelskirchen (DE); Raddatz, Siegfried, Dr., 51065 Köln (DE); Krämer, Thomas, Dr., 42111 Wuppertal (DE); Müller-Gliemann, Matthias, Dr., 42719 Solingen (DE); Bellemann, Hans-Peter, Dr., 42113 Wuppertal (DE); Beuck, Martin, Dr., 40699 Erkrath (DE); Kazda, Stanislav, Prof. Dr., 42115 Wuppertal (DE); Wohlfeil, Stefan, Dr., 40721 Hilden (DE)

(57) **Zusammenfassung**

Die Erfindung betrifft Phenylessigsäurederivate, die durch einen fünfgliedrigen, über ein N-Atom gebundenen Stickstoffheterocyclus substituiert sind. Sie werden durch Umsetzung von mit Abgangsgruppen substituierten Phenylessigsäurederivaten mit den entsprechenden Stickstoffheterocyclen hergestellt und können als Wirkstoffe in Arzneimitteln zur Behandlung von Bluthochdruck und Artherosklerose verwendet werden.

## Beschreibung

Die Erfindung betrifft neue heterocyclisch substituierte Phenylessigsäurederivate, Verfahren zu ihrer Herstellung, sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksenkenden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des symphathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 102, EP 399 731; EP 399 732 und EP 324 347 heterocyclische Verbindungen mit A II-antagonistischer Wirkung bekannt.

Die Erfindung betrifft heterocyclisch substituierte Phenylessigsäurederivate der allgemeinen Formel (I) 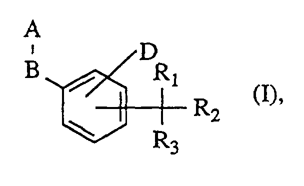 in welcher
- A: für einen über ein Stickstoffatom gebundenen, heterocyclischen Rest der Formel 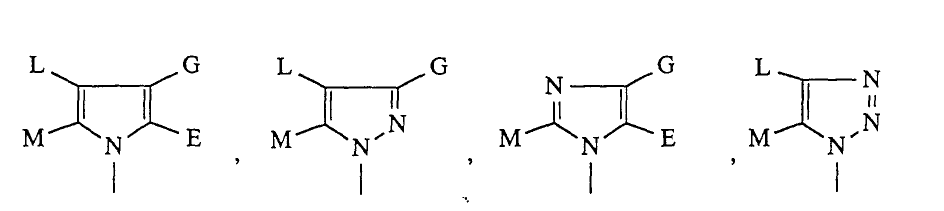 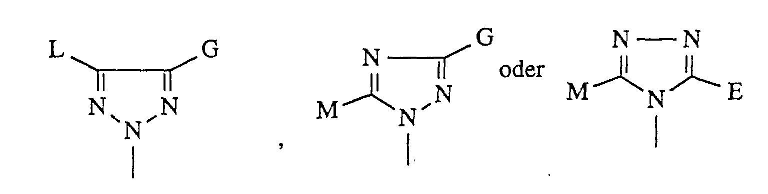 steht, worin
E, G, L und M gleich oder verschieden sind und Cycloalkyl mit 3 bis 8 Kohlenstoffatomen, Wasserstoff, Tetrazolyl, Halogen, Perfluoralkyl mit bis zu 6 Kohlenstoffatomen oder eine Gruppe der Formel 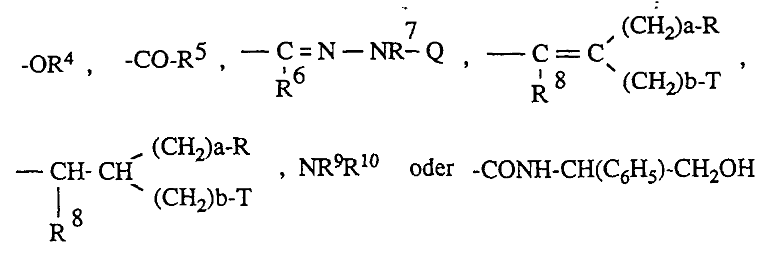 bedeuten,
worin
R⁴ Wasserstoff, geradkettiges oder verzweigtes Alkyl oder Acyl mit bis zu 8 Kohlenstoffatomen oder Phenyl bedeutet, das seinerseits bis zu 2fach gleich oder verschieden durch Halogen, Hydroxy, Nitro oder durch geradkettiges oder verzweigtes Alkyl oder Alkoxy mit jeweils bis zu 8 Kohlenstoffatomen substituiert ist, oder eine Hydroxyschutzgruppe bedeutet,
R⁵ Wasserstoff, Hydroxy oder geradkettiges oder verzweigtes Alkyl oder Alkoxy mit jeweils bis zu 8 Kohlenstoffatomen oder eine Gruppe der Formel -NR⁹R¹⁰ oder -NR⁹-SO₂R¹¹ bedeutet,
worin
R⁹ und R¹⁰ gleich oder verschieden sind und Wasserstoff, geradkettiges oder verzweigtes Alkyl oder Acyl mit jeweils bis zu 8 Kohlenstoffatomen oder Phenyl bedeuten, das gegebenenfalls durch Nitro, Cyano, bedeuten, das gegebenenfalls durch Nitro, Cyano, Halogen, Trifluormethyl oder durch geradkettiges oder verzweigtes Alkyl mit bis zu 6 Kohlenstoffatomen substituiert ist,
R¹¹ geradkettiges oder verzweigtes Alkyl mit bis zu 8 Kohlenstoffatomen, Benzyl, 2-Phenylvinyl oder Phenyl bedeutet, das gegebenenfalls bis zu 2fach gleich oder verschieden durch Halogen, Trifluormethyl oder durch geradkettiges oder verzweigtes Alkyl oder Alkoxy mit jeweils bis zu 6 Kohlenstoffatomen substituiert ist,
R⁶, R⁷ und R⁸ gleich oder verschieden sind und Wasserstoff, Hydroxy, Acetyloxy, geradkettiges oder verzweigtes Alkyl mit bis zu 8 Kohlenstoffatomen oder Phenyl bedeuten,
Q eine Gruppe der Formel -C ≡ N , -CO-NR¹²R¹³, -SO₂-NR¹⁴R¹⁵ oder -CO-NR¹⁶-SO₂R¹⁷ bedeutet,
worin
R¹², R¹³, R¹⁴, R¹⁵ und R¹⁶ gleich oder verschieden sind und die oben angegebene Bedeutung von R⁹ und R¹⁰ haben und mit dieser gleich oder verschieden sind,
R¹⁷ die oben angegebene Bedeutung von R¹¹ hat und mit dieser gleich oder verschieden ist,
a und b gleich oder verschieden sind und eine Zahl 0, 1, 2, 3 oder 4 bedeuten,
R Wasserstoff oder Phenyl, Thienyl oder Furyl bedeutet, die Carboxy, geradkettiges oder verzweigtes Alkyl, Alkoxy oder Alkoxycarbonyl mit jeweils bis zu 8 Kohlenstoffatomen, Nitro, Hydroxy, Halogen, Trifluormethyl oder Trifluormethoxy substituiert sind,
T Wasserstoff oder eine Gruppe der Formel -OR^{4'} oder -CO-R^{5'} bedeutet,
worin
R^{4'} und R^{5'} die oben angegebene Bedeutung von R⁴ und R⁵ haben und mit dieser gleich oder verschieden sind, oder
E, G, L und M geradkettiges oder verzweigtes Alkyl, Alkenyl oder Alkadienyl mit jeweils bis zu 10 Kohlenstoffatomen bedeuten, die gegebenenfalls bis zu 2fach durch Halogen oder durch einen 5- bis 7-gliedrigen, gesättigen oder ungesättigten heterocyclischen Ring mit bis zu 4-Heteroatomen aus der Reihe N, S oder O, durch Phenyl, Cycloalkyl mit 3 bis 8 Kohlenstoffatomen oder durch eine der Gruppen 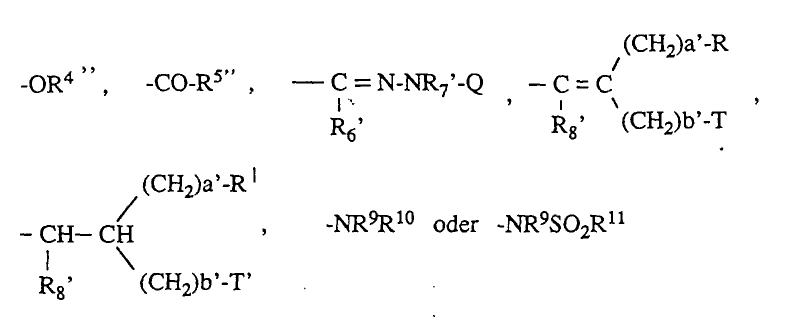 substituiert sind,
worin
R^{4"}, R^{5"}, R^{6'}, R^{7'}, R^{8'}, a', b' R' und T' die oben angegebene Bedeutung von R⁴, R⁵, R⁶, R⁷, R⁸, a, b, R und T haben und dieser gleich oder verschieden sind, und
R⁹, R¹⁰ und R¹¹ die oben angegebene Bedeutung haben oder
jeweils E und G, G und L oder L und M gemeinsam mit dem Heterocyclus einen an diesen ankondensierten 5- bis 7-gliedrigen, gesättigten, partiell ungesättigten oder aromatischen carbocyclischen oder heterocyclischen Ring, letzteren gegebenenfalls mit bis zu 4 Heteroatomen aus der Reihe N, S oder O bilden, wobei diese ankondensierten Ringe gegebenenfalls bis zu 3fach gleich oder verschieden durch Halogen oder durch eine Gruppe der Formel-V-W oder W substituiert sind,
worin
V ein geradkettiges oder verzweigtes Alkylen mit bis zu 6 Kohlenstoffatomen bedeutet,
W Wasserstoff oder eine Gruppe der Formel -OR^{4"},-CO-R^{5"} oder -NR^{9'}R^{10'} bedeutet,
worin
R^{4"} und R^{5"} die oben angegebene Bedeutung haben, und
R^{9'} und R^{10'} die oben angegebene Bedeutung von R⁹ und R¹⁰ haben und mit dieser gleich oder verschieden sind,
- B: für unverzweigtes Alkyl, Alkenyl oder Alkinyl mit jeweils bis zu 4 Kohlenstoffatomen steht,
- D: für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano, Carboxy oder für die Gruppe -NR⁹R¹⁰ steht,
worin
R⁹ und R¹⁰ die oben angegebene Bedeutung haben,
- R¹ und R²: gleich oder verschieden sind und
für Wasserstoff, Hydroxy oder für geradkettiges oder verzweigtes Alkyl mit bis zu 10 Kohlenstoffatomen stehen, das gegebenenfalls durch Halogen, Cycloalkyl mit 3 bis 8 Kohlenstoffatomen oder Phenyl substituiert ist, das seinerseits durch Halogen, Nitro, Hydroxy oder durch geradkettiges oder verzweigtes Alkyl oder Alkoxy mit jeweils bis zu 8 Kohlenstoffatomen substituiert sein kann,
für Cycloalkyl oder Cycloalkenyl mit 3 bis 12 Kohlenstoffatomen oder Phenyl stehen, die gegebenenfalls durch Halogen, oder durch geradkettiges oder verzweigtes Alkyl oder Alkoxy mit jeweils bis zu 6 Kohlenstoffatomen substituiert sind,
oder
- R¹ und R²: gemeinsam mit dem Kohlenstoffatom einen 3- bis 7-gliedrigen, gesättigten oder ungesättigten Carbocyclus bilden, der gegebenenfalls bis zu 2fach gleich oder verschieden durch geradkettiges oder verzweigtes Alkyl oder Alkoxy mit jeweils bis zu 6 Kohlenstoffatomen, Phenyl, Hydroxy oder Halogen substituiert ist,
- R³: für Tetrazolyl steht, oder
für eine Gruppe der Formel -C ≡ N, -CO-R¹⁸, -CO-NR¹⁹R²⁰, -CO-NR²¹-SO₂-R²² oder -SO₂-NR²³R²⁴ steht,
worin
R¹⁸ Hydroxy, geradkettiges oder verzweigtes Alkoxy mit bis zu 10 Kohlenstoffatomen oder Phenoxy bedeutet,
R¹⁹ und R²⁰ gleich oder verschieden sind, die oben angegebene Bedeutung von R⁹ und R¹⁰ haben und mit dieser gleich oder verschieden sind, oder
R¹⁹ die oben angegebene Bedeutung hat und
R²⁰ eine Gruppe der Formel 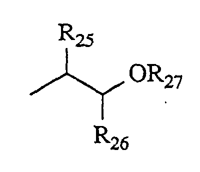 bedeutet,
worin
R²⁵ und R²⁶ gleich oder verschieden sind und Wasserstoff, Phenyl, Benzyl oder einen 5- bis 7-gliedrigen, gesättigten oder ungesättigten Heterocyclus mit jeweils bis zu 3 Heteroatomen aus der Reihe N, S oder O bedeutet, die ihrerseits einfach oder zweifach durch Halogen, Hydroxy, Nitro, Cyano, Hydroxy, Carboxy oder durch geradkettiges oder verzweigtes Alkyl, Alkoxy oder Alkoxycarbonyl mit jeweils bis zu 8 Kohlenstoffatomen substituiert sein können,
R²⁷ Wasserstoff oder geradkettiges oder verzweigtes Alkyl oder Acyl mit jeweils bis zu 8 Kohlenstoffatomen bedeutet,
R²¹, R²³ und R²⁴ gleich oder verschieden sind und die oben angegebene Bedeutung von R⁹ und R¹⁰ haben und mit diesen gleich oder verschieden sind, und
R²² die oben angegebene Bedeutung von R¹¹ hat und mit dieser gleich oder verschieden ist
oder einen Rest der Formel 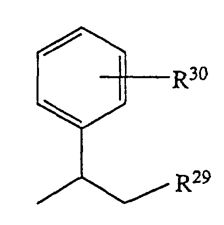 bedeutet,
worin
R²⁹ Hydroxy, Carboxy oder geradkettiges oder verzweigtes Alkoxycarbonyl mit bis zu 6 Kohlenstoffatomen bedeutet, und
R³⁰ Wasserstoff oder Halogen bedeutet,
und deren Salze.

Die erfindungsgemäßen heterocyclisch substituierten Phenylessigsäurederivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der heterocyclisch substituierten Phenylessigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff- , Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Bevorzugt sind Verbindungen der allgemeinen Formel (I),
in welcher
- A: für einen über ein Stickstoffatom gebundenen heterocyclischen Rest der Formel 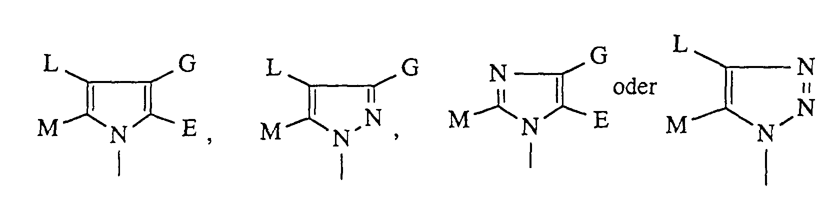 steht,
worin
E, G, L und M gleich oder verschieden sind und Wasserstoff, Tetrazolyl, Cyclopropyl, Cyclopentyl, Cyclohexyl, Fluor, Chlor, Brom, Jod, Perfluoralkyl mit bis zu 4 Kohlenstoffatomen oder eine Gruppe der Formel 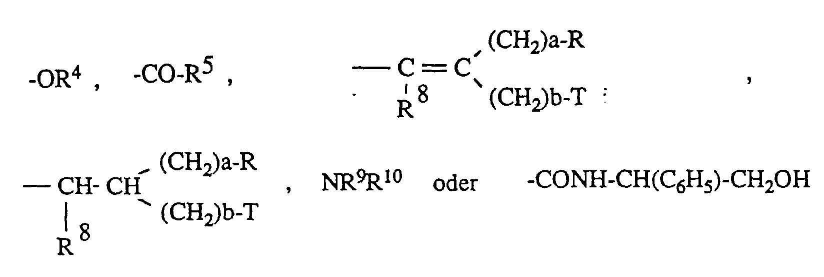 bedeuten,
worin
R⁴ Wasserstoff, geradkettiges oder verzweigtes Alkyl oder Acyl mit jeweils bis zu 6 Kohlenstoffatomen oder Phenyl bedeutet, das seinerseits durch Fluor, Chlor, Brom, Hydroxy, Nitro oder durch geradkettiges oder verzweigtes Alkyl oder Alkoxy mit jeweils bis zu 6 Kohlenstoffatomen substituiert ist, oder Methoxyethoxymethyl bedeutet,
R⁵ Wasserstoff, Hydroxy oder geradkettiges oder verzweigtes Alkyl oder Alkoxy mit jeweils bis zu 6 Kohlenstoffatomen oder eine Gruppe der Formel -NR⁹R¹⁰ oder -NR⁹-SO₂R¹¹ bedeutet,
worin
R⁹ und R¹⁰ gleich oder verschieden sind und Wasserstoff, geradkettiges oder verzweigtes Alkyl oder Acyl mit jeweils bis zu 6 Kohlenstoffatomen oder Phenyl bedeuten, das gegebenenfalls durch Nitro, Trifluormethyl, Fluor, Chlor, Brom oder durch geradkettiges oder verzweigtes Alkyl mit bis zu 4 Kohlenstoffatomen substituiert ist,
R¹¹ geradkettiges oder verzweigtes Alkyl mit bis zu 6 Kohlenstoffatomen, Benzyl, 2-Phenylvinyl oder Phenyl bedeutet, das gegebenenfalls durch Fluor, Chlor, Brom, Trifluormethyl oder durch geradkettiges oder verzweigtes Alkyl oder Alkoxy mit jeweils bis zu 4 Kohlenstoffatomen substituiert ist,
R⁸ Wasserstoff, Hydroxy, Acetyloxy, geradkettiges oder verzweigtes Alkyl mit bis zu 6 Kohlenstoffatomen oder Phenyl bedeutet,
a und b gleich oder verschieden sind und eine Zahl 0, 1, 2 oder 3 bedeuten,
R Wasserstoff oder Phenyl, Thienyl oder Furyl bedeutet, die gegebenenfalls bis zu 2fach gleich oder verschieden durch Carboxy, geradkettiges oder verzweigtes Alkyl, Alkoxy oder Alkoxycarbonyl mit jeweils bis zu 6 Kohlenstoffatomen, Nitro, Hydroxy, Fluor, Chlor, Brom, Trifluormethyl oder Trifluormethoxy substituiert sind,
T Wasserstoff oder eine Gruppe der Formel -OR^{4'} oder -CO-R^{5'} bedeutet,
worin
R^{4'} und R^{5'} die oben angegebene Bedeutung von R⁴ und R⁵ haben und mit dieser gleich oder verschieden ist, oder
E, G, L und M geradkettiges oder verzweigtes Alkyl, Alkenyl oder Alkadienyl mit jeweils bis zu 8 Kohlenstoffatomen bedeuten, die gegebenenfalls bis zu 2fach durch Fluor, Brom, Chlor, Phenyl, Tetrazolyl, Cyclopropyl, Cyclopentyl, Cyclohexyl oder durch eine der Gruppen 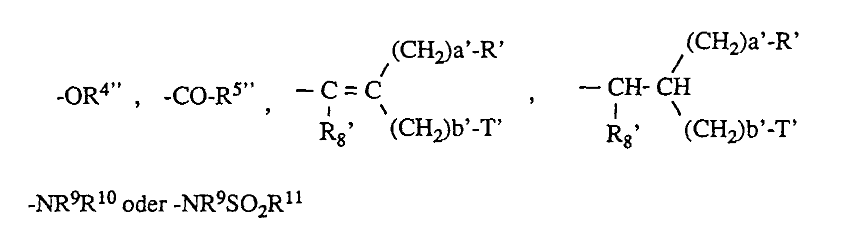 substituiert sind,
worin
R^{4"}, R^{5"}, R^{8'}, a', b', R' und T' die oben angegebene Bedeutung von R⁴, R⁵, R⁸, a, b, R und T haben und mit dieser gleich oder verschieden sind, und
R⁹, R¹⁰ und R¹¹ die oben angegebene Bedeutung haben, oder
jeweils E und G, G und L oder L und M gemeinsam mit dem Heterocyclus einen an diesen ankondensierten Cyclopentyl-, Cyclopentenyl-, Cyclohexyl-, Cyclohexenyl-, Phenyl-, Pyridyl-, Thienyl- oder Pyrimidinyl-Ring bilden, wobei diese Ringe gegebenenfalls bis zu 2fach gleich oder verschieden durch Fluor, Chlor, Brom oder durch eine Gruppe der Formel -V-W oder W substituiert sind,
worin
V geradkettiges oder verzweigtes Alkylen mit bis zu 4 Kohlenstoffatomen bedeutet,
W Wasserstoff oder eine Gruppe der Formel -OR^{4"},-CO-R^{5"} oder -NR^{9'}R^{10'} bedeutet,
worin
R^{4"} und R^{5"} die oben angegebene Bedeutung haben, und
R^{9'} und R^{10'} die oben angegebene Bedeutung von R⁹ und R¹⁰ haben und mit dieser gleich oder verschieden sind,
- B: für unverzweigtes Alkyl, Alkenyl oder Alkinyl mit jeweils bis zu 3 Kohlenstoffatomen steht,
- D: für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
- R¹ und R²: gleich oder verschieden sind und
für Wasserstoff, Hydroxy oder für geradkettiges oder verzweigtes Alkyl mit bis zu 8 Kohlenstoffatomen stehen, die gegebenenfalls durch Fluor, Chlor, Brom, Cyclopropyl, Cyclopentyl, Cyclohexyl oder Phenyl substituiert ist, das seinerseits durch Fluor, Chlor, Brom oder durch geradkettiges oder verzweigtes Alkyl oder Alkoxy mit jeweils bis zu 6 Kohlenstoffatomen substituiert sein kann,
für Cyclobutyl, Cyclopentyl, Cyclohexyl, Cycloheptyl, Cyclooctyl oder Phenyl stehen, die gegebenenfalls durch Fluor, Chlor, Brom oder durch geradkettiges oder verzweigtes Alkyl oder Alkoxy mit jeweils bis zu 4 Kohlenstoffatomen substituiert sind,
oder
- R¹ und R²: gemeinsam einen Cyclopropyl-, Cyclopentyl- oder Cyclohexylring bilden, der gegebenenfalls durch geradkettiges oder verzweigtes Alkyl oder Alkoxy mit jeweils bis zu 4 Kohlenstoffatomen, Phenyl, Fluor oder Chlor substituiert ist,
- R³: für Tetrazolyl steht, oder
für eine Gruppe der Formel -C ≡ N, -CO-R¹⁸, -CO-NR¹⁹R²⁰, -CO-NR²¹-SO₂R²² oder -SO₂-NR²³R²⁴ steht,
worin
R¹⁸ Hydroxy, geradkettiges oder verzweigtes Alkoxy mit bis zu 8 Kohlenstoffatomen oder Phenoxy bedeutet,
R¹⁹ und R²⁰ gleich oder verschieden sind und die oben angegebene Bedeutung von R⁹ und R¹⁰ haben und mit dieser gleich oder verschieden sind oder
R¹⁹ die oben angegebene Bedeutung hat und
R²⁰ eine Gruppe der Formel 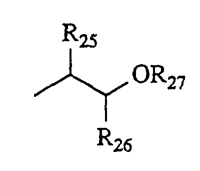 bedeutet,
worin
R²⁵ und R²⁶ gleich oder verschieden sind und Wasserstoff, Phenyl, Benzyl, Pyridyl oder Thienyl bedeuten, die ihrerseits einfach oder zweifach durch Hydroxy, Carboxy, Fluor, Chlor, Brom oder durch geradkettiges oder verzweigtes Alkyl, Alkoxy oder Alkoxycarbonyl mit jeweils bis zu 6 Kohlenstoffatomen substituiert sein können,
R²⁷ Wasserstoff oder geradkettiges oder verzweigtes Alkyl oder Acyl mit jeweils bis zu 6 Kohlenstoffatomen bedeutet,
R²¹, R²³ und R²⁴ gleich oder verschieden sind und die oben angegebene Bedeutung von R⁹ und R¹⁰ haben und mit dieser gleich oder verschieden ist,
R²² die oben angegebene Bedeutung von R¹¹ hat und mit dieser gleich oder verschieden ist,
oder einen Rest der Formel 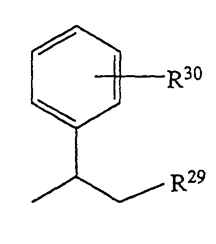 bedeutet,
worin
R²⁹ Hydroxy, Carboxy oder geradkettiges oder verzweigtes Alkoxycarbonyl mit bis zu 6 Kohlenstoffatomen bedeutet, und
R³⁰ Wasserstoff, Fluor oder Chlor bedeutet,
und deren Salze.

Besonders bevorzugt sind Verbindungen der allgemeinen Formel (I),
in welcher
- A: für einen über ein Stickstoffatom gebundenen Imidazolring der Formel 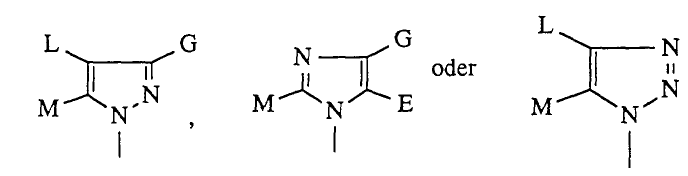 steht,
worin
E, G, L und M gleich oder verschieden sind und Cyclopropyl, Wasserstoff, Tetrazolyl, Fluor, Jod, Chlor, Brom, Trifluormethyl, Perfluoralkyl mit bis zu 4 Kohlenstoffatomen oder eine Gruppe der Formel 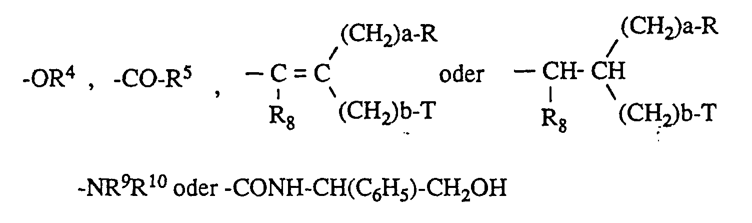 bedeuten,
worin
R⁴ Wasserstoff, geradkettiges oder verzweigtes Alkyl oder Acyl mit jeweils bis zu 4 Kohlenstoffatomen oder Phenyl bedeutet, das seinerseits durch Fluor, Chlor oder durch geradkettiges oder verzweigtes Alkyl oder Alkoxy mit jeweils bis zu 4 Kohlenstoffatomen substituiert ist, oder Methoxyethoxymethyl bedeutet,
R⁵ Wasserstoff, Hydroxy oder geradkettiges oder verzweigtes Alkyl oder Alkoxy mit jeweils bis zu 4 Kohlenstoffatomen oder eine Gruppe der Formel -NR⁹R¹⁰ oder -NR⁹-SO₂R¹¹ bedeutet,
worin
R⁹ und R¹⁰ gleich oder verschieden sind und Wasserstoff oder geradkettiges oder verzweigtes Alkyl oder Acyl mit jeweils bis zu 4 Kohlenstoffatomen bedeuten,
R¹¹ geradkettiges oder verzweigtes Alkyl mit bis zu 4 Kohlenstoffatomen, Benzyl, 2-Phenylvinyl oder Phenyl bedeutet, das gegebenenfalls durch Fluor, Chlor, Brom, Trifluormethyl oder durch Methyl oder Ethyl substituiert ist,
R⁸ Wasserstoff, Hydroxy, Acetyloxy oder geradkettiges oder verzweigtes Alkyl mit bis zu 4 Kohlenstoffatomen bedeutet,
a und b gleich oder verschieden sind und eine Zahl 0, 1 oder 2 bedeuten,
R Wasserstoff oder Phenyl, Thienyl oder Furyl bedeutet, die gegebenenfalls durch Carboxy, geradkettiges oder verzweigtes Alkyl, Alkoxy oder Alkoxycarbonyl mit jeweils bis zu 4 Kohlenstoffatomen, Fluor, Chlor oder Trifluormethyl substituiert sind,
T Wasserstoff oder eine Gruppe der Formel -OR^{4'} oder -CO-R^{5'} bedeutet,
worin
R^{4'} und R^{5'} die oben angegebene Bedeutung von R⁴ und R⁵ haben und mit dieser gleich oder verschieden sind, oder
E, G, L und M geradkettiges oder verzweigtes Alkyl, Alkenyl oder Alkadienyl mit jeweils bis zu 6 Kohlenstoffatomen bedeuten, die gegebenenfalls bis zu 2fach durch Fluor, Chlor, Brom, Tetrazolyl, Cyclohexyl, Phenyl oder durch eine der Gruppen 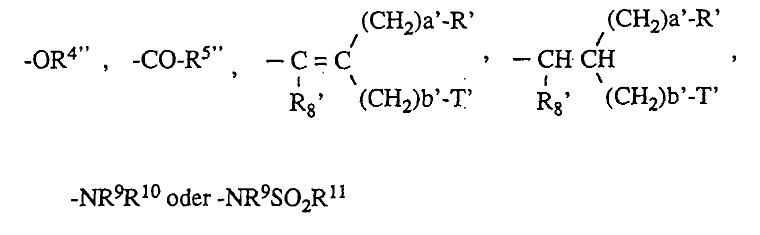 substituiert sind,
worin
R^{4"}, R^{5"}, R^{8'}, a', b', R' und T' die oben angegebene Bedeutung von R⁴, R⁵, R⁸, a, b, R und T haben und mit dieser gleich oder verschieden sind, und
R⁹, R¹⁰ und R¹¹ die oben angegebene Bedeutung haben, oder
E und G oder L und M gemeinsam mit dem Heterocyclus einen an diesen ankondensierten Cyclohexyl-, Phenyl- Thienyl, Pyridyl- oder Pyrimidiylring bilden, wobei diese Ringe gegebenenfalls durch Fluor, Chlor, Brom oder eine Gruppe der Formel -V-W oder W substituiert sind,
worin
V geradkettiges oder verzweigtes Alkylen mit bis zu 3 Kohlenstoffatomen bedeutet,
W Wasserstoff oder eine Gruppe der Formel -OR^{4"}, -COR^{5"} oder -NR^{9'}R^{10'} bedeutet,
worin
R^{4"} und R^{5"} die oben angegebene Bedeutung haben,
R^{9'} und R^{10'} die oben angegebene Bedeutung von R⁹ und R¹⁰ haben und mit dieser gleich oder verschieden ist,
- B: für die -CH₂-, -(CH₂)₂-, -(CH₂)₃-, -HC=CH- oder -C ≡ C-Gruppe steht,
- D: für Wasserstoff, Fluor, Chlor, Brom, Trifluormethyl, Trifluormethoxy oder Methyl steht,
- R¹ und R²: gleich oder verschieden sind und
für Wasserstoff, Hydroxy oder für geradkettiges oder verzweigtes Alkyl mit bis zu 7 Kohlenstoffatomen stehen, das gegebenenfalls durch Fluor, Chlor, Cyclopropyl, Cyclopentyl, Cyclohexyl oder Phenyl substituiert ist, das gegebenenfalls durch Fluor, Chlor oder durch geradkettiges oder verzweigtes Alkyl oder Alkoxy mit jeweils bis zu 4 Kohlenstoffatomen substituiert ist, oder für Cyclopentyl oder Cycloheptyl stehen,
oder
- R¹: für Wasserstoff steht
und
- R²: für Cyclopentyl, Cyclohexyl, Cycloheptyl, Cyclooctyl oder Phenyl steht, die gegebenenfalls durch Fluor, Chlor oder durch Methyl, Ethyl oder Methoxy substituiert sind,
oder
- R¹ und R²: gemeinsam einen Cyclopropyl, Cyclopentyl oder Cyclohexyl-Ring bilden, der gegebenenfalls durch Methyl, Methoxy, Phenyl oder Fluor substituiert ist,
- R³: für Tetrazolyl steht, oder
für eine Gruppe der Formel -C ≡ N, -CO-R¹⁸, -CO-NR¹⁹R²⁰, -CO-NR²¹-SO₂R²² oder -SO₂-NR²³R²⁴ steht,
worin
R¹⁸ Hydroxy, geradkettiges oder verzweigtes Alkoxy mit bis zu 6 Kohlenstoffatomen oder Phenoxy bedeutet,
R¹⁹ und R²⁰ gleich oder verschieden sind und die oben angegebene Bedeutung von R⁹ und R¹⁰ haben und mit dieser gleich oder verschieden sind, oder
R¹⁹ Wasserstoff bedeutet und
R²⁰ eine Gruppe der Formel 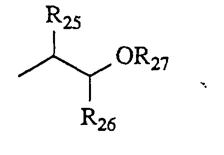 bedeutet,
worin
R²⁵ und R²⁶ gleich oder verschieden sind und Wasserstoff, Phenyl oder Benzyl bedeuten, die ihrerseits einfach oder zweifach durch Hydroxy, Fluor, Chlor oder durch geradkettiges oder verzweigtes Alkyl mit bis zu 4 Kohlenstoffatomen substituiert sein können,
R²⁷ Wasserstoff oder geradkettiges oder verzweigtes Alkyl oder Acyl mit jeweils bis zu 4 Kohlenstoffatomen bedeutet,
R²¹, R²³ und R²⁴ gleich oder verschieden sind und die oben angegebene Bedeutung von R⁹ und R¹⁰ haben und mit dieser gleich oder verschieden ist,
R²² die oben angegebene Bedeutung von R¹¹ hat und mit dieser gleich oder verschieden ist,
oder einen Rest der Formel 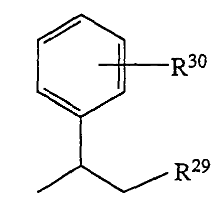 bedeutet,
worin
R²⁹ Hydroxy, Carboxy, Methoxy- oder Ethoxycarbonyl bedeutet, und
R³⁰ Wasserstoff, Fluor oder Chlor bedeutet,
und deren Salze.

Außerdem wurde ein Verfahren zur Herstellung der erfindungsgemäßen Verbindungen der allgemeinen Formel (I) gefunden, dadurch gekennzeichnet,
daß man
Verbindungen der allgemeinen Formel (II) 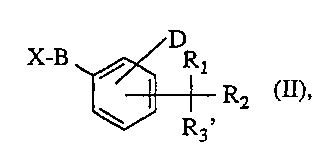 in welcher
- B, D, R¹ und R²: die oben angegebene Bedeutung haben,
- X: für eine typische Abgangsgruppe wie beispielsweise Chlor, Brom, Jod, Tosylat oder Mesylat, vorzugsweise für Brom, steht
und
- R^{3'}: für Alkoxycarbonyl mit bis zu 6 Kohlenstoffatomen oder Cyano steht,
mit Verbindungen der allgemeinen Formel (III)

A-H (III),

worin
- A: die oben angegebene Bedeutung hat,
in inerten Lösemitteln, gegebenenfalls in Anwesenheit einer Base und gegebenenfalls unter Schutzgasatmosphäre zu Verbindungen der allgemeinen Formel (Ia) 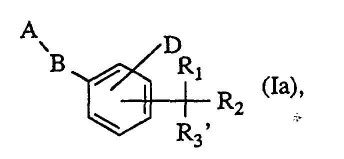 in welcher
- A, B, D, R¹, R² und R^{3'}: die oben angegebene Bedeutung haben,
umsetzt.
im Fall der Säuren (R³ = COOH) die Ester- oder Cyanogruppe nach üblicher Methode verseift,
im Fall der Herstellung der Amide und Acylsulfonsäureamide (R³ = -CO-NR¹⁹R²⁰, -CO-NR²¹SO₂-R²²) entweder direkt die Ester oder die Säuren nach üblicher Aktivierung, gegebenenfalls in Anwesenheit einer Base, eines Hilfs-, säurebindenden- und/oder eines Dehydratisierungsmittels, mit Aminen oder Sulfonsäureaminen der allgemeinen Formeln (IV) oder (V)

HNR¹⁹R²⁰ (IV),

HNR²¹SO₂R²² (V),

in welchen
- R¹⁹, R²⁰, R²¹ und R²²: die oben angegebene Bedeutung haben,
umsetzt,
und
im Fall, daß R³ für den Tetrazolring steht, die Cyanoverbindung (II, R^{3'} = CN) entweder in Anwesenheit von Aminhydrochloriden, vorzugsweise Triethylaminhydrochlorid, mit Natriumazid, oder mit einem Zinnazid, vorzugsweise Trimethylzinnazid, umsetzt,
und gegebenenfalls sowohl den Substituenten D als auch die Substituenten E, G, M und L auf jeder Stufe, vorzugsweise über die Säurestufe (R³ = COOH) nach üblicher Methode wie beispielsweise Halogenierung, Dehydrohalogenierung, Reduktion, Oxidation, Alkylierung oder Hydrolyse einführt oder in andere funktionelle Gruppen überführt,
gegebenenfalls die Isomeren trennt, und im Fall der Herstellung der Salze mit einer entsprechenden Base oder Säure umsetzt.

Das erfindungsgemäße Verfahren kann durch folgendes Formelschema beispielhaft erläutert werden:

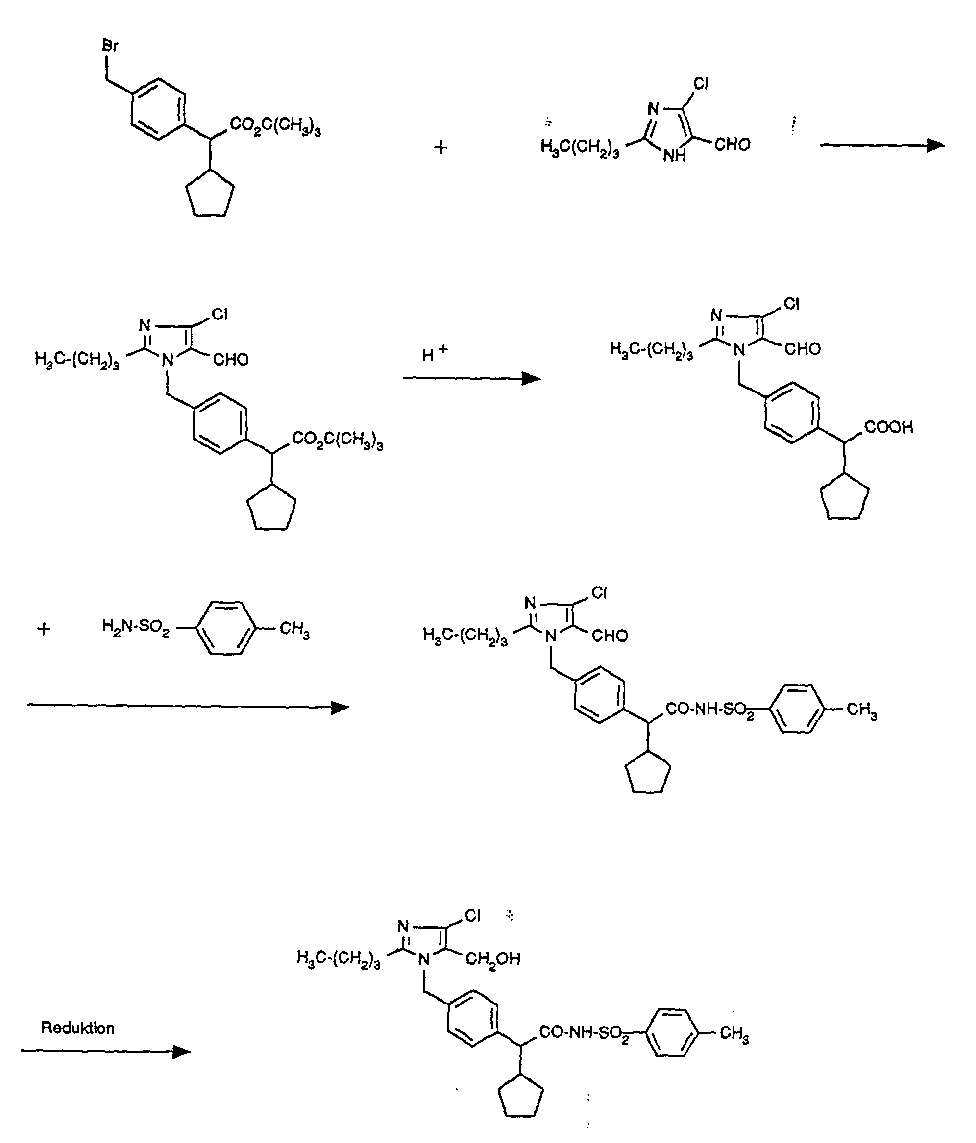

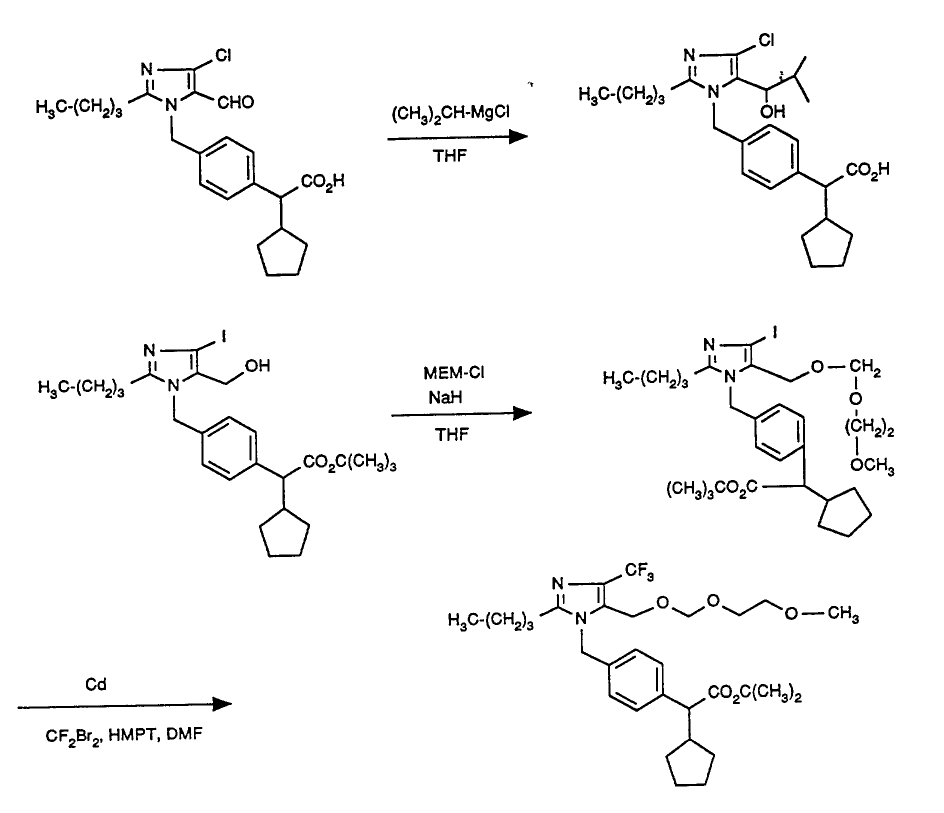

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure, erfolgen.

Die Hydrolyse wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuem. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Amidierung und die Sulfonamidierung mit den Verbindungen der allgemeinen Formeln (IV) und (V) erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide (II, R^{3'} = CO-Halogen), die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +120°C, vorzugsweise von -10°C bis +30°C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der Verbindungen der allgemeinen Formeln (IV) und (V), eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Frerman et al., J. Biol. Chem. 225, 507 (1982) und N.B. Benoton, K. Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren (R³ = CO₂H), eingesetzt.

Die Hydrolyse der Nitrile erfolgt im allgemeinen in Wasser oder einem der oben aufgeführten Lösemittel, vorzugsweise in Ethanol, Isopropanol, Ethylenglycol oder Glycine oder deren Gemische in Anwesenheit einer Base, Säure, Wasserstoffperoxid oder Alkali- oder Erdalkalimetallperoxiden wie beispielsweise Natriumperoxid.

Als Basen eignen sich im allgemeinen für die Hydrolyse Alkali- oder Erdalkalihydroxide wie beispielsweise Kaliumhydroxid, Natriumhydroxid oder Bariumhydroxid. Bevorzugt ist Natriumhydroxid.

Als Säuren eignen sich im allgemeinen anorganische Säuren wie beispielsweise Salzsäure oder Schwefelsäure.

Die Hydrolyse der Nitrile erfolgt im allgemeinen bei einem Temperaturbereich von -20°C bis +200°C, bevorzugt von +20°C bis +150°C.

Die Reduktion einer Doppel- oder Mehrfachbindung (B = C₁-C₄-Alkenyl, Alkinyl) erfolgt im allgemeinen durch Hydrierung mit Wasserstoff in Anwesenheit eines Katalysators wie beispielsweise Platin oder Platinoxide, Rhodium, Ruthenium, Chlorotris(triphenylphosphin)rhodium, oder Palladium auf Tierkohle, bevorzugt mit Palladium auf Tierkohle in einem Temperaturbereich von 0°C bis +150°C, bevorzugt von +25°C bis +100°C.

Als Lösemittel für die Hydrierung eignen sich protische Lösemittel wie beispielsweise Methanol, Ethanol und/oder aprotische Lösemittel wie beispielsweise Tetrahydrofuran, Toluol, Dimethylformamid, Methylenchlorid, Dioxan oder Essigester.

Die Hydrierung wird bei einem Druck von 1 bis 300 atm, vorzugsweise bei 1 bis 20 atm durchgeführt.

Die eingesetzte Menge an Katalysatoren beträgt 0,05 bis 5 mol, bevorzugt 0,1 bis 1,5 mol, bezogen 1 mol der Verbindungen der allgemeinen Formeln (I) oder (Ia) (B = -HC=CH- oder ―C≡C―).

Die oben aufgeführte Derivatisierung der Substituenten D, E, G, L und M erfolgt im allgemeinen nach literaturbekannten Methoden, wobei beispielhaft die Reduktion von Aldehyden oder Alkoxycarbonylverbindungen zu Alkoholen (a) und die Alkylierung (b) mit folgendem erläutert werden sollen:
a) Die Reduktion von Alkoxycarbonylverbindungen oder Aldehyden zu den entsprechenden Alkoholen erfolgt im allgemeinen mit Hydriden, wie Lithiumaluminiumhydrid oder Natriumborhydrid, bevorzugt mit Lithiumaluminumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0°C bis +150°C, bevorzugt von +20°C bis +100°C, bei Normaldruck.
b) Die Alkylierung erfolgt im allgemeinen in einem der oben aufgeführten Lösemitteln mit Alkylierungsmitteln wie beispielsweise (C₁-C₈)-Alkylhalogenide, Sulfonsäureester oder substituierte oder unsubstituierte (C₁-C₆)-Dialkyl- oder (C₁-C₁₀)-Diarylsulfate, vorzugsweise Methyljodid, p-Toluolsulfonsäureester oder Dimethylsulfat. Ebenso kann die Alkylierung mit organometallischen Verbindungen, wie beispielsweise (C₁-C₆)-Alkyllithiumverbindungen, (C₁-C₈)-Alkylmagnesiumhalogeniden, Arylmagnesiumhalogeniden, insbesondere den Chloriden und Bromiden, oder Perfluor-(C₁-C₁₀)-allylcadmiumverbindungen erfolgen, wobei die jeweilige Alkylkette geradkettig oder verzweigt sein kann.
   In diesen Fällen eignen sich als Lösemittel insbesondere Dimethylformamid, HMPT und Tetrahydrofuran.
   Die Alkylierungen werden im Fall der Alkyllithium- und Alkyl- bzw. Arylmagnesiumhalogeniden in einem Temperaturbereich von -80°C bis 70°C, bevorzugt von -78°C bis 0°C, im Fall der Perfluorcadmiumverbindungen von Raumtemperatur bis +100°C, insbesondere von +40°C bis +80°C, durchgeführt.

Die Verbindungen der allgemeinen Formel (II) sind teilweise bekannt und können hergestellt werden, indem man beispielsweise

Verbindungen der allgemeinen Formel (VI) 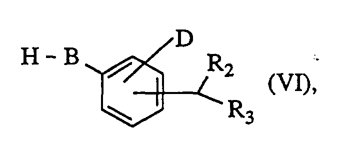 in welcher
- B, D, R² und R^{3'}: die oben angegebene Bedeutung haben,
zunächst mit Verbindungen der allgemeinen Formel (VII)

R³¹-Z (VII),

in welcher
- R³¹: die oben angegebene Bedeutung von R¹ oder R² hat, aber nicht für Wasserstoff steht,
und
- Z: für Halogen, vorzugsweise für Brom, steht,
in inerten Lösemitteln, gegebenenfalls in Anwesenheit einer Base, alkyliert,
und in einem zweiten Schritt an dem Substituenten B eine Bromierung, gegebenenfalls in Anwesenheit eines Katalysators nach üblicher Methode durchführt.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Neben den oben aufgeführten Basen eignet sich vorzugsweise Kalium-tert.butylat für die Alkylierung.

Die Bromierung erfolgt im allgemeinen mit Br₂ oder N-Bromsuccinimid, vorzugsweise mit N-Bromsuccinimid, in einem der oben aufgeführten Lösemittel, vorzugsweise Tetrachlorkohlenstoff, in einem Temperaturbereich von 0°C bis +150°C, vorzugsweiese von 0°C bis +80°C, und Normaldruck.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VI), eingesetzt wird.

Die Verbindungen der allgemeinen Formel (VI) sind an sich bekannt oder können nach bekannten Methoden hergestellt werden [vgl. J. Chem. Soc., Perkin Trans. 1, (9), 1706 - 1707; J. Chem. Soc., Chem. Commun., (2), 167 - 168].

Die Verbindungen der allgemeinen Formel (VII) sind an sich bekannt [vgl. Beilstein 5, 19/5, 24/5, 29] oder können nach üblicher Methode aus den entsprechenden Alkoholen oder Cycloalkenen hergestellt werden.

Ebenso sind die Verbindungen der allgemeinen Formel (III) an sich bekannt [vgl. z.B. Beilstein 25, 163; 23, 45; US 4 355 040] oder können nach üblicher Methode hergestellt werden.

Die Verbindungen der allgemeinen Formel (Ia) sind neu und können nach den oben aufgeführten Verfahren hergestellt werden.

Die Verbindungen der allgemeinen Formeln (IV) und (V) sind bekannt oder können nach bekannten Verfahren hergestellt werden [vgl. z.B. Beilstein 11/104, R.V. Vitzgert, Uspekhi, Khimii 32, 3 (1963); Russian Chem. Rev. 32, 1 (1969); Beilstein 4, 87].

Die erfindungsgemäßen heterocyclisch substituierten Phenylessigsäurederivate (I) zeigen ein nicht vorhersehbares, wertvolles pharmakologisches Wirkspektrum.

Die erfindungsgemäßen Verbindungen besitzen eine spezifische A-II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüber hinaus inhibieren sie die Proliferation von glatten Muskelzellen.

Sie können deshalb in Arzneimittel zur Behandlung der arterieller Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen Applikationsvolumen pro Einzelgabe = 100 µl): | | |
|---|---|---|
| KCl | 22,7;32,7;42,7;52,7 | mmol/l |
| 1Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration, bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

**Tabelle A:**

| Hemmung der Gefäßkonzentration an isolierten Aortenringen von Kaninchen in vitro | | |
|---|---|---|
| IC₅₀ (mol/l) gegen Kontraktionen, induziert durch: | | |

| Bsp.Nr.: | AII | KCl |
|---|---|---|
| I | > 2,2.10⁻⁴ | - |
| II | 4,1.10⁻⁶ | > 10⁻⁴ |

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Antiotensin II in einem Assay-Volumen von 0,25 ml durchgeführt, das im einzelnen die partiell gereinigten Membranen (50 - 80 µg), ³H-Angiotensin II(3-5 nM), Test-Pufferlösung (50 mM Tris, pH 7,2, 5 mM MgCl₂, 0,25% BSA) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀.-Werte; IC₅₀-Werte: Konzentration bei der die zu untersuchende Substanz eine 50%ige Hemmung der totalen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermittln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg, Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

### Lösungsmittel

A = Dichlormethan:Methanol = 10:1
B = Dichlormethan:Methanol = 50:1
C = Dichlormethan:Methanol = 20:1
D = Petrolether:Essigester = 1:2
E = Petrolether:Essigester = 2:1
F = Methylenchlorid:Methanol = 9:1
G = Methylenchlorid:Methanol:Essigsäure = 9:1:0,1
H = Essigester:Petrolether = 7:3
I = Essigester:Petrolether = 3:7
J = Essigester:Petrolether = 4:1
K = Essigester:Petrolether = 1:1
L = Essigester:Petrolether = 10:1
M = Petrolether:Essigester = 5:1
N = Petrolether:Ether = 5:1.
O = Toluol: Dioxan:Eisessig = 75:21:3
P = Methylenchlorid:Methanol = 95:5
Q = Essigester:Methanol = 4:1

### Definition der Isomeren

- 4dia =: Gemisch der vier möglichen Diastereomeren wenn zwei asymmetrische C-Atome im Molekül sind.
- diaA/rac =: racemisches Diastereomer mit dem größeren Rf-Wert
- diaB/rac =: racemisches Diastereomer mit dem kleineren Rf-Wert
- diaA/ent =: Diastereomer mit dem größeren Rf-Wert (ein Enantiomer)
- diaB/ent =: Diastereomer mit dem kleineren Rf-Wert (ein Enantiomer)
- 2dia/ent =: Gemisch zweier enantiomerenreiner Diastereomeren
- rac =: Racemat
- ent =: Enantiomer

### Ausgangsverbindungen

### Beispiel 1

### 4-Methylphenylessigsäure-tert.butylester

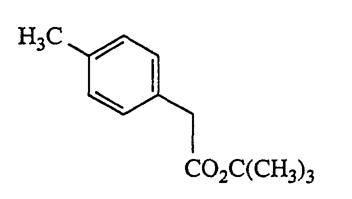

450 g (3 mol) 4-Methylphenylessigsäure, 1,13 1 (12 mol) tert.Butanol und 90 g (0,74 mol) Dimethylaminopyridin werden in 21 Dichlormethan gelöst. Nach Zugabe von 680 g (3,3 mol) Dicyclohexylcarbodiimid, gelöst in 400 ml Dichlormethan, wird 20 h bei 25°C gerührt, der ausgefallene Harnstoff abgesaugt, mit 200 ml Dichlormethan gewaschen und die organische Phase je zweimal mit 500 ml 2 N Salzsäure und Wasser gewaschen. Die organische Phase wird eingeengt und destilliert.
Ausbeut: 408 g (66% der Theorie)
Siedepunkt: 73- 78°C /0,2 mm

### Beispiel 2

### 2-Cyclopentyl-2-(4-methylphenyl)essigsäure-tert.butylester

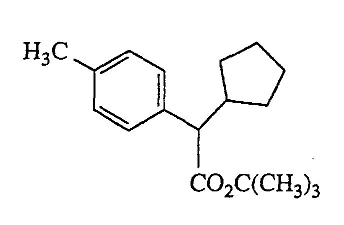

33,5 g (0,3 mol) Kalium-tert.butylat werden in 100 ml DMF unter Feuchtigkeitsausschluß bei 0°C vorgelegt, und 51,6 g (0,25 mol) 4-Methylphenylessigsäure-tert.butylester in 250 ml DMF zugetropft. Es wird 30 min bei 0°C gerührt und 32,2 ml (0,3 mol) Cyclopentylbromid in 150 ml DMF bei 5-15°C zugetropft und 20 h bei 25°C gerührt. Nach Einengen wird der Rückstand zwischen Wasser/Diethylether verteilt, die Etherphase über Natriumsulfat getrocknet und eingeengt. Das Produkt kristallisiert aus.
Ausbeute: 67 g (97,5% der Theorie)
Festpunkt: 51 - 53°C

### Beispiel 3

### 2-(4-Brommethyl-phenyl)-2-cyclopentyl-essigsäure-tert.butylester

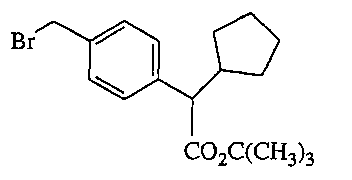

27,4 g (0,1 mol) 2-Cyclopentyl-2-(4-methylphenyl)-essigsäure-tert.butylester werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltriert. Nach Einengen des Filtrates fällt das Produkt aus. Es wird mit Petrolether (40/60) gewaschen und getrocknet.
Ausbeute: 20 g (57% der Theorie)
Festpunkt: 73 - 76°C

### Beispiel 4

### 2-(4-Trifluormethansulfonyloxy-phenyl)-2-cyclopentyl-essigsäure-tert.butylester

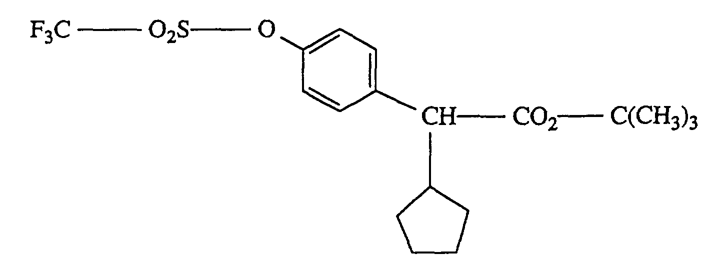

Zu einer Lösung von 13,8 g (50 mmol) 2-(4-Hydroxy-phenyl)-2-cyclopentyl-essigsäure-tert.butylester in 25 ml Pyridin gibt man bei 0°C 14,1 g (55 mmol) Trifluormethansulfonsäureanhydrid. Die Lösung wird noch 2 h bei Raumtemperatur gerührt und dann auf Eiswasser gegossen. Die übliche wäßrige Aufarbeitung liefert 19,3 g eines Rohproduktes, das an Kieselgel chromatographiert wird (CH₂Cl₂).
Ausbeute: 18,7 g (91,4%) der Titelverbindung.
R_{f} (CH₂Cl₂): 0,9.

### Beispiel 5

### 2-(4-Vinyl-phenyl)-2-cyclopentyl-essigsäure-tert.butylester

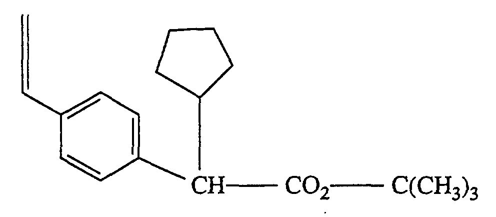

Zu einer Lösung von 46 mg Trifurylphosphin und 50 mg Pd(dba)2 in 20 ml N-Methylpyrrolidinon gibt man 2,04 g (5 mmol) der Verbindung aus Beispiel 4, 1,58 g (5 mmol) Tributylvinylzinn und 1,27 g (30 mmol) LiCl. Die Lösung wird 16 h bei Raumtemperatur gerührt, dann mit Wasser versetzt und wäßrig aufgearbeitet. Das erhaltene Rohprodukt wird an Kieselgel chromatographiert. Man erhält 1,14 g (80,0%) der Titelverbindung.
R_{f} (Petrolether/Ether 20:1):0,6.

### Beispiel 6

### 2-[4-(2-Hydroxyethyl)phenyl]-2-cyclopentyl-essigsäure-tert.butylester

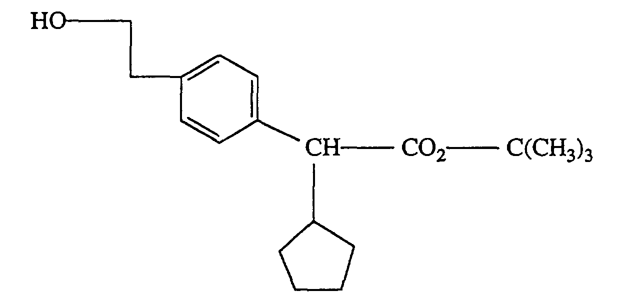

Zu einer Lösung von 400 mg (1,4 mmol) der Verbindung des Beispiels 5 in 0,3 ml CH₂Cl₂ gibt man bei 0°C 188 mg (1,54 mmol) Borabicyclononan und rührt anschließend jeweils 2 h bei Raumtemperatur und 2 h bei 80°C. Anschließend versetzt man mit 100 µl H₂O (5,6 mmol), 473 µl H₂O₂ (4,6 mmol) und 513 µl NaOH (3M, 1,54 mmol), rührt bei 60°C und anschließend 16 h bei Raumtemperatur. Die übliche wäßrige Aufarbeitung liefert 647 mg eines Rohprodukts, das an Kieselgel chromatographiert wird.
Ausbeute: 134 mg (34,5%) der Titelverbindung.
R_{f} (Petrolether/Ether 1:1): 0,24.

### Beispiel 7

### 2-[4-(2-Toluolsulfonyloxyethyl)phenyl]-2-cyclopentyl-essigsäure-tert.butylester

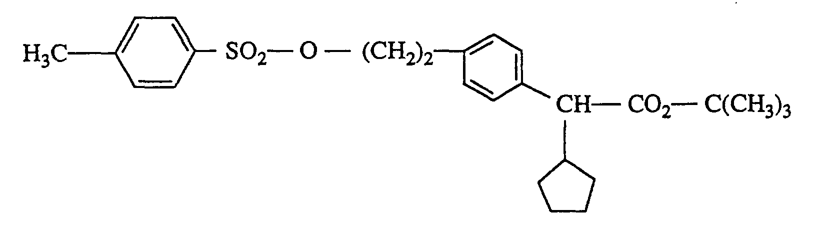

Zu einer Lösung von 100 mg (0,33 mmol) der Verbindung des Beispiels 6, 48 mg (0,34 mmol) Triethylamin und 4 mg (0,03 mmol) Dimethylaminopyridin in 350 ul CH₂Cl₂ gibt man bei 4°C 65 mg (0,34 mmol) Toluolsulfonylchlorid, rührt 4 h bei 4°C nach und anschließend 1 h bei Raumtemperatur. Man versetzt die Lösung mit 12 µl (0,66 mmol) H₂O, läßt 0,5 h bei Raumtemperatur rühren, gibt 4 µl Essigsäure zu und arbeitet wäßrig auf. Man erhält ein Rohprodukt, das nach Chromatographie an Kieselgel (Cyclohexan/Ether 15:1) 103 mg (67%) der Titelverbindung liefert.
R_{f} (Petrolether/Ether 1:1): 0,78.

### Beispiel 8

### 2-[4-(3-Hydroxypropenyl)phenyl]-2-cyclopentyl-essigsäure-teit.butylester

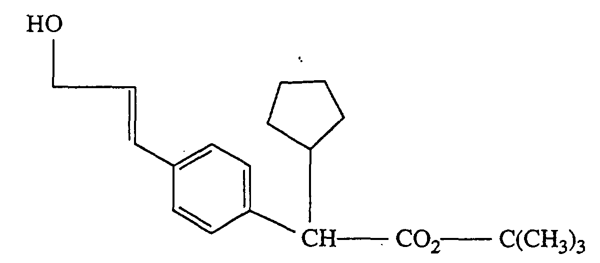

Zu einer Lösung von 2,04 g (5,0 mmol) der Verbindung des Beispiels 4 in 10 ml N-Methylpyrrolidinon werden 1,7 g (5,0 mmol) 3-Tributylstannyl-3-propenol, 46 mg Trifurylphosphin, 1,27 g (30 mmol) LiCl und 50 mg Pd(dba)2 gegeben. Die entstandene Lösung wird 4,5 h bei Raumtemperatur gerührt und anschließend wäßrig aufgearbeitet. Das erhaltene Rohprodukt wird an Kieselgel chromatographiert (Petrolether/Essigester 5:1). Man erhält 830 mg (52,5 % der Theorie) der Titelverbindung.
R_{f} (Petrolether/Ether 7:3): 0,34.

### Beispiel 9

### 2-[4-(3-Hydroxypropyl)phenyl]-2-cyclopentyl-essigsäure-tert.butylester

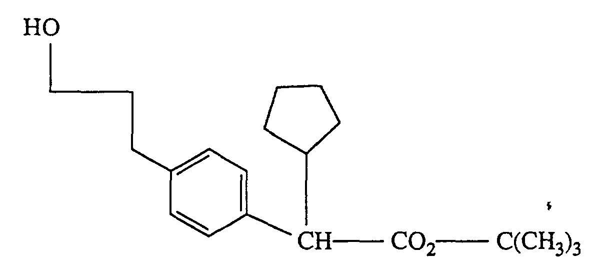

300 mg (0,94 mmol) der Verbindung des Beispiels 8 werden zu einer Suspension von 90 mg Pd auf Calciumcarbonat in 4 ml Ethanol gegeben und 4.0 h bei 50°C und 90 bar Druck mit H₂ hydriert. Das erhaltene Reaktionsgemisch wird vom Katalysator über eine Doppelschicht aus Kieselgel und Celite abfiltriert und alle flüchtigen Anteile werden im Vakuum abgezogen. Der erhaltene Rückstand wird an Kieselgel chromatographiert (Petrolether/Essigester 40:1). Man erhält 234 mg (78%) der Titelverbindung.
R_{f} (Petrolether/Ether 7:3): 0,34.

### Beispiel 10

### 2-[4-(3-Toluolsulfonyloxypropyl)phenyl-2-cyclopentyl-essigsäure-tert.butylester

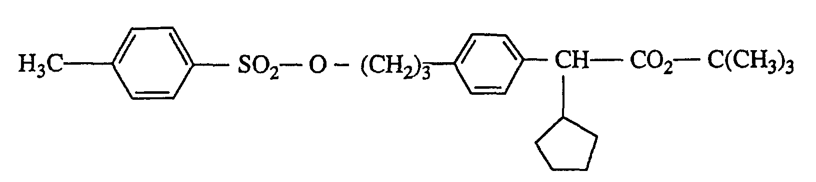

Zu einer Lösung von 200 mg (0,63 mmol) der Verbindung des Beispiels 9 in 650 µl CH₂Cl₂ gibt man 96 ul (0,69 mmol) Triethylamin und 8 mg (0,06 mmol) Dimethylaminopyridin bei 0°C. Bei dieser Temperatur fügt man 131 mg (0,69 mmol) Toluolsulfonylchlorid zu und läßt die erhaltene Mischung langsam auf Raumtemperatur erwärmen. Nach 16 h versetzt man mit 23 µl (1,26 mmol) H₂O, läßt 0,5 h nachrühren und arbeitet wäßrig auf. Das erhaltene Rohprodukt wird an Kieselgel chromatographiert (Petrolether/Essigester 40:1). Man erhält 170 mg (57% der Theorie) der Titelverbindung.
R_{f} (Petrolether/Essigester 20:1): 0,15.

### Beispiel 11

### 2,2-Dimethyl-2-(4-methylphenyl)essigsäure-tert.butylester

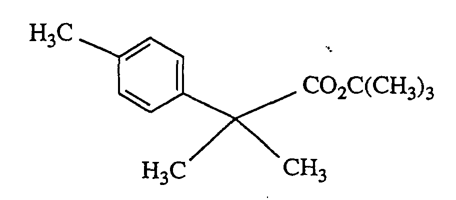

Zu 19,6 g (0,175 mmol) Kalium-tert.butylat in 70 ml DMF werden unter Rühren bei 0°C 30 g (0,145 mol) 4-Methylphenylessigsäure-tert.butylester in 175 ml DMF getropft. Nach 30 min Rühren werden 25 g (0,175 mol) Methyliodid in 100 ml DMF unter Lichtausschluß langsam zugetropft. Nach Erwärmen auf Raumtemperatur wird über Nacht gerührt, erneut auf 0°C gekühlt und die Sequenz mittels Zugabe von Kalium-tert.butylat in DMF und Methyliodid in DMF wiederholt.

Nach Einengen wird der Rückstand zwischen Diethylether und Wasser verteilt, die Wasserphase noch zweimal ausgeethert, die vereinigten Etherphasen über Natriumsulfat getrocknet und eingeengt.
Ausbeute: 26,5 g (78% der Theorie).

### Beispiel 12

### 2-(4-Brommethyl-phenyl)-essigsäure-methylester

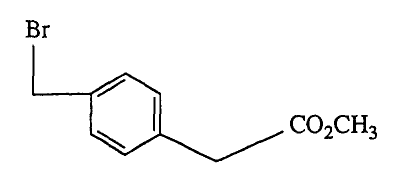

5 g (22 mmol) 2-(4-Brommethyl-phenyl)-essigsäure werden in 50 ml 2,2-Dimethoxypropan unter Zusatz von 2 ml wäßriger konz. Salzsäure über Nacht bei Raumtemperatur gerührt. Nach Einengen wird zweimal jeweils Methanol zugesetzt, wieder eingeengt und anschließend im Vakuum getrocknet.
Ausbeute: 5,3 g (>99% der Theorie).

### Beispiel 13

### 2-[4-(2-Butyl-4-chlor-5-formyl-imidazol-1-yl-methyl)-phenyl]-essigsäure

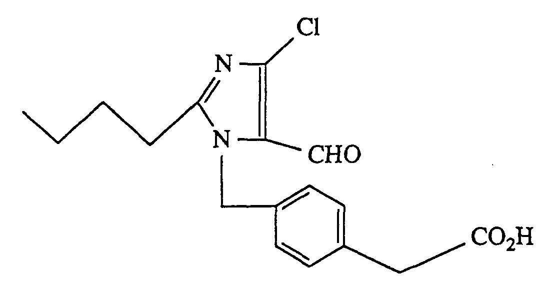

3,3 g (9,5 mmol) 2-[4-(2-Butyl-4-chlor-5-formyl-imidazol-1-yl-methyl)-phenyl]-essigsäure-methylester werden in 100 ml Dioxan/Wasser (1:1) bei 0°C gelöst und unter Rühren tropfenweise mit einer Lösung von 0,44 g (10,4 mmol) Lithiumhydroxidmonohydrat in 5 ml Wasser versetzt. Nach 3 h Rühren bei Raumtemperatur wird zur Hälfte eingeengt, mit Wasser verdünnt, mit Diethylether gewaschen, die wäßrige Phase mit IN Salzsäure angesäuert und dreimal mit Essigsäureethylester extrahiert. Die vereinigten Essigesterphasen werden über Natriumsulfat getrocknet und eingeengt.
Ausbeute: 3,2 g (> 99% der Theorie).

### Beispiel 14

### 2-(1H-imidazolyl)-2-oxo-essigsäure-tert.butylester

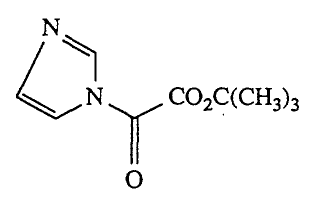

100 g (0,79 mol) Oxalylchlorid werden in 1,3 1 THF unter Rühren bei 0°C unter Argon mit 58,5 g (0,79 mol) tert.Butanol versetzt. Nach einstündigem Rühren bei 0°C werden 161 g (2,37 mol) Imidazol in 0,7 1 THF innerhalb von 60 min zugetropft. Nach weiteren 15 min wird filtriert, der Feststoff mit 0,5 1 THF gewaschen, das Filtrat auf 1 1 Volumen eingeengt, nochmals filtriert, anschließend vollständig eingeengt, über Nacht bei 0°C unter Argon stehengelassen und nochmals über eine Fritte filtriert.
Ausbeute: 137 g (89% der Theorie)
¹H-NMR (250 MHz, CDCl₃): δ = 1,63 (s, 9H, tert.Butyl).

### Beispiel 15

### 2-(4-Methyl-phenyl)-2-oxo-essigsäure-tert.butylester

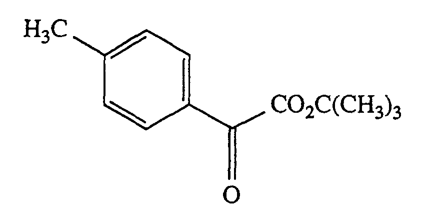

Zu 136,9 g (0,7 mol) 2-(1H-imidazolyl)-2-oxo-essigsäure-tert.butylester in 2,5 1 THF werden unter Rühren bei -78°C unter Argon 0,7 1 einer IM Lösung (0,7 mol) von p-Toluolmagnesiumbromid in Diethylether getropft. Das Gemisch wird anschließend innerhalb von 3 h auf 0°C gebracht, in Eiswasser gegossen und unter Zusatz von Essigsäure zur Verhinderung der Emulsionsbildung dreimal mit Diethylether extrahiert. Die vereinigten Etherphasen werden mit wäßriger Kochsalzlösung gewaschen, über Natriumsulfat getrocknet, und das Produkt wird destilliert.
Ausbeute: 77,3 g (50% der Theorie)
Siedepunkt: 116-119°C (1 mm Hg).

### Beispiel 16

### 2-(4-Methyl-phenyl)-2-phenyl-2-hydroxy-essigsäure-tert.butylester

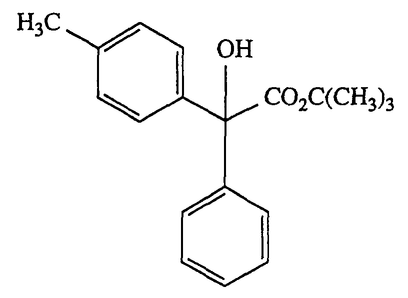

13,2 g (60 mmol) 2-(4-Methyl-phenyl)-2-oxo-essigsäure-tert.butylester werden unter Argon in 70 ml THF bei 0°C unter Rühren tropfenweise mit 30 ml einer 2M Lösung (60 mmol) von Phenyllithium in Benzol/Diethylether (3:1) versetzt und noch 1 h bei Raumtemperatur gerührt. Das Gemisch wird mit 80 ml Diethylether verdünnt, unter Rühren in 150 ml Wasser eingegossen, mit IN Essigsäure neutralisiert und dreimal mit Diethylether extrahiert. Die vereinigten organischen Phasen werden über Natriumsulfat getrocknet, eingeengt, und das Produkt wird über Kieselgel 60 mit Petrolether 40-60/Essigsäureethylester (4:1) chromatographiert.
Ausbeute: 13,3 g (74% der Theorie).

### Beispiel 17

### 2-(4-Methyl-phenyl)-2-phenyl-essigsäure

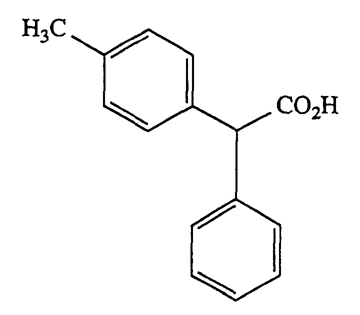

1,49 g (5 mmol) 2-(4-Methyl-phenyl)-2-phenyl-2-hydroxy-essigsäure-tert.butylester werden in 60 ml Dichlormethan bei Raumtemperatur mit 6,3 ml (40 mmol) Triethylsilan und 12,6 ml (60 mmol) Trifluoressigsäure für 3 h gerührt. Das Gemisch wird mit 60 ml Wasser verdünnt, mit gesättigter wäßriger Bicarbonatlösung auf einen pH-Wert von pH = 2-3 eingestellt und geschüttelt. Nach Abtrennen der organischen Phase wird die wäßrige Phase noch zweimal mit Dichlormethan extrahiert. Die vereinigten organischen Phasen werden über Natriumsulfat getrocknet, filtriert und eingeengt. Das Produkt wird über Kieselgel 60 mit Petrolether 40-60/Essigsäureethylester (6:4) chromatographiert.
Ausbeute: 0,80 g (71% der Theorie).

### Beispiel 18

### 5-[(1-Cyclopentyl)-1-(3-methylphenyl)]-methyl-tetrazol

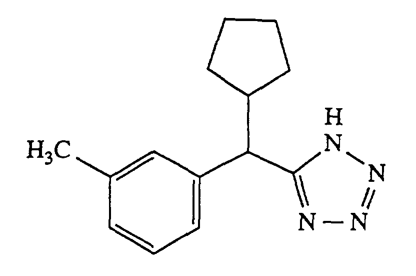

19,5 g (97,8 mmol) 2-Cyclopentyl-2-(3-methylphenyl)-acetonitril werden in 350 ml DMF p.a. gelöst, mit 63,6 g (97,8 mmol) Natriumazid und 134,33 g (97,8 mmol) Triethylammoniumchlorid versetzt und 24 h unter Rückfluß gekocht. Nach dem Abkühlen setzt man IM Schwefelsäure zu und extrahiert mit Ether. Die organische Phase wird mit 2M Natronlauge geschüttelt und die alkalische wäßrige Phase darauf mit IM Schwefelsäure angesäuert. Das Produkt wird mit Ether extrahiert, die etherische Lösung mit Natriumsulfat getrocknet und eingedampft.
Ausbeute: 20,0 g (82,4 mmol)
R_{f} (Dichlormethan:Methanol = 20:1): 0,40.

### Beispiel 19

### 5-[(1-Cyclopentyl)-1-(3-methylphenyl)]methyl-2-triphenylmethyl-tetrazol

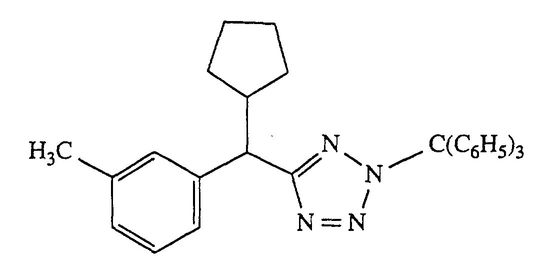

19,9 g (82,0 mmol) der Verbindung aus Beispiel 18 werden in 250 ml Dichlormethan gelöst und bei Raumtemperatur 24 h mit 24,8 g (86,7 mmol) Triphenylmethylchlorid und 13,7 g (98,7 mmol) Triethylamin umgesetzt. Man extrahiert nacheinander mit 250 ml Wasser, 200 ml IM wäßriger Zitronensäure und 200 ml Wasser. Die organische Phase wird mit Natriumsulfat getrocknet, eingedampft und im Hochvakuum von Restlösemittel befreit.
Ausbeute: 38,8 g (80,1 mmol)
R_{f} (Toluol): 0,40.

### Beispiel 20 und 21

### 2-Cyclopentyl-2-(4-methylphenyl)-acetonitril (20)

### 2,2-Dicyclopentyl-2-(4-methylphenyl)-acetonitril (21)

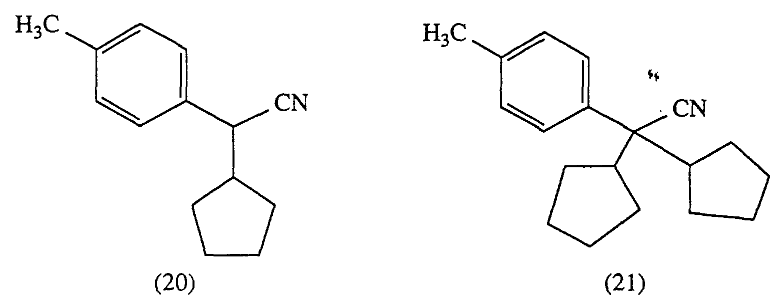

Eine Lösung von 4-Methylphenyl-acetonitril (5,0 g; 38 mmol) und Cyclopentylbromid (5,7 ml; 53 mmol) in DMF (70 ml) wurde bei -10°C mit Kalium-tert.butylat (6,6 g; 59 mmol) versetzt und über Nacht bei Raumtemperatur gerührt. Die Reaktionslösung wurde mit 1M-KHSO₄-Lösung angesäuert (pH 3-4) und eingeengt. Der Rückstand wurde in Methylenchlorid aufgenommen, dreimal mit Wasser gewaschen, über Na₂SO₄ getrocknet, filtriert und eingeengt. Filtration des Rückstands über Kieselgel (Hexan/Essigester-Stufengradient) und anschließende MPLC (Lichroprep Si60, Hexan:Essigester = 10:1) ergaben 3,45 g 2,2-Dicyclopentyl-2-p-tolyl-acetonitril [33,8% der Theorie; R_{f} = 0,53 (Hexan:Essigester = 10:1)] und 4,29 g 2-Cyclopentyl-2-(4-methylphenyl)-acetonitril (56,5% der Theorie; R_{f} 0,4) als gelbe Öle.

### Beispiel 22

### 5-[1-Cyclopentyl-1-(4-methylphenyl)methyl]-tetrazol

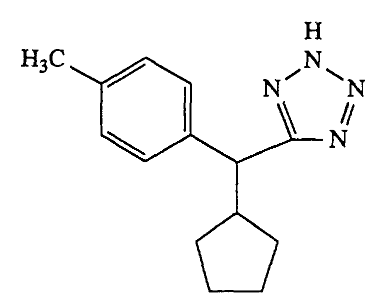

Eine Lösung der Verbindung aus Beispiel 20 (7,2 g; 36 mmol), Triethylammoniumchlorid (18 g; 0,13 mol) und Natriumazid (8,2 g; 0,13 mol) in DMF (110 ml) wurde 60 h unter Argon unter Rückfluß gekocht. Die Reaktionslösung wurde eingeengt, mit 1M-KHSO₄-Lösung angesäuert, zwischen Wasser und Essigester verteilt. Die organische Phase wurde gewaschen, über Na₂SO₄ getrocknet und eingeengt, um 10,6 g eines gelben Öls zu ergeben [Theorie: 8,7 g, Rest DMF; R_{f}: 0,53 (Dichlormethan:Methanol:Essigsäure = 20:1:0,1)].

### Beispiel 23

### 5-[1-Cyclopentyl-1-(4-methylphenyl)]-2-triphenylmethyl-tetrazol

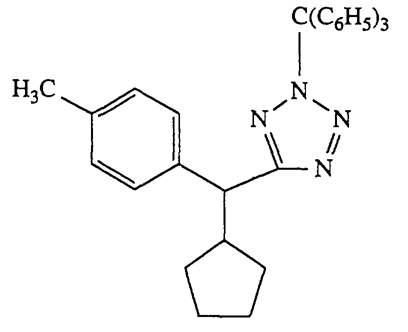

Eine Lösung von Beispiel 22 (10,6 g roh; 36 mmol), Triphenylmethylchlorid (11,5 g; 41 mmol) und Triethylamin (7,0 ml; 49 mmol) in Dichlormethan (160 ml) wurde 5 h unter Rückfluß gekocht, nach Abkühlen mit 1M-KHSO₄-Lösung gewaschen, über Na₂SO₄ getrocknet und eingeengt. Der Rückstand wurde mit Hexan/Essigester (5:1) verrieben und filtriert, um 12,7 g fahlgelbe Kristalle zu ergeben [63,8% der Theorie; Fp: 138-9°C; R_{f}: 0,58 (Hexan:Essigester = 5:1)].

### Beispiel 24 und 25

### 5-[1-(4-Brommethyl-phenyl)-1-cyclopentyl-methyl]-2-triphenylmethyl-tetrazol (24)

### 5-[1-Brom-1-(4-brommethyl-phenyl)-1-cyclopentyl-methyl]-2-triphenylmethyltetrazol (25)

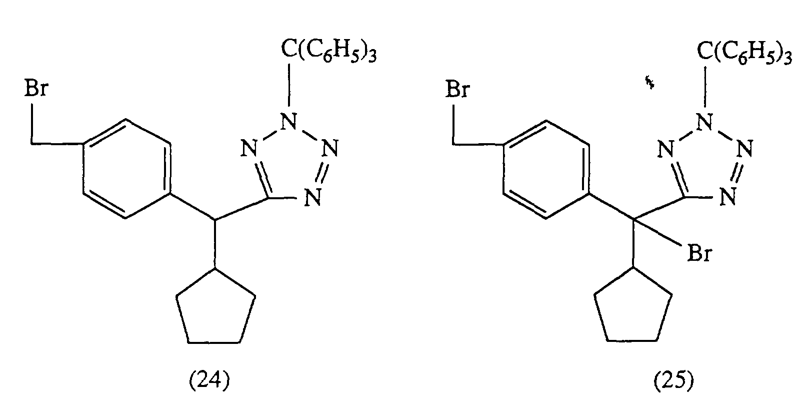

Eine Lösung von Beispiel 23 (5,0 g; 11 mmol), N-Bromsuccinimid (2,1 g; 11 mmol) und einer Spatelspitze Azoisobuttersäurenitril in Tetrachlorkohlenstoff (500 ml) wurde 2 h unter Rückfluß gekocht, abgekühlt, filtriert und eingeengt, um 6,6 g eines Gemischs von Startmaterial, Bromid und Dibromid zu ergeben (Theorie: 5,9 g).

### Beispiel 26

### 5-{1-Cyclopentyl-1-[4-(2-butyl-4-chlor-5-formyl-imidazol-1-yl-methyl)-phenyl]-methyl}-2-triphenylmethyl-tetrazol

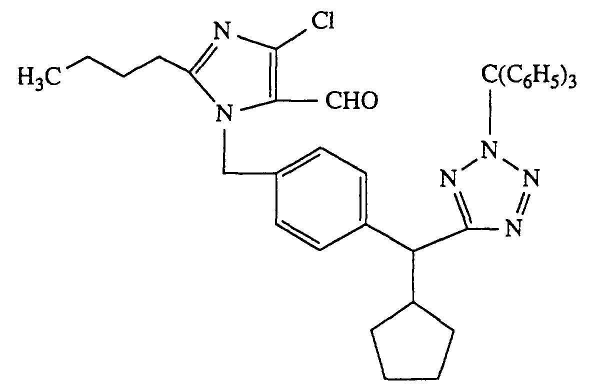

Zu einer Suspension von 80% Natriumhydrid/Paraffinöl-Dispersion (0,35 g; 12 mmol) in DMF (30 ml) wurde bei -10°C eine Lösung von 2-Butyl-4-chlor-5-fonnyl-imidazol (1,53 g; 8,2 mmol) in DMF (60 ml) zugetropft, und 15 min wurde bei 0°C nachgerührt. Bei -10°C wurde eine Lösung von Beispiel 24 (6,6 g roh; 11 mmol) in DMF (60 ml) zugetropft und bei Raumtemperatur über Nacht gerührt. Einengen und Kieselgelchromatographie (Toluol:Essigester = 9:1) lieferten 2,28 g eines gelben Öls [29% der Theorie; R_{f}: 0,57 (Toluol:Essigester = 5:1)].

### Beispiel 27

### 2-(4-Brommethyl-phenyl)-2,2-dicyclopentyl-acetonitril

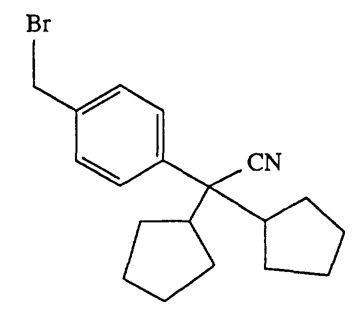

Bromierung von Beispiel 21 analog Beispiel 24 lieferte 1,88 g eines klaren Öls [70% der Theorie; R_{f}: 0,58 (Hexan:Essigester = 10:1)].

### Beispiel 28

### 2-Butyl-5-hydroxymethyl-4-jod-imidazol

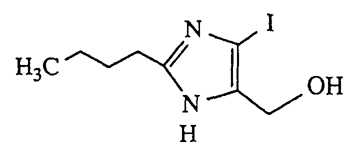

Eine Lösung von 2-Butyl-4-hydroxymethyl-imidazol (15,4 g; 100 mmol) in Dioxan (210 ml) und 2-Methoxyethanol (140 ml) wurde mit 4-DMAP (1,2 g; 10 mmol) und N-Jodsuccinimid (25 g; 111 mmol) versetzt und über Nacht bei 50°C gerührt. Einengen ergab 44,4 g eines braungelben Breis (Theorie; 28 g), der ohne weitere Reinigung weiter umgesetzt wurde.

Zur Charakterisierung wurden 2 g des Rohprodukts mit Essigester angelöst und mit 5%-NaHCO₃-Lösung sowie gesättigter Kochsalzlösung gewaschen. Trocknen und Einengen der organischen Phasen ergab 1,2 g eines gelben Feststoffs [97% der Theorie; Fp: 135-40°C (Zers.); R_{f}: 0,24 (Methylenchlorid:Methanol = 10:1)].

### Beispiel 29

### 2-Butyl-5-formyl-4-jod-imidazol

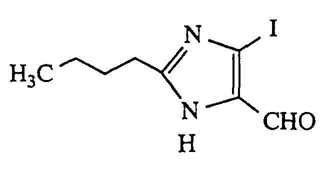

Eine Lösung von Beispiel 28 (42 g roh; 95 mmol) in Essigester (90 ml) wurde mit einer Lösung von Ammoniumcemitrat (125 g; 228 mmol) in Wasser (320 ml) versetzt und über Nacht gerührt. Die wäßrige Phase wurde dreimal mit Essigester extrahiert, dann mit NaHCO₃ alkalisch gestellt und nochmals mit Essigester extrahiert. Die vereinten organischen Phasen wurden mit 5%-NaHCO₃-Lösung und gesättigter Kochsalzlösung gewaschen, getrocknet und eingeengt. Kieselgelchromatographie des Rückstands (Hexan/Essigester-Stufengradient) ergab 19,2 g eines gelben Feststoffs [72% der Theorie; Fp: 80-5°C; R_{f}: 0,54 (Methylenchlorid:Methanol = 10:1)].

### Beispiel 30

### (2-Hxdroxy-phenyl)-glycinol-Hydrochlorid

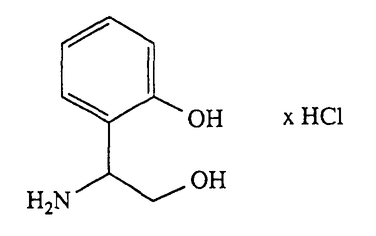

2,18 g (10 mmol) (2-Hydroxy-phenyl)-glycin-methylester-hydrochlorid werden in 20 ml trockenem THF gelöst und bei Raumtemperatur (25°C) 18 Stunden mit 3,34 g (33 mmol) Triethylamin und 2,39 g (22 mmol) Trimethylchlorsilan umgesetzt. Der entstandene Niederschlag wird abgesaugt, mit trockenem THF gewaschen und das Filtrat bei 25°C mit Lithiumalanat (0,76 g/38,0 mmol) umgesetzt. Darauf saugt man überschüssiges Reagenz ab, wäscht mit trockenem THF nach, versetzt mit Wasser und verdünnt mit Ether (pH ≈10). Die wäßrige Phase wird mit 2 M Salzsäure auf pH = 2 gestellt, mit Ether gewaschen und lyophilisiert. Ausbeute 1,20 g (6,3 mmol).
R_{f} = 0,38 (Laufmittel: BABA*)
* Herstellung des Laufmittels BABA:
200 ml n- Butylacetat, 36 ml n-Butanol, 100 ml Eisessig und 60 ml Puffer (87,9 ml 1/15 M wäßriger Kaliumdihydrogenphosphatlösung und 12,1 ml 1/15 M wäßriger Dinatriumhydrogenphosphatlösung) werden geschüttelt und die organische Phase als Laufmittel eingesetzt.

In Analogie zur Vorschrift des Beispiels 30 werden die in Tabelle I aufgeführten Verbindungen hergestellt.

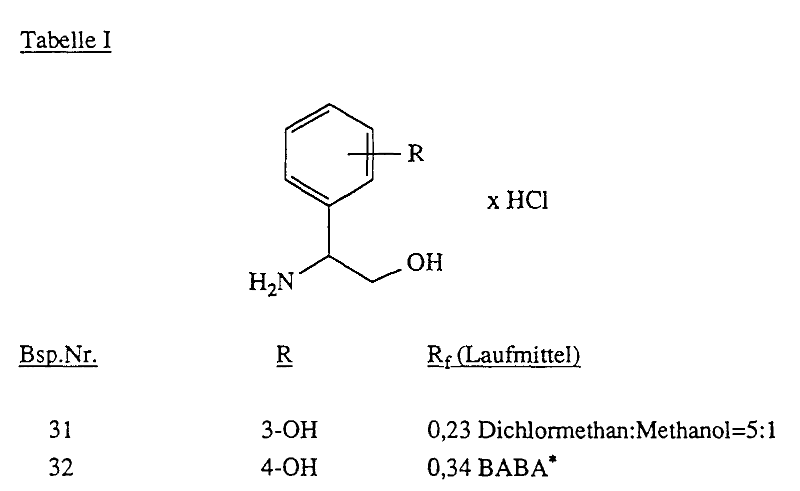

### Beispiel 33

### 2-[4-(2-Butyl-4-chlor-5-formyl-imidazol-1-yl-methyl)phenyl]-essigsäuremethylester

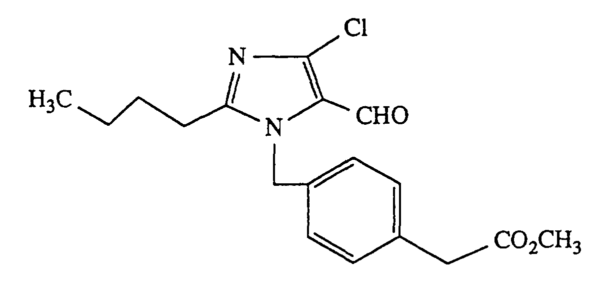

Die Verbindung wurde in Analogie zum Verfahren des Beispiels 26 hergestellt.

### Herstellungsbeispiele

### Beispiel I

### 2-[4-(2-Butyl-4-chlor-5-formyl-imidazol-1-yl-methyl)phenyl]-2-cyclopentylessigsäure-tertbutylester

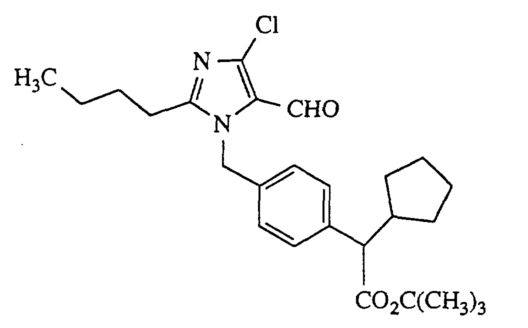

Unter Schutzgas werden 1,6 g (0,053 mol) Natriumhydrid (80%ig) in 50 ml DMF suspendiert, 10 g (0,053 mol) 2-Butyl-5-formyl-4-chlorimidazol (Herstellung nach EP 324 377) in 100 ml DMF bei 0°C zugetropft, anschließend bei 0°C 15 min gerührt und 18,9 g (0,053 mol) 2-(4-Brommethylphenyl)-2-cyclopentylessigsäure-tert.-butylester in 100 ml DMF zugetropft. Es wird 2 h bei 0°C nachgerührt, das Lösemittel abgedampft, der Rückstand in Diethylether aufgenommen, abfiltriert und nach Einengen über Kieselgel 60 mit Dichlormethan chromatographiert.
Ausbeute: 16,2 g (66,7% der Theorie)
Festpunkt: 101-102°C

### Beispiel II

### 2-[4-(2-Butyl-4-chlor-5-formyl-imidazol-1-yl-methyl)phenyl]-2-cyclopentylessigsäure

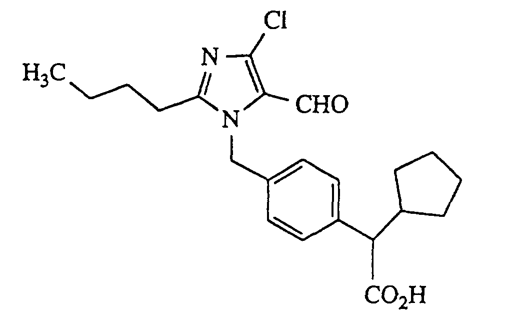

2,3 g (5 mmol) der Verbindung aus Beispiel I werden in 5 ml Dichlormethan und 5 ml Trifluoressigsäure 5 h bei 25°C gerührt. Nach Einengen (wird das Rohprodukt über Kieselgel 60 mit Dichlormethan/Methanol (100:5) chromatographiert.
Ausbeute: 1,8 g (87,6% der Theorie)
Festpunkt: 95-98°C

### Beispiel III

### N-4-Tolylsulfonyl-2-[4-(2-butyl-4-chlor-5-formyl-imidazol-1-yl-methyl)-phenyl]-2-cyclopentyl-essigsäureamid

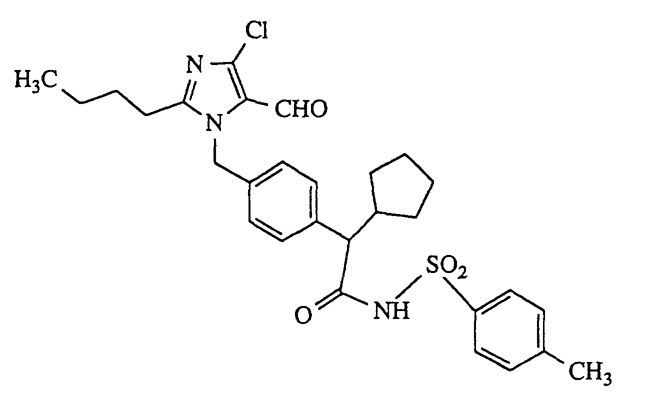

1,2 g (2,6 mmol) der Verbindung aus Beispiel II werden in 30 ml THF gelöst, anschließend werden nacheinander bei 0°C 0,72 mol (5,2 mmol) Triethylamin, 0,23 ml (2,9 mmol) Mesylchlorid, 320 mg (2,6 mmol) DMAP und 535 mg (3,1 mmol) 4-Tolylsulfonamid in 10 ml THF zugegeben. Es wird 20 h bei 25°C gerührt, 0,4 ml Eisessig und 30 ml Wasser zugegeben, dreimal mit 30 ml Essigester extrahiert, die organische Phase eingeengt und der Rückstand über Kieselgel 60 mit Dichlormethan/Methanol (100:2) chromatographiert.
Ausbeute: 1,3 g (90,3% der Theorie)
Festpunkt: 85°C

### Beispiel IV

### N-4-Tolylsulfonyl-2-[4-(2-butyl-4-chlor-5-hydroxymethyl-imidazol-1-yl-methyl)-phenyl]-2-cyclopentylessigsäureamid

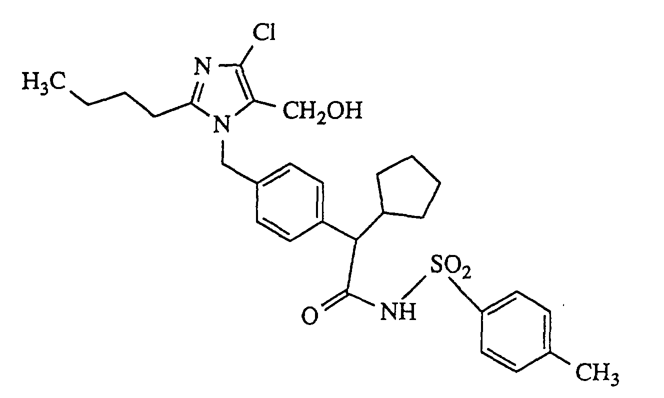

556 mg (1 mmol) der Verbindung aus Beispiel III werden in 10 ml Ethanol gelöst und mit 37,8 mg (1 mmol) Natriumboranat umgesetzt. Nach 15 min werden 20 ml Wasser zugegeben, mit verdünnter Salzsäure angesäuert und zweimal mt 20 ml Essigester extrahiert. Die organische Phase wird über Natriumsulfat getrocknet, eingeengt und über Kieselgel 60 mit Essigester/Petrolether (1:1) chromatographiert.
Ausbeute: 502 mg (90% der Theorie)
Festpunkt: 96°C

### Beispiel V

### 2-[4-(2-Butyl-4-chlor-5-formyl-imidazol-1-yl-methyl)phenyl]-2-cyclopentylessigsäure-L-phenylglycinolamid

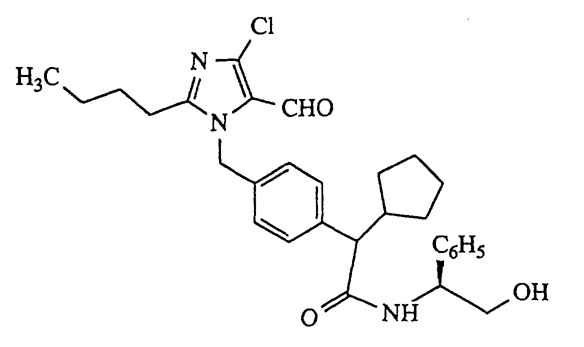

325 mg (0,79 mol) der Verbindung aus Beispiel II werden in 5 ml THF gelöst und bei -30°C mit 0,22 ml (1,58 mmol) Triethylamin und 0,07 ml (0,87 mmol) Mesylchlorid versetzt und 30 min gerührt. Nach Zugabe von 97 mg (0,79 mmol) DMAP, 130 mg (0,95 mmol) L-Phenylglycinol in 5 ml THF wird 20 h bei 25°C gerührt. Nach Zugabe von 10 ml Wasser wird mit 0,15 ml Eisessig angesäuert, dreimal mit 10 ml Eisessig extrahiert, die organische Phase über Natriumsulfat getrocknet, eingeengt und der Rückstand über Kieselgel 60 mit Essigester/Petrolether (1:1) chromatographiert
Ausbeute: 264 mg (64% der Theorie)
Festpunkt: 110°C

### Beispiel VI

### 2-[4[(2-(Carboxy-propyl)-benzimidazol-1-yl)-methyl]phenyl]-2-cyclopropyl-essigsäure-tert.butyleester

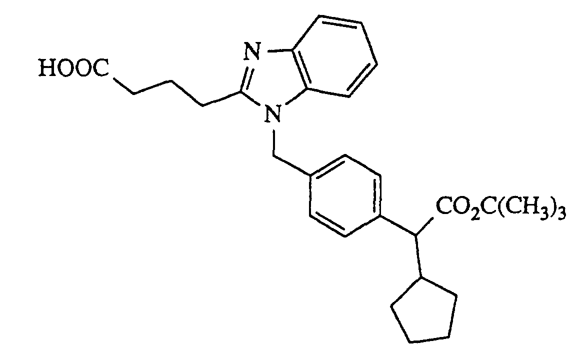

9,7 g (20 mmol) der Verbindung des Beispiels XVI werden in 100 ml Methanol gelöst und über 2 h bei Raumtemperatur mit 40 ml 1 M Natronlauge umgesetzt. Der Alkohol wird abgedampft, das Gemisch mit 100 ml Wasser versetzt und die so erhaltene Lösung mit 2 M Salzsäure auf pH 6 gestellt. Der anfallende Niederschlag wird abgesaugt, mit Wasser gewaschen und im Vakuum über Phosphorpentoxid getrocknet.
Ausbeute: 9,0 g
R_{f}= 0,37 (Dichlormethan:Methanol = 10:1)

In Analogie zur Vorschrift der Beispiele I - VI werden die in Tabelle I und II aufgeführten Beispiele hergestellt:

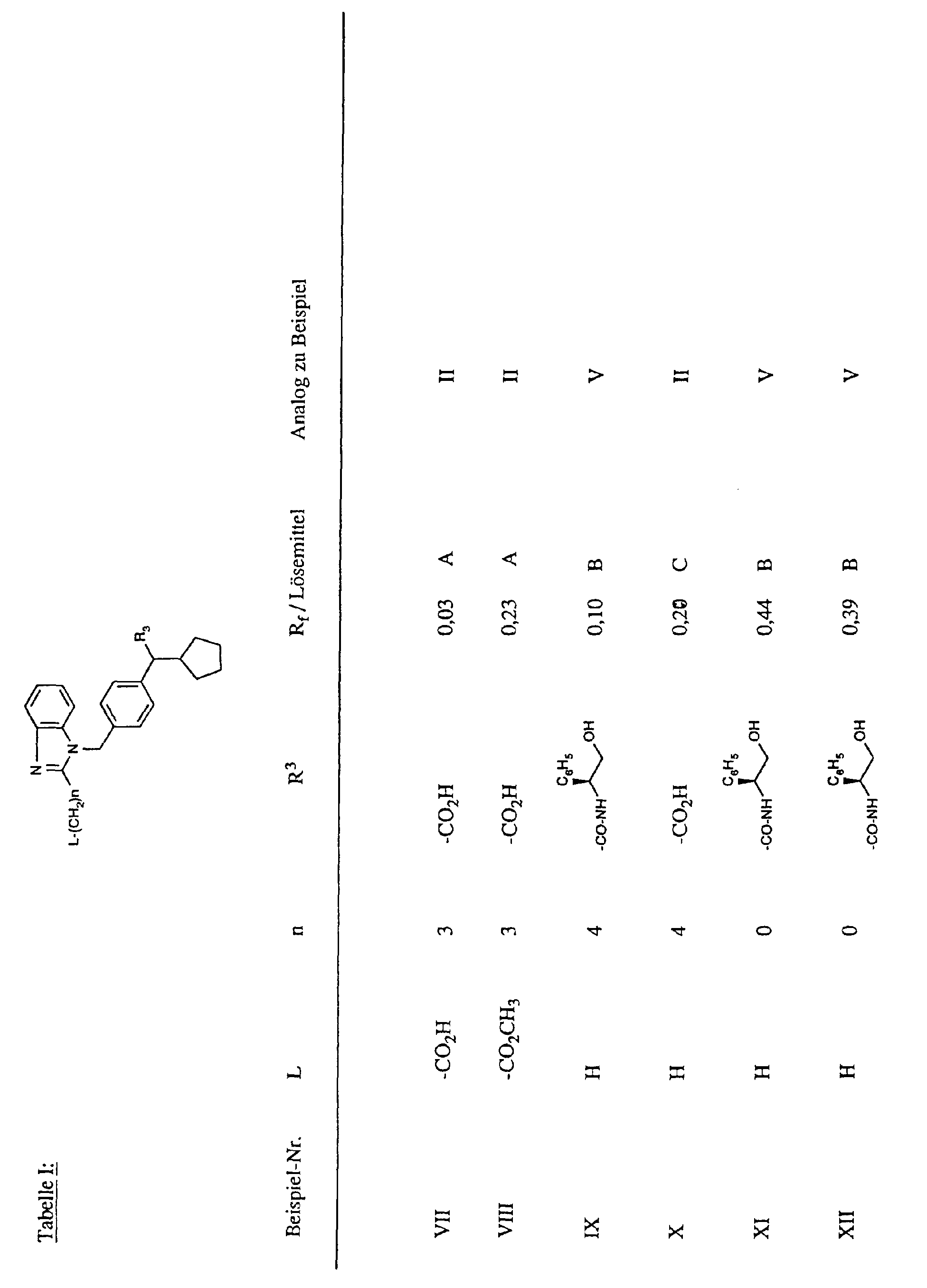

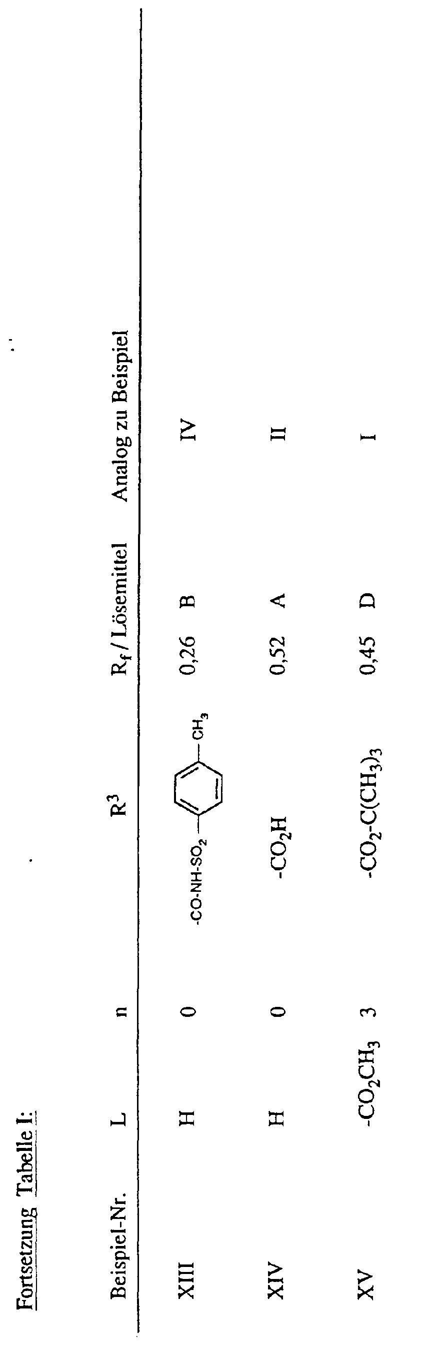

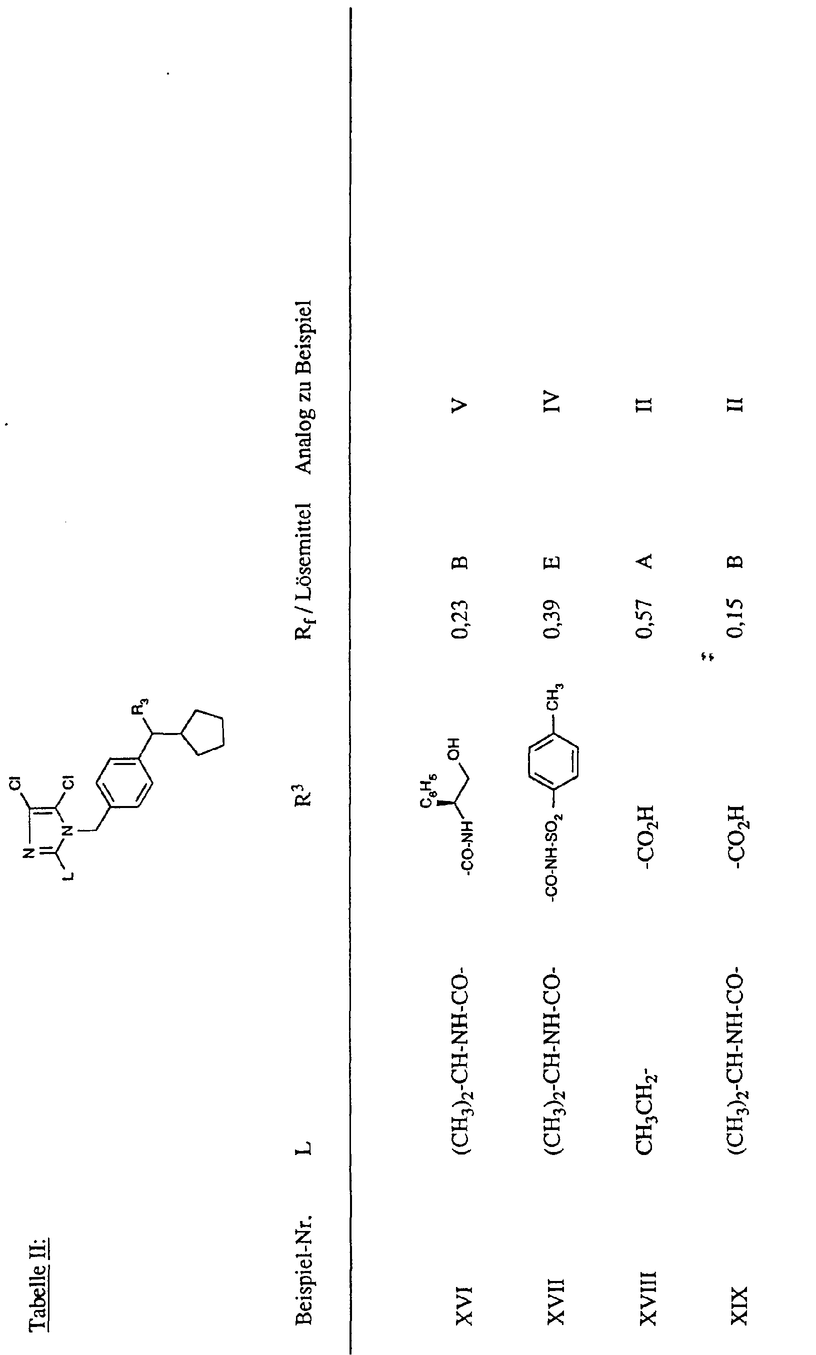

### Beispiel XX

### 2-[4-[3-(2-Butyl-4-chlor-5-formyl-imidazol-1-yl)propyl]phenyl]-2-cyclopentylessigsäure-tert.butylester

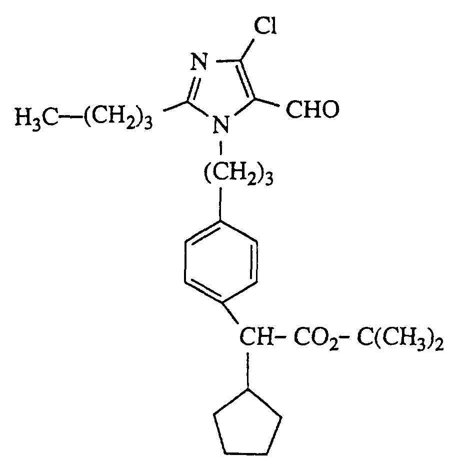

Zu einer Lösung von 47 mg (0,254 mmol) 2-Butyl-4-chlor-5-formyl-imidazol in 259 µl DMF gibt man 8 mg (0,25 mmol) NaH und läßt 0,5 h bei 0°C rühren. Anschließend fügt man 120 mg (0,25 mmol) der Verbindung des Beispiels 10 und 5 mg LiJ zu und läßt 24 h bei Raumtemperatur und rührt anschließend 24 h bei 60°C. Die wäßrige Aufarbeitung liefert ein Rohprodukt, das nach Chromatographie an Kieselgel (Petrolether/Ether 10:1) 46 mg (38%) der Titelverbindung liefert.
R_{f} (Petrolether/Ether 5:1): 0,48.

### Beispiel XXI

### 2-[4-[2-(2-Butyl-4-chlor-5-formyl-imidazol-1-yl)ethyl]phenyl]-2-cyclopentyl-essigsäure-tert.butylester

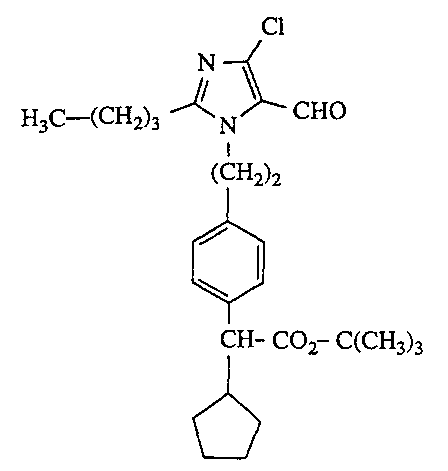

Zu einer Lösung von 43 mg (0,23 mmol) 2-Butyl-4-chlor-5-formyl-imidazol in 250 ul DMF fügt man bei 0°C 6,6 mg (0,22 mmol) einer 80%igen Suspension von NaH in Mineralöl und rührt 1 h bei dieser Temperatur nach. Man fügt 100 mg (0,22 mmol) der Verbindung des Beispiels 7 in 250 ul DMF und 10 mg LiJ zu und rührt 4 h bei 0°C und anschließend 16 h bei Raumtemperatur. Die übliche wäßrige Aufarbeitung lieferte ein Rohprodukt, das nach Chromatrographie an Kieselgel 36 mg (33%) der Titelverbindung liefert.
R_{f} (Petrolether/Ether 5:1): 0,42.

Die in den Tabellen III und IV aufgeführten Verbindungen werden in Analogie zu den dort angegebenen Beispielen hergestellt:

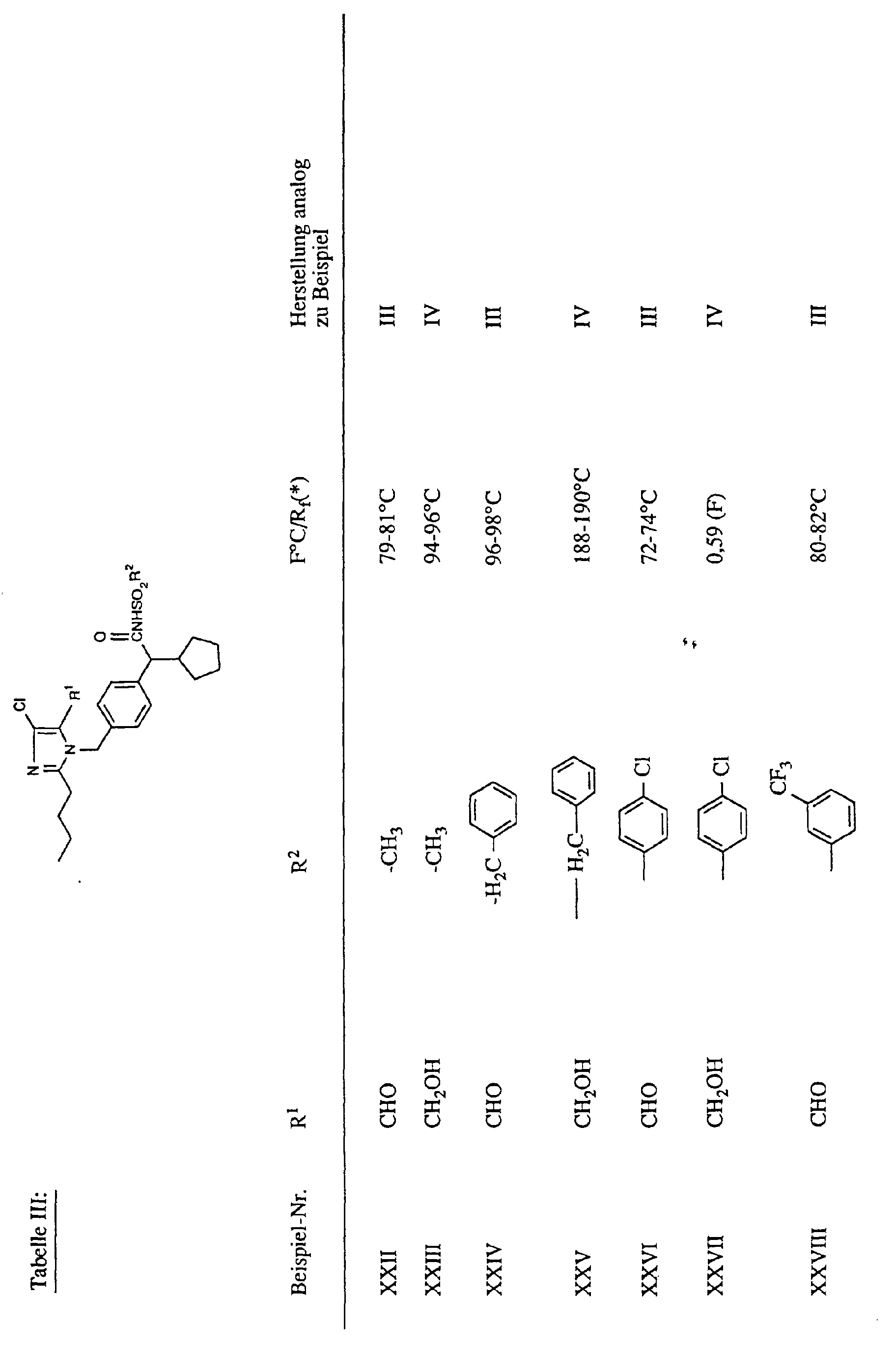

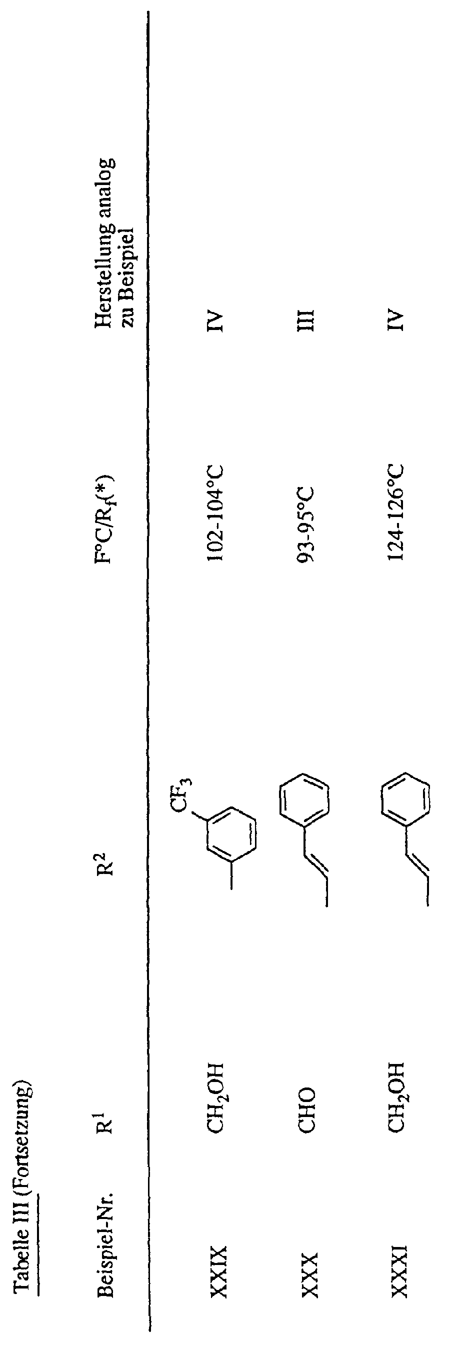

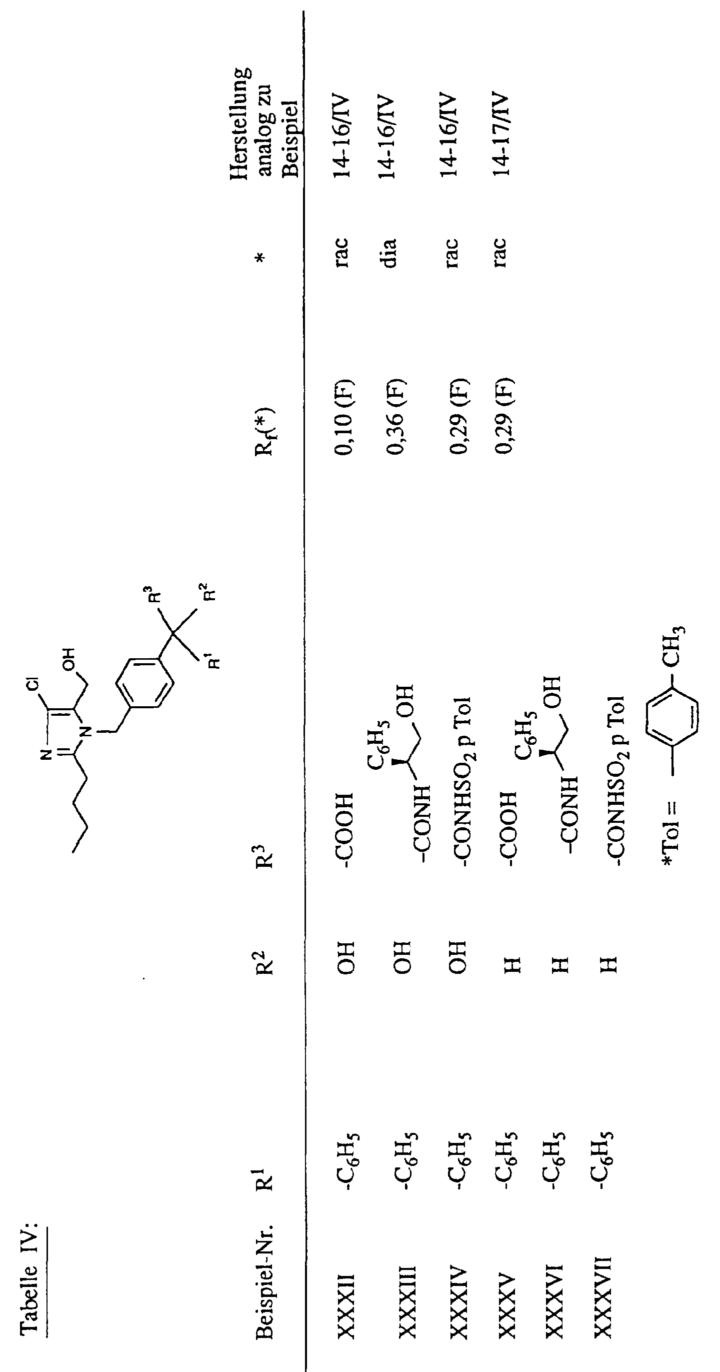

### Beispiel XXXVIII

### 2-[4-(2-Butyl-5-formyl-imidazol-1-yl-methyl)phenyl]-2-cyclopentyl-essigsäure-tert.butylester

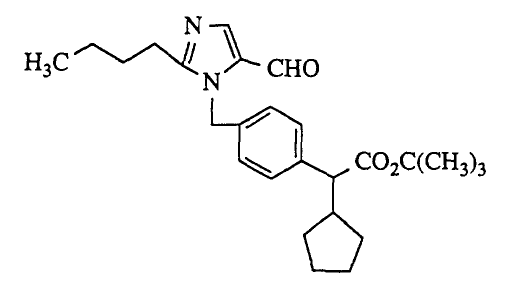

Eine Lösung von 21,8 g (47,5 mmol) 2-[4-(2-Butyl-4-chlor-5-formyl-imidazol-1-yl-methyl)phenyl]-2-cyclopentyl-essigsäure-tert.butylester in 200 ml Methanol wird bei 25°C in Gegenwart von 2,18 g Palladium auf Aktivkohle (5%ig) und 6,47 g (47,5 mmol) Natriumacetat-Trihydrat 1 h bei ca. 2 bar Wasserstoffdruck hydriert. Anschließend wird vom Katalysator abfiltriert, eingeengt und der Rückstand über Kieselgel 60 mit Essigester/Petrolether (1:1) chromatographiert.
Ausbeute: 14 g (70% der Theorie)
R_{f} (Essigester/Petrolether = 1:1): 0,41

### Beispiel XXXIX

### (E,E)-[2-n-Butyl-1-{(1-tert.-butoxycarbonyl-1-cyclopentyl)methylphenyl-4-yl)-methyl}-1H-imidazol-5-yl]-2,4-pentadiensäureethylester

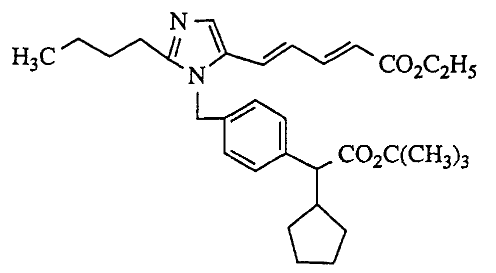

Unter Schutzgas werden 250 mg (10,4 mmol) Natriumhydrid (80%ig) in 20 ml THF suspendiert, 2,18 g (8,7 mmol) 4-Phosponocrotonsäuretriethylester bei 25°C zugetropft, anschließend 1 h bei 25°C gerührt und 2,54 g (6,0 mmol) 2-[4-(2-n-Butyl-5-formyl-imidazol-1-yl-methyl)phenyl]-2-cyclopentylessigsäure-tert.butyl-ester in 10 ml THF zugetropft. Es wird 20 h bei 25°C nachgerührt. Nach Einengen wird der Rückstand zwischen Wasser/Essigester verteilt, die organische Phase über Natriumsulfat getrocknet, eingeengt und der Rückstand über Kieselgel 60 mit Essigester/Petrolether (1:2) chromatographiert.
Ausbeute: 1,5 g (48 % der Theorie)
R_{f} (Essigester/Petrolether = 1:1): 0,55.

### Beispiel XL

### (E,E)-[2-n-Butyl-2-{(1-carboxy-1-cyclopentyl)methylphenyl-4-yl)methyl}-1H-imidazol-5-yl]-2,4-pentadiensäureethylester

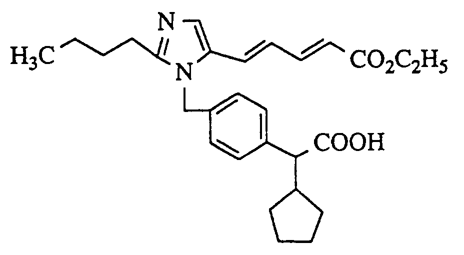

1,44 g (2,77 mmol) der Verbindung aus Beispiel XXXVIII werden analog Beispiel II umgesetzt.
Ausbeute: 1,29 g (100% der Theorie)
R_{f} (Essigester/Petrolether = 1:1): 0,40.

### Beispiel XLI

### 3-[2-n-Butyl-1-((1-tert.-butoxycarbonyl-1-cyclopentyl)methyl-phenyl-4-yl)-methyl}-1H-imidazol-5-yl]-3-hydroxy-2-(2-thienyl)methyl-propionsäuremethylester

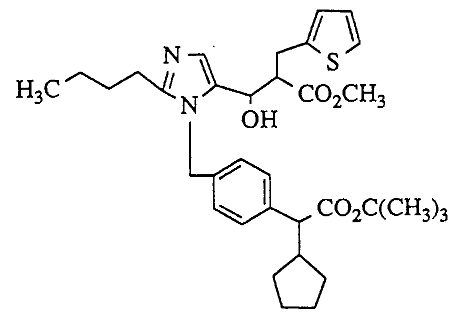

Zu einer Lösung von 1,24 g (12,25 mmol) N,N-Diisopropylamin in 15 ml THF werden unter Schutzgas bei -78°C 7,2 ml einer 1,6 N Lösung von n-Butyllithium in n-Hexan injiziert. Anschließend wird die Reaktionsmischung kurz auf 0°C erwärmt, erneut auf -78°C gekühlt und 1,79 g (10,5 mmol) 3-Thienylpropionsäuremethylester in 5 ml THF zugegeben. Es wird 45 min bei -78°C gerührt, 2,98 g (7,0 mmol) der Verbindung aus Beispiel XLin 5 ml THF zugegeben und 30 min bei -78°C nachgerührt. Danach wird langsam auf 25°C erwärmt, 15 ml ges. Ammoniumchlorid-Lösung zugegeben und dreimal mit je 50 ml Essigester extrahiert. Die organische Phase wird über Natriumsulfat getrocknet, eingeengt und der Rückstand an Kieselgel 60 mit Essigester/Petrolether (4:1) chromatographiert
Ausbeute: 3,0 g (76% der Theorie)
R_{f} (Essigester/Petrolether = 4:1): 0,37.

### Beispiel XLII

### 3-Acetoxy-3-[2-n-butyl-1- {1-tert.-butoxycarbonyl-1-cyclopentyl)methylphenyl-4-yl)methyl}-1H-imidazol-5-yl]-2-(2-thienyl)methyl-propionsäuremethylester

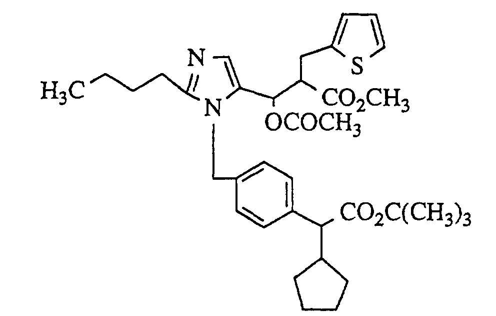

3,0 g (5,0 mmol) der Verbindung aus Beispiel XLI werden in 70 ml Dichlormethan gelöst. Anschließend werden nacheinander 220 mg (1,8 mmol) N,N-Dimethylaminopyridin (DMAP) und 8,0 g (7,95 mmol) Acetanhydrid zugegeben und 2 h bei 25°C gerührt. Es werden 150 ml Ether zugegeben, nacheinander mit je 25 ml ges. Natriumhydrogencarbonat-Lösung und ges. Natriumchlorid-Lösung gewaschen, über Natriumsulfat getrocknet, eingeengt und an Kieselgel 60 mit Essigester/Petrolether (1:1) chromatographiert.
Ausbeute: 2,14 g (67% der Theorie)
R_{f} (Essigester/Petrolether = 1:1): 0,34.

### Beispiel XLIII

### (E)-3-[2-n-Butyl-1-{(1-tert.-butoxycarbonyl-1-cyclopentyl)methylphenyl-4-yl)-methyl}-1H-imidazol-5-yl]-2-(2-thienyl)methyl-2-propensäuremethylester

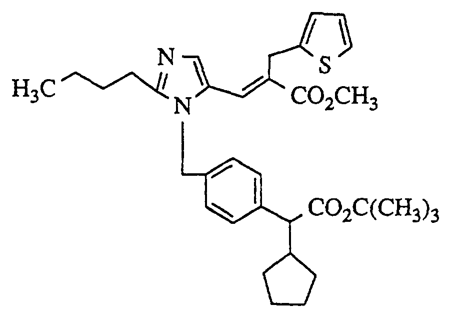

2,14 g (3,4 mmol) der Verbindung aus Beispiel XLII werden in 30 ml Toluol gelöst. Anschließend werden 1,28 g (8,4 mmol) 1,8-Diazabicyclo[5.4.0]undec-7-en (DBU) zugegeben und 3,5 h bei 80°C gerührt. Nach dem Abkühlen wird in Toluol/H₂O aufgenommen, die organische Phase mit ges. Natriumchlorid-Lösung gewaschen, über Natriumsulfat getrocknet, eingeengt und der Rückstand über Kieselgel 60 mit Essigester/Petrolether (1:1) chromatographiert.
Ausbeute: 1,45 g (75% der Theorie)
R_{f} (Essigester/Petrolether = 1:2): 0,36.

### Beispiel XLIV

### (E)-3-[2-n-Butyl-1-{(1-carboxy-1-cyclopentyl)methylphenyl-4-yl)methyl}-1H-imidazol-5-yl]-2-(2-thienyl)methyl-2-propensäuremethylester

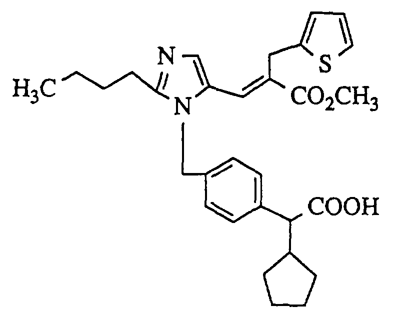

1,39 g (2,4 mmol) der Verbindung aus Beispiel XLIII werden analog Beispiel II umgesetzt.
Ausbeute: 1,25 g (100% der Theorie)
R_{f} (Essigester/Petrolether = 3:1): 0,48.

### Beispiel XLV

### (E)-3-[2-n-Butyl-1-{(1-carboxy-1-cyclopentyl)methylphenyl-4-yl)methyl)-1H-imidazol-5-yl]-2-(2-thienyl)methyl-2-propensäure

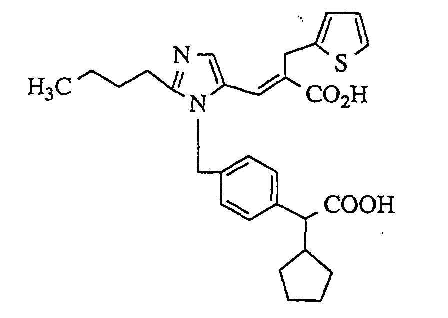

500 mg (0,96 mmol) der Verbindung aus Beispiel XLIV werden in 20 ml Methanol gelöst, dazu werden 5 ml einer 7 N NaOH gegeben und 2 h bei 25°C gerührt. Die Reaktionsmischung wird mit Salzsäure auf pH 1 angesäuert, zweimal mit je 20 ml Essigester extrahiert, die organische Phase über Natriumsulfat getrocknet, eingeengt und über Kieselgel 60 mit Essigester/Methanol (30:1) chromatographiert.
Ausbeute: 150 mg (31% der Theorie)
R_{f} (Essigester/Methanol = 10:1): 0,66.

### Beispiel XLVI

### (E)-3-[2-n-Butyl-1-{(1-cyclopentyl-1-L-phenylglycinolcarbamoyl)methylphenyl-4-yl)methyl}-1H-imidazol-5-yl]-2-(2-thienyl)methyl-2-propensäuremethylester

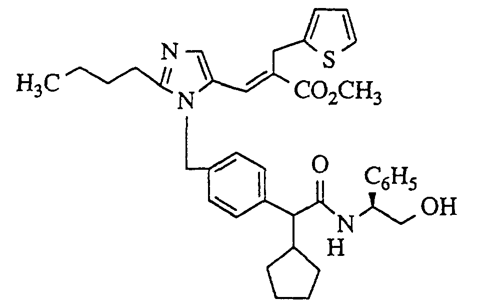

500 mg (0,96 mmol) der Verbindung aus Beispiel XLV werden analog Beispiel V umgesetzt.
Ausbeute: 270 mg (44% der Theorie)
R_{f} (Essigester/Petrolether = 4:1): 0,53.

In Analogie zu Vorschriften der Beispiele XLIII bis XLVI werden die in Tabelle V aufgeführten Beispiele hergestellt:

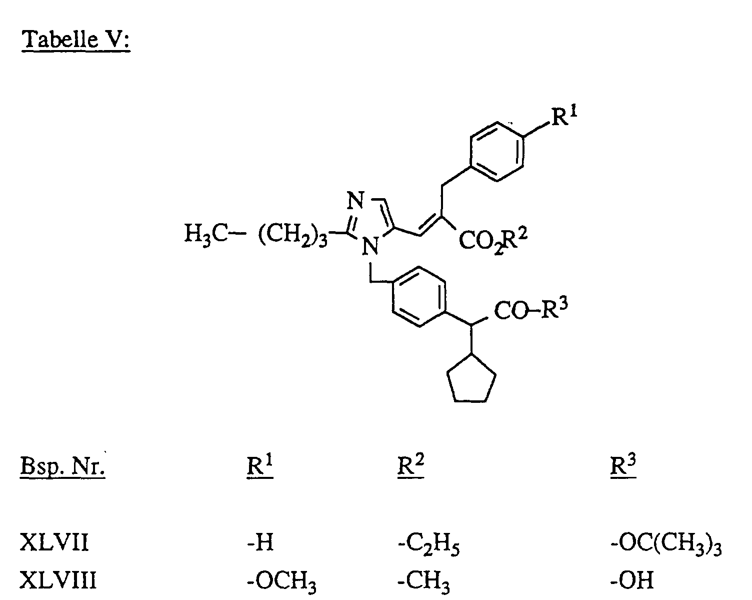

Die in Tabelle VI aufgeführten Beispiele werden in Analogie zu den dort genannten Beispielen hergestellt.

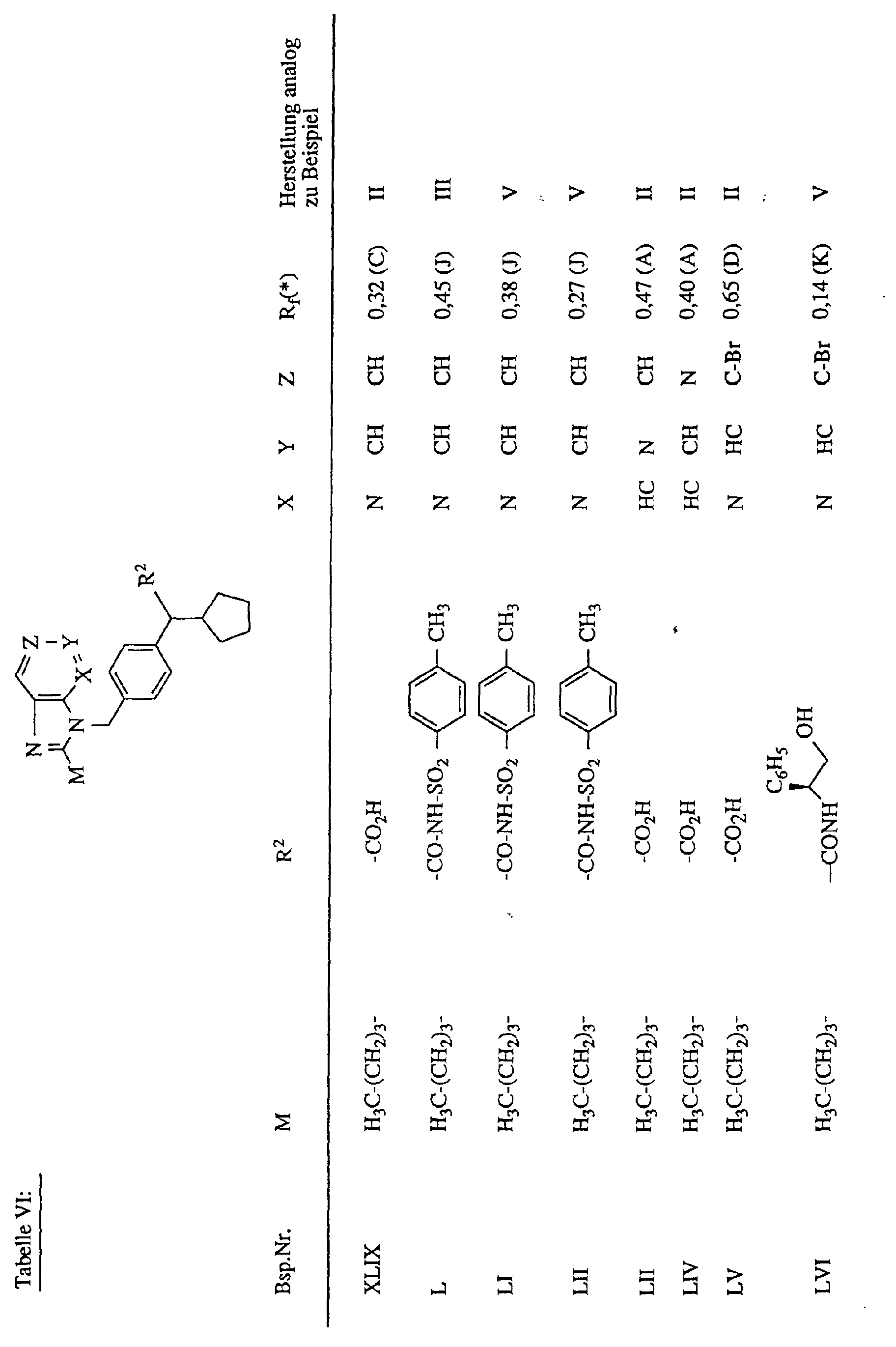

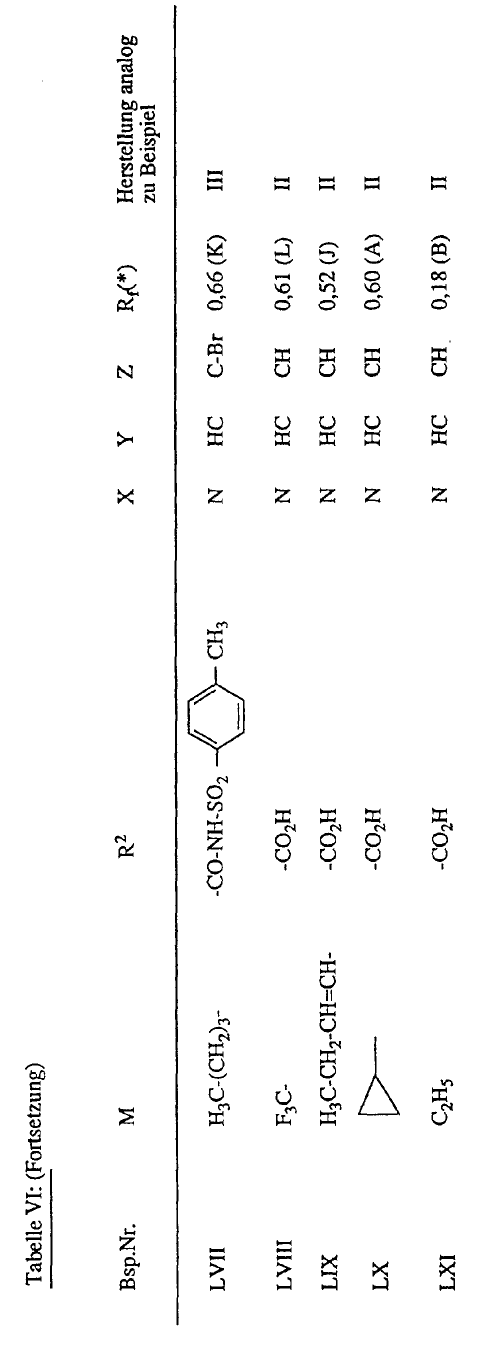

### Beispiele LXII und LXIII

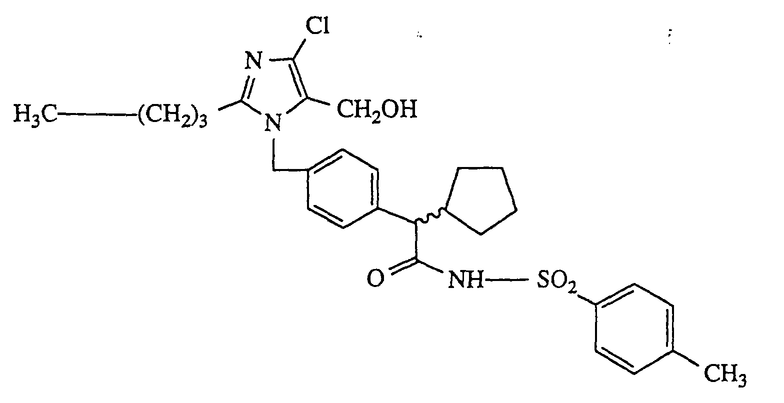

Nach Trennung des Racemates aus Beispiel IV an chiraler Phase werden die beiden Enantiomere
a) (+)-Enantiomer des N-4-Tolylsulfonyl-2-[4-(2-butyl-4-chlor-5-hydroxymethyl-imidazol-1-yl-methyl)phenyl]-2-cyclopentylessisäureamids
   α$\frac{\text{20}}{\text{D}}$ = 99,3 (c = 1, C₂H₅OH) (LXII)
b) (-)-Enantiomer des N-4-Tolylsulfonyl-2-[4-(2-butyl-4-chlor-5-hydroxymethyl-imidazol-1-yl-methyl)phenyl]-2-cyclopentylessigsäureamids
   α$\frac{\text{20}}{\text{D}}$ = -91,4 (c = 1, C₂H₅OH) (LXIII).

### Beispiel LXIV und LXV

### 2-[4-(2-Butyl-4-chlor-5-hydroxymethyl-imidazol-1-yl-methyl)phenyl]-2-cyclopentylessigsäure-L-phenylglycinolamid

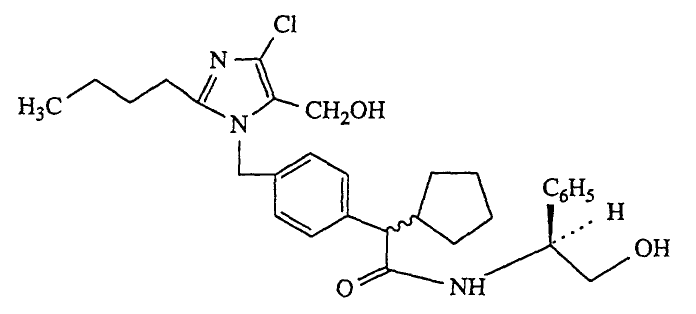

1,5 g (2,9 mmol) der Verbindung aus Beispiel V werden in 25 ml Ethanol mit 0,1 g (2,9 mmol) Natriumboranat versetzt. Nach 1 h bei Raumtemperatur wird das Reaktionsgemisch mit 2 N HCl angesäuert (pH 4) und dreimal mit CH₂Cl₂ extrahiert. Die organische Phase wird über Na₂SO₄ getrocknet, eingeengt und das Produkt aus Essigester/Petrolether kristallisiert. Man erhält ein 1:1-Gemisch der Diastereomere.
Festpunkt: 112-5°C
Ausbeute: 1,0 g (66% der Theorie).

Die Diastereomere werden chromatographisch von Kieselgel mit Petrolether/Essigester (3:7) getrennt.
Diastereomer A: Festpunkt 82-87°C
Diastereomer B: Festpunkt 151-4°C.

### Beispiel LXVI

N-4-Tolylsulfonyl-2-[4-(2-butyl-5-carboxy-4-chlor-imidazol-1-yl-methyl)-phenyl]-2-cyclopentylessigsäureamid

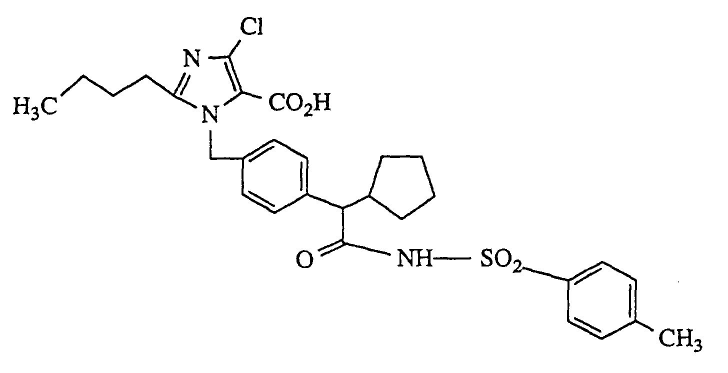

250 mg (0,45 mmol) der Verbindung aus Beispiel III werden in 2,5 ml (0,45 mmol) Pyridin gelöst und bei 60°C mit 162 mg Tetrabutylammoniumpermanganat, gelöst in 1,5 ml Pyridin, versetzt und 2 h bei 60°C gerührt. Das Pyridin wird abgedampft, der Rückstand in Wasser aufgenommen, mit verdünnter HCI angesäuert und mit Essigester extrahiert. Die organische Phase wird eingeengt und der Rückstand an Kieselgel 60 mit CH₂Cl₂/MeOH (10:1) chromatographiert.
Festpunkt: 138°C
Ausbeute: 90 mg (35% der Theorie).

### Beispiel LXVII

### 2-[4-(2-Butyl-4-chlor-5-hydroxymethyl-imidazol-1-yl-methyl)phenyl]-2-cyclopentylessigsäure-tert.butylester

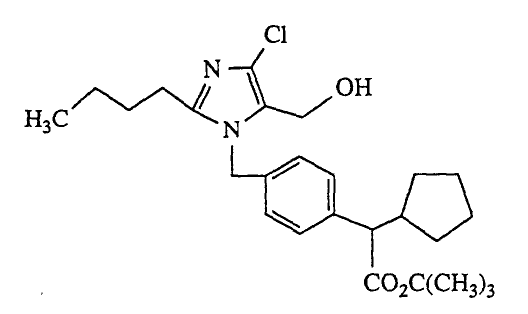

3,2 g (7 mmol) der Verbindung aus Beispiel I werden in 30 ml EtOH mit 265 mg (7 mmol) Natriumboranat versetzt und 1 h bei 25°C gerührt. Nach Zugabe von verdünnter HCl (pH 6) wird dreimal mit CH₂Cl₂ extrahiert, die organische Phase über Na₂SO₄ getrocknet, eingeengt und der Rückstand an Kieselgel 60 mit Petrolether/Essigester (1:1) chromatographiert.
Festpunkt: 112-4°C
Ausbeute: 2,4 g (74,4% der Theorie).

### Beispiel LXVIII

### 2-[4-(2-Butyl-4-chlor-5-hydroxymethyl-imidazol-1-yl-methyl)phenyl]-2-cyclopentylessigsäure

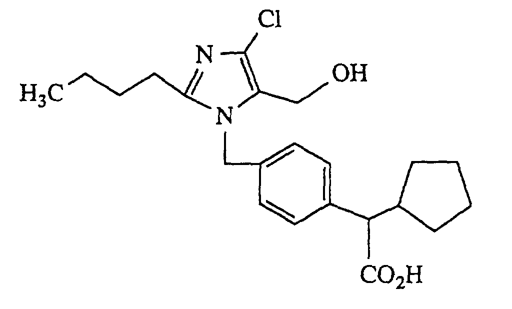

2,1 g (4,5 mmol) der Verbindung aus Beispiel IX werden in 10 ml CH₂Cl₂ und 10 ml Trifluoressigsäure 5 h bei Raumtemperatur gerührt. Das Reaktionsgemisch wird eingedampft. Der Rückstand in CH₂Cl₂ aufgenommen, mit Wasser gewaschen, getrocknet, eingeengt und aus Essigester/Petrolether (1:1) umkristallisiert.
Ausbeute: 0,6 g (33% der Theorie)
Festpunkt: 160-3°C.

### Beispiel LXIX

### 2-[4-(2-Butyl-5-carboxy-4-chlor-imidazol-1-yl-methyl)phenyl]-2-cyclopentyl-essigsäure-tert.butylester

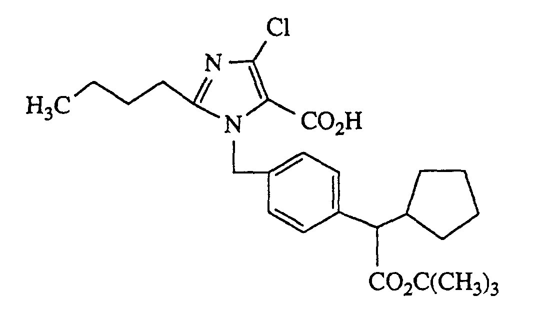

6,9 g (15 mmol) der Verbindung aus Beispiel I werden analog Beispiel XCV mit Tetrabutylammoniumpermanganat oxidiert. Das Produkt wird aus Essigester/Petrolether (1:1) umkristallisiert.
Festpunkt: 75-80°C
Ausbeute: 4,3 g (60,5% der Theorie).

### Beispiel LXX

### 2-[4-(2-Butyl-5-carboxy-4-chlor-imidazol-1-yl-methyl)phenyl]-2-cyclopentylessigsäure

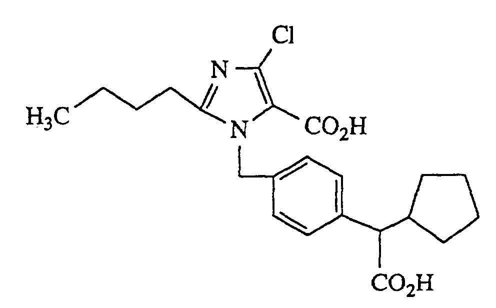

1,2 g (2,5 mmol) der Verbindung aus Beispiel LXIX werden analog Beispiel II mit Trifluoressigsäure umgesetzt. Der Rückstand wird aus Aceton/H₂O umkristallisiert.
Ausbeute: 0,85 g (81,1% der Theorie)
Festpunkt: 196-7°C.

### Beispiel LXXI

### 2-[4-(2-Butyl-4-chlor-5-{N-(2-hydroxy-1(S)-phenyl-ethyl)carbamoyl]-imidazol-1-yl-methyl)phenyl]-2-cyclopentyl-essigsäure-tert.butylester

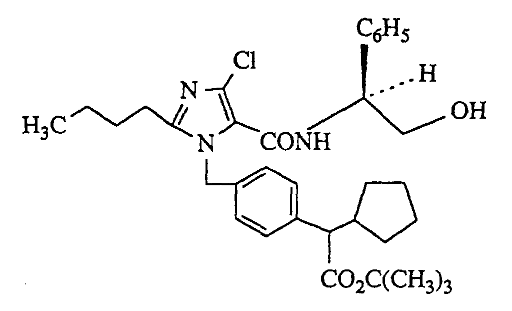

1,4 g (3 mmol) der Verbindung aus Beispiel LXIX werden mit L-Phenylglycinol analog Beispiel V umgesetzt. Das Produkt wird aus Essigester umkristallisiert. Man erhält ein 1:1 -Gemisch der beiden Diastereomere.
Festpunkt: 114-8°C
Ausbeute: 1,3 g (73% der Theorie).

### Beispiel LXXII

### 2-[4-(2-butyl-4-chlor-5-{N-(2-hydroxy-1(S)-phenyl-ethyl)carbamoyl}-imidazol-1-yl-methyl)-phenyl]-2-cyclopentyl-essigsäure

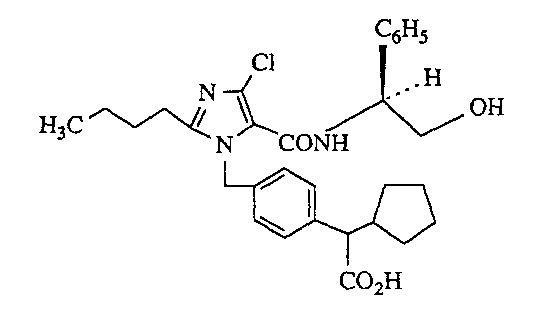

0,8 g (1,35 mmol) der Verbindung aus Beispiel LXXI werden analog Beispiel II mit Trifluoressigsäure umgesetzt. Das Produkt wird aus Ethanol umkristallisiert. Man erhält ein 1:1-Gemisch der Diastereomere.
Festpunkt: 183-189°C
Ausbeute: 0,56 g (77,4% der Theorie).

### Beispiel LXXIII

### 2-Butyl-4-chlor-1-(4-(1-cyclopentyl-1-tetrazol-5-yl)methyl-benzyl)-5-hydroxymethyl-imidazol

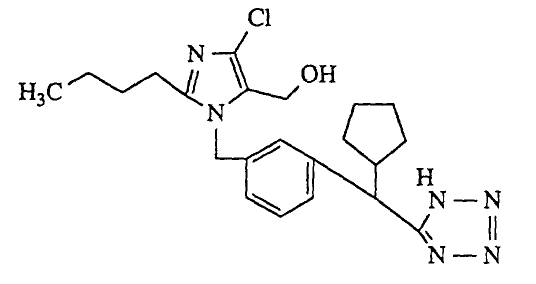

630 mg (1,04 mmol) 2-Butyl-4-chlor-1-(4-(1-cyclpentyl-1-(2-triphenylmethyl-tetrazol-5-yl)-methyl-benzyl)-5-hydroxymethyl-imidazol werden 4, Tage in 5 ml Trifluoressigsäure gerührt (Raumtemperatur). Man stellt mit 2 M Natronlauge auf pH = 13 und schüttelt mit Ether. Die wäßrige Phase wird mit 1 M Salzsäure auf pH = 5 gebracht und der anfallende Niederschlag abgesaugt, mit Wasser gewaschen und im Hochvakuum über Phosphorpentoxid getrocknet.
Ausbeute: 217 mg
R_{f} (Dichlormethan/Methanol = 10:1): 0,31.

### Beispiel LXXIV

### 2-[3-(2-Butyl-4-chlor-5-formyl-imidazol-1-yl-methyl)phenyl]-2-cyclopentyl-essigsäure-L-phenylglycinolamid-O-acetat

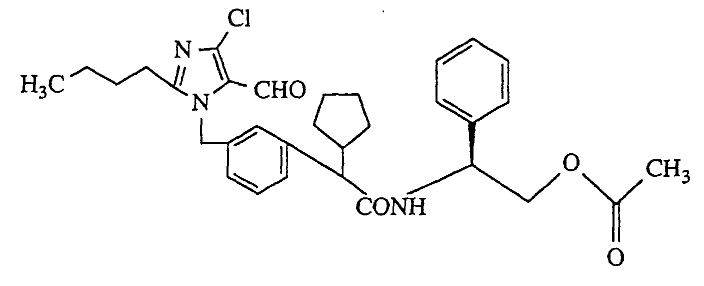

1,7 g (3,02 mmol) 2-[3-(2-Butyl-4-chlor-5-formyl-imidazol-1-yl-methyl)phenyl]-2-cyclopentyl-essigsäure-L-phenylglycinolamid werden bei Raumtemperatur mit 0,34 g (3,3 mmol) Acetanhydrid, 0,46 g (4,5 mmol) Triethylamin und 36 mg (0,3 mmol) 4-(N,N-Dimethylamino)-pyridin verrührt. Nach 4 h wird auf Ether/Wasser gegossen, die organische Phase mit wäßriger Natriumhydrogencarbonatlösung und 1 M Salzsäure gewaschen, getrocknet (Natriumsulfat) und eingedampft. Die chromatographische Aufreinigung (Kieselgel 60, Merck, Dichlormethan:Methanol = 100:1) liefert 670 mg Produkt.
R_{f} (Dichlormethan/Methanol = 20:1): 0,55.

Die in den Tabellen VII, VIII und IX aufgeführten Beispiele werden in Analogie zu den dort genannten Beispielen hergestellt.

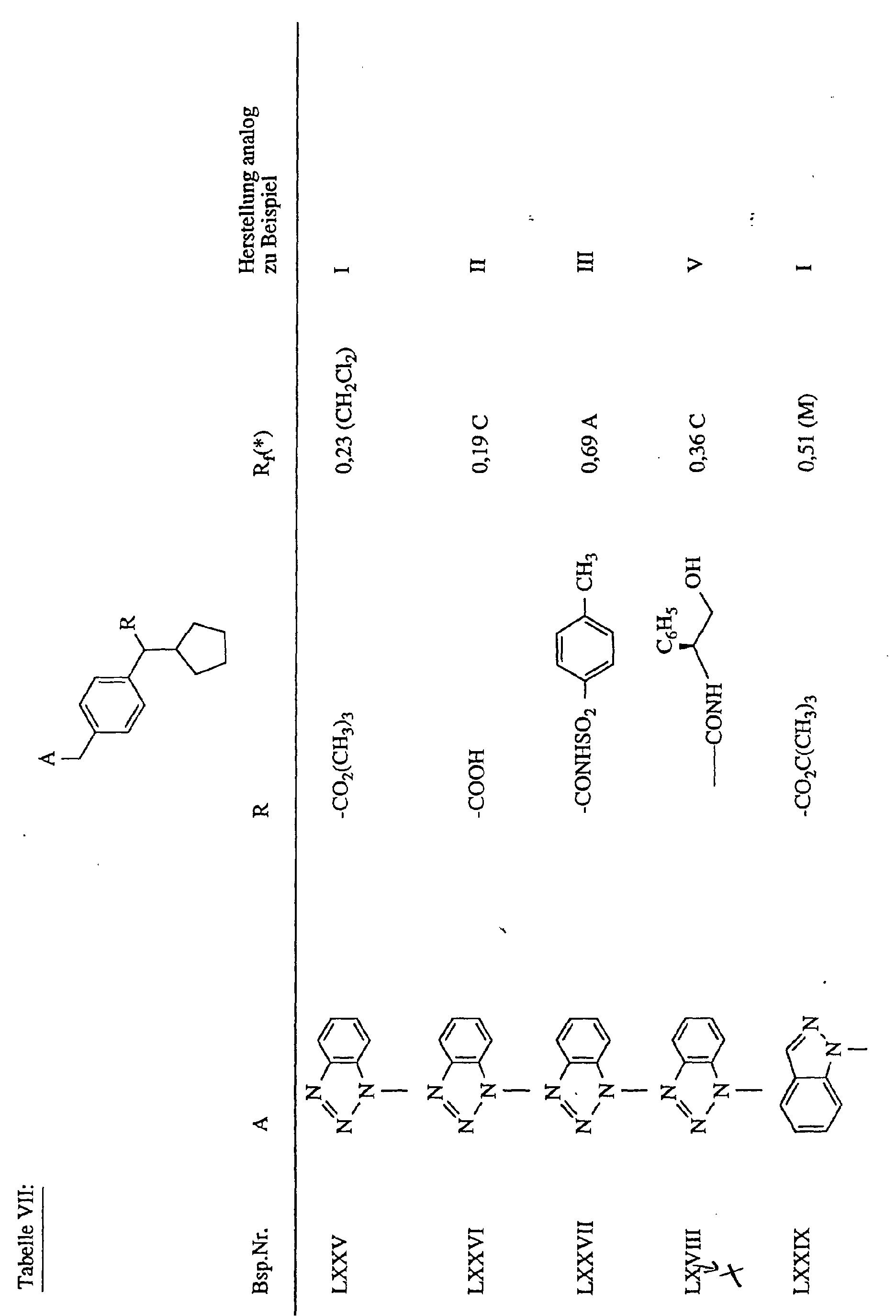

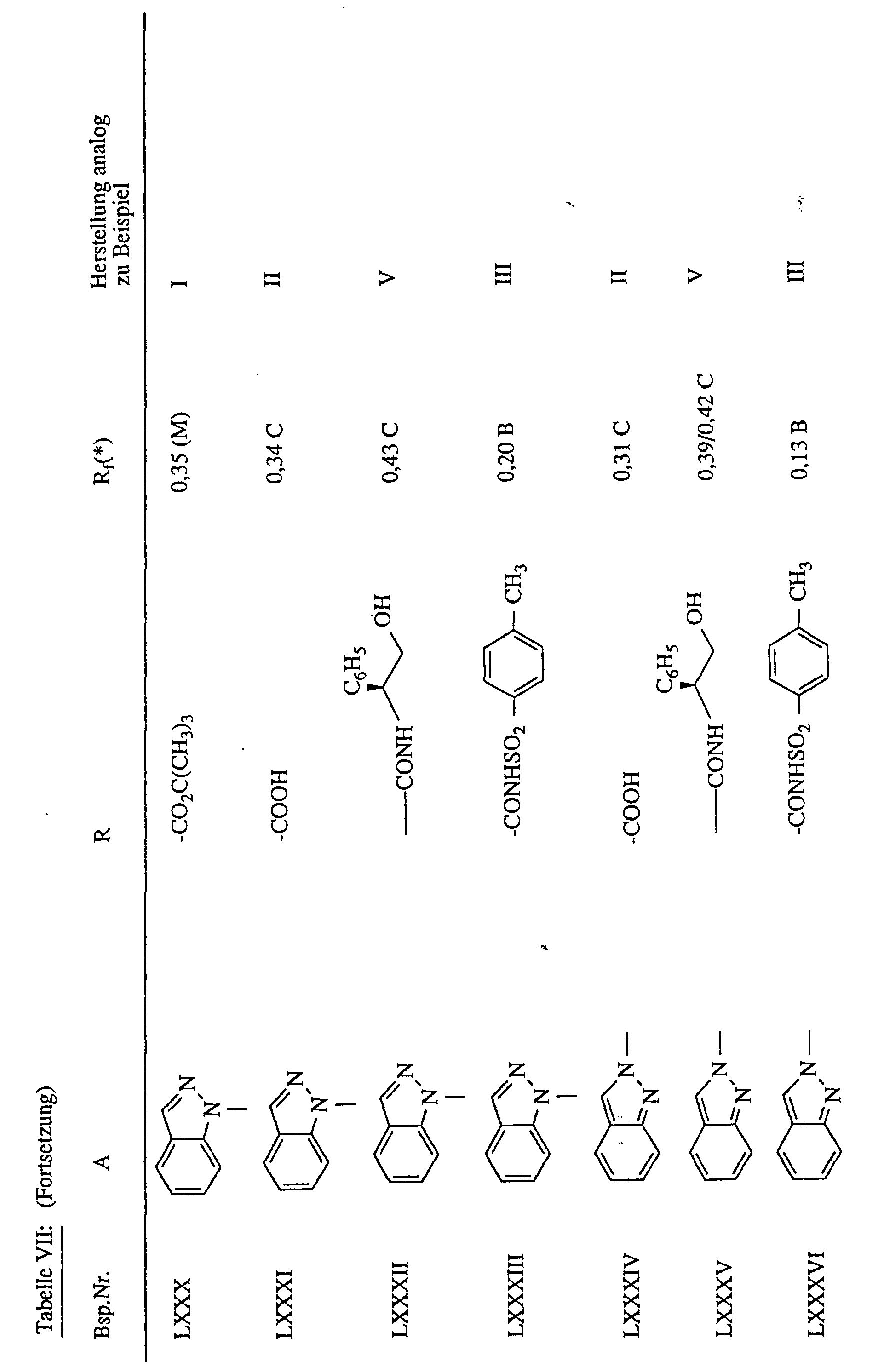

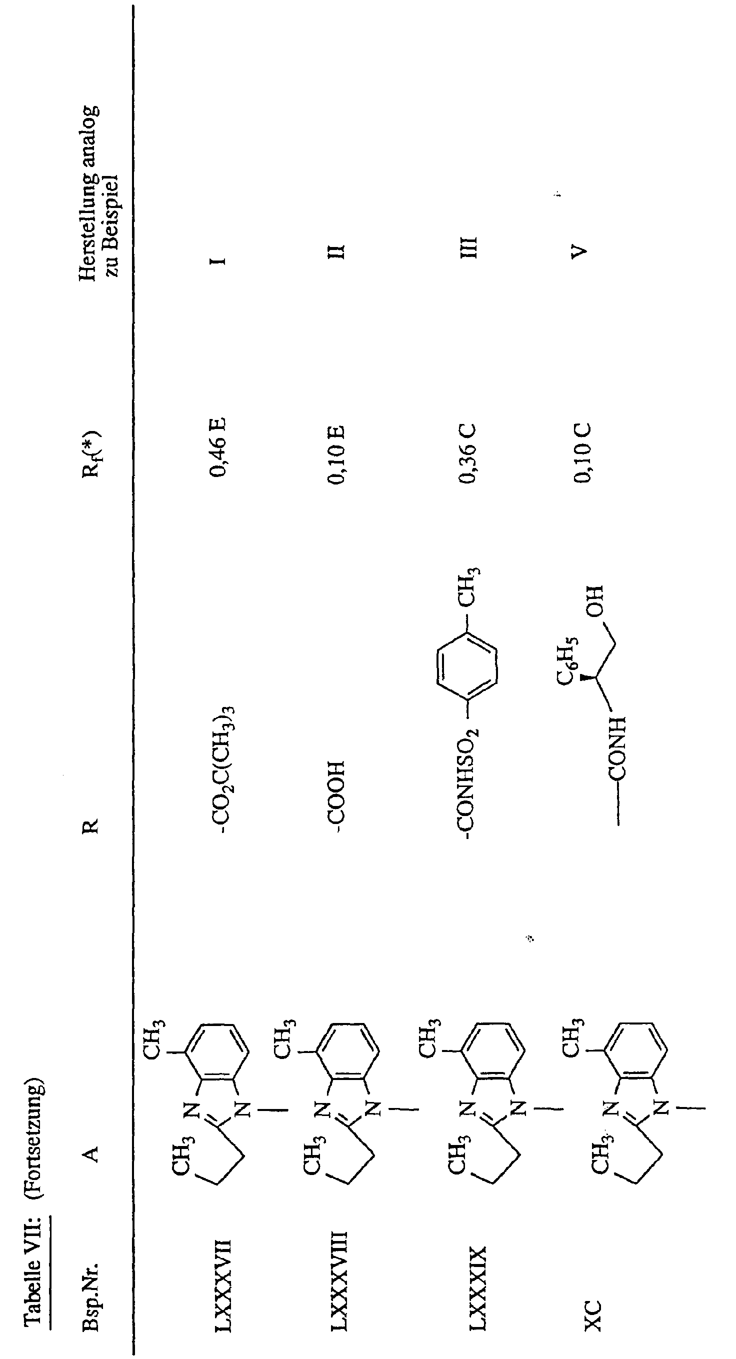

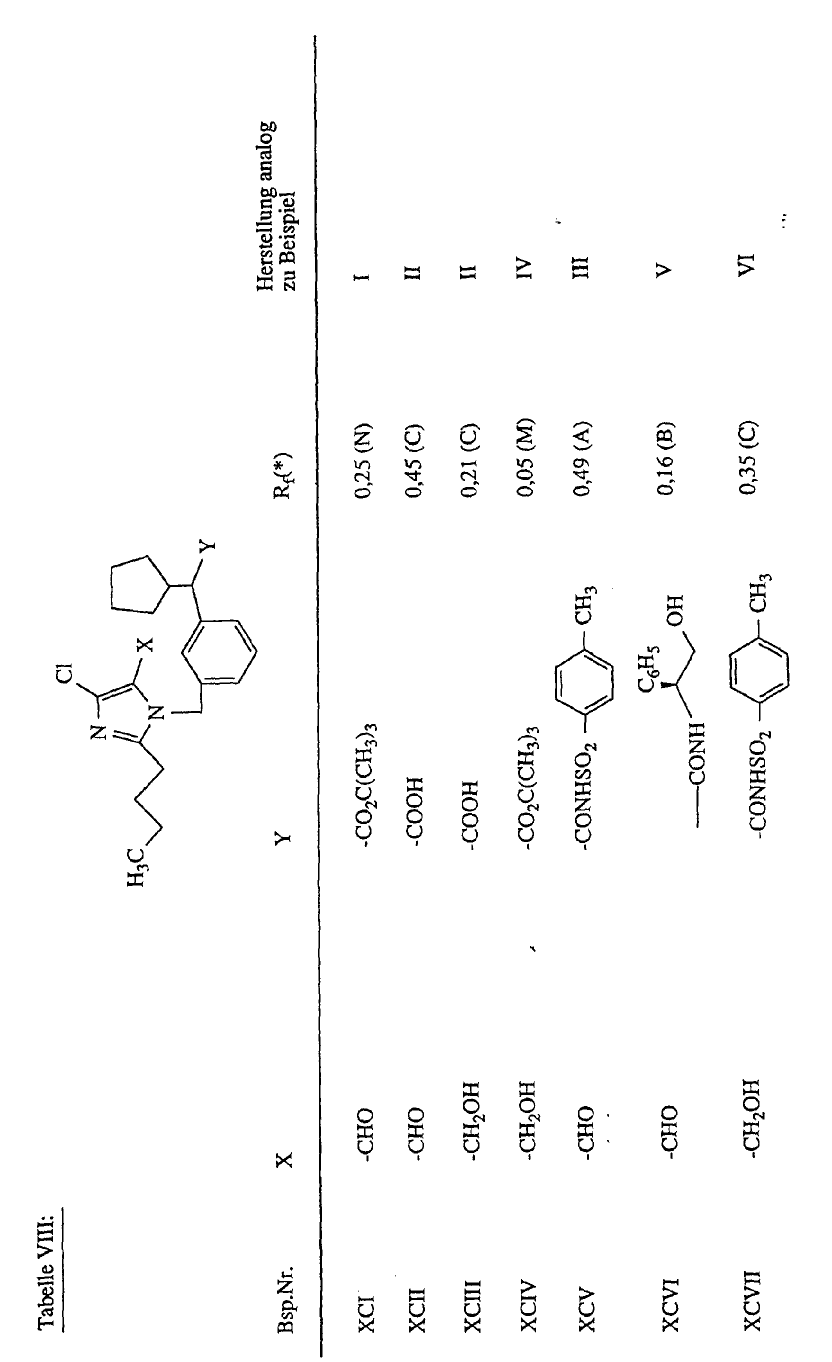

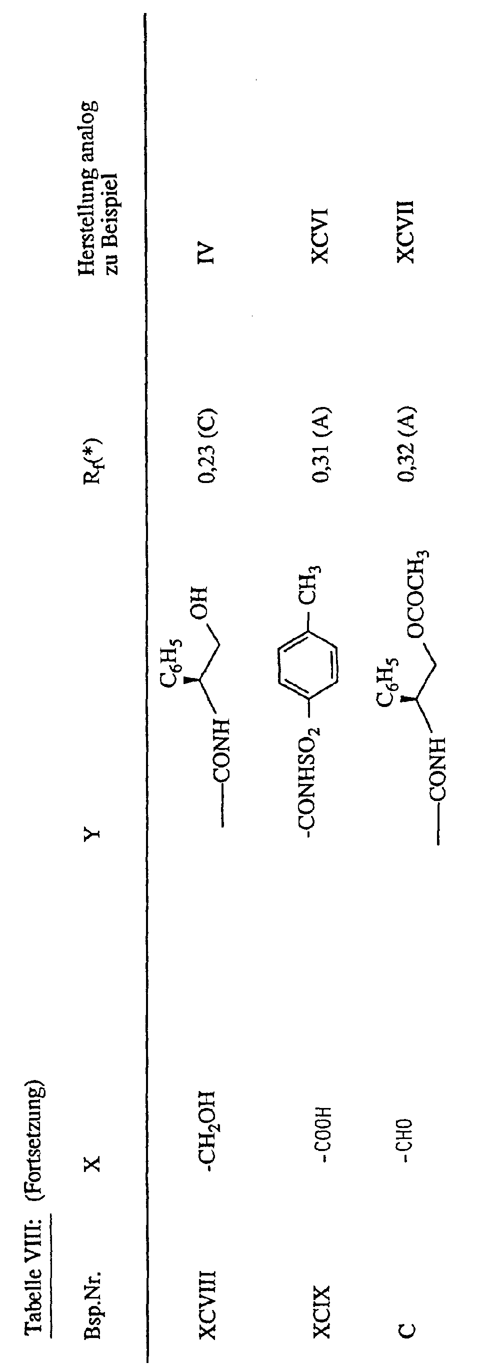

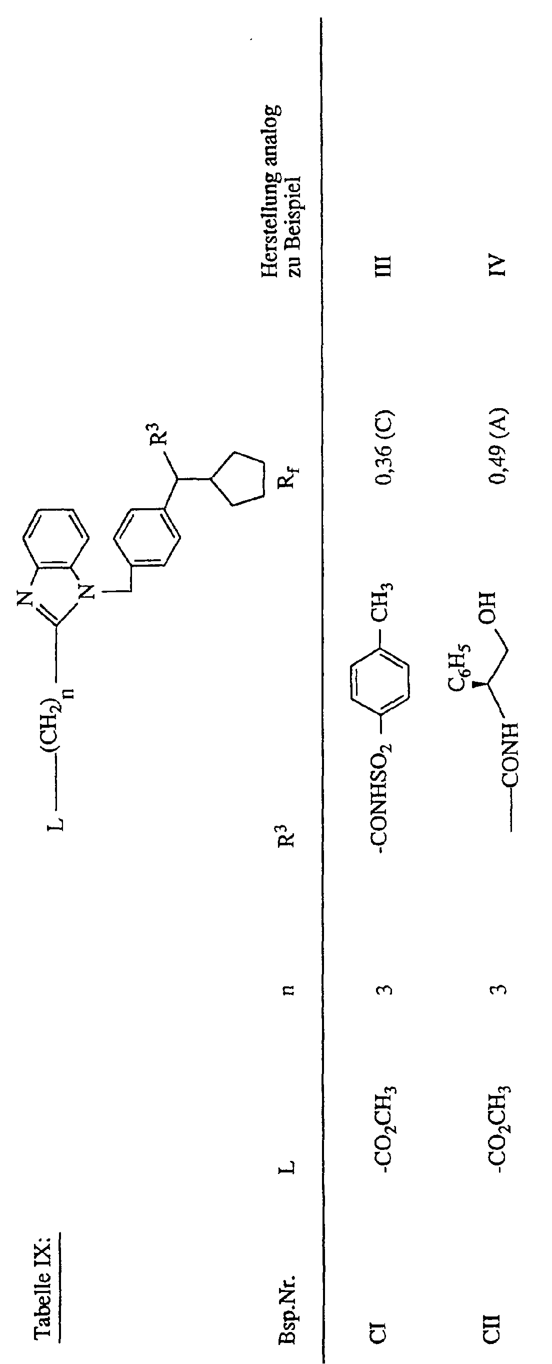

In Analogie zu den Vorschriften der Beispiele II und V werden die in Tabelle X aufgeführten Beispiele hergestellt:

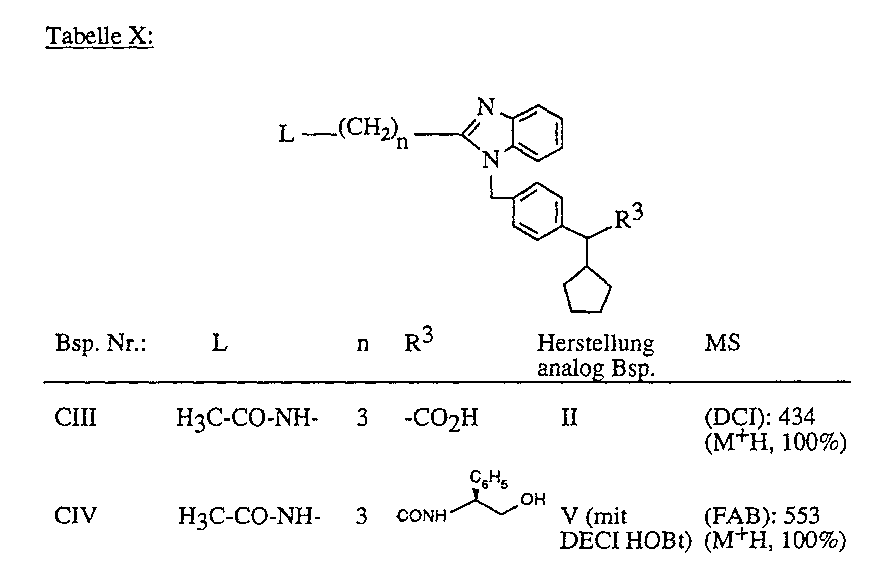

Die in der Tabelle X aufgeführten Beispiele werden in Analogie zu den dort genannten Beispielen hergestellt:

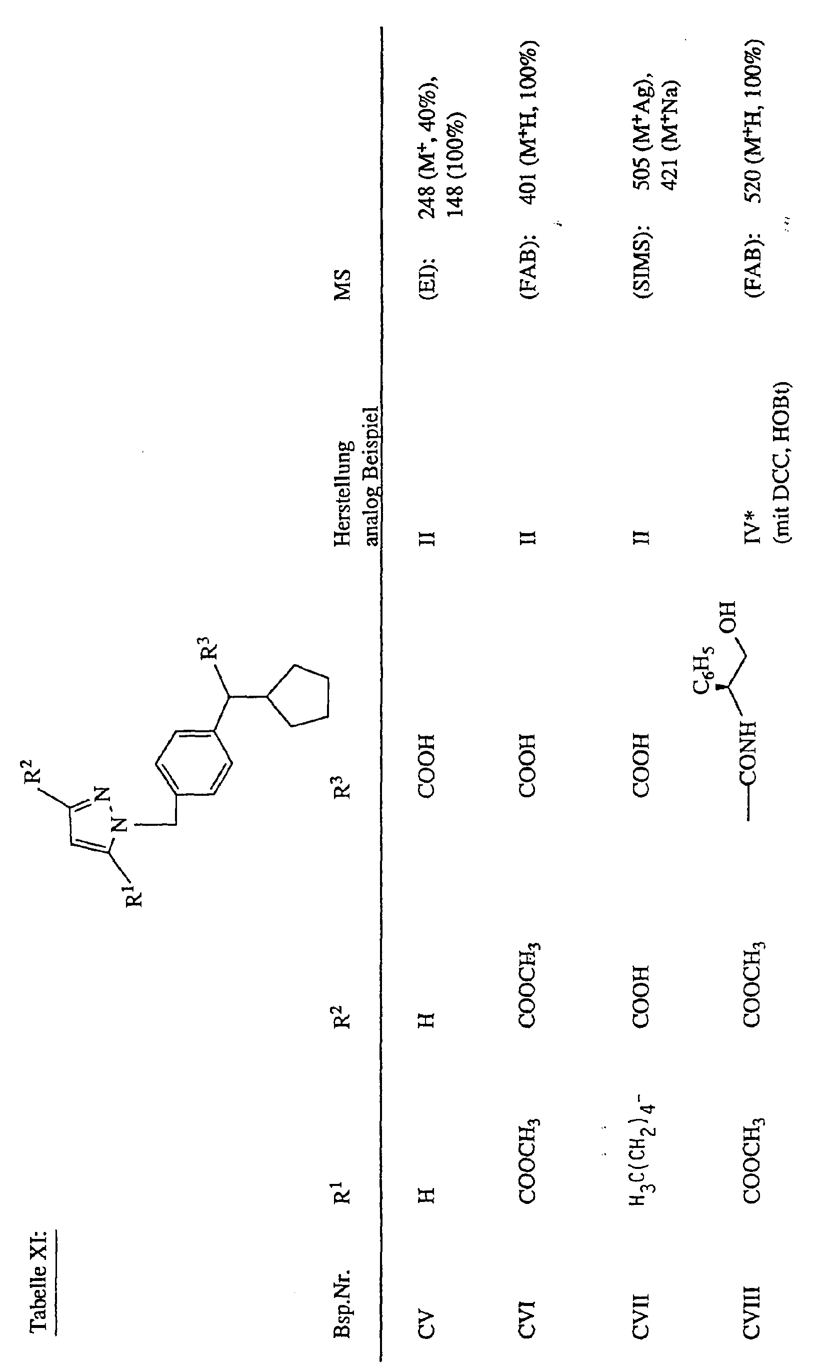

### Beispiel CIX

### 5-{1-Cyclopentyl-1-[4-(2-butyl-4-chlor-5-formyl-imidazol-1-yl-methyl)-phenyl]-methyl}-tetrazol

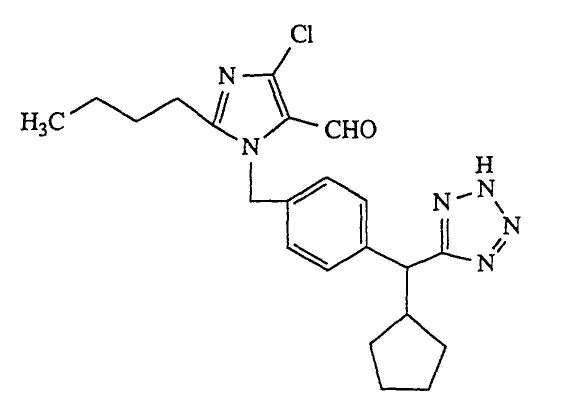

Beispiel 26 (1,7 g; 2,5 mmol) wurde über Nacht in 4N-HCl/Dioxan (160 ml) gerührt, mit 1N-Natronlauge alkalisch gestellt und eingeengt. Der Rückstand wurde zwischen Wasser und Ether verteilt. Die wäßrige Phase wurde sauer gestellt, mit Ether extrahiert. Die organische Phase wurde getrocknet und eingeengt, um 0,89 g eines gelben Öls zu ergebn [83,9% der Theorie; R_{f} 0,29 (Dichlormethan:Methanol = 10:1)].

### Beispiel CX

### 5- {1-Cyclopentyl-1-[4-(2-butyl-4-chlor-5-hydroxymethyl-imidazol-1-yl-methyl)-phenyl]-methyl} -tetrazol

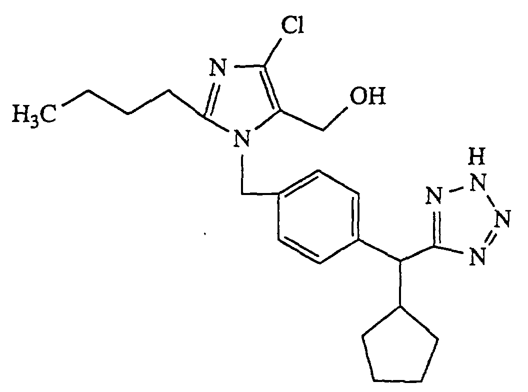

Eine Lösung von Beispiel 20 (400 mg; 0,94 mmol) in Methanol (100 ml) wurde mit Natriumborhydrid (36 mg; 0,94 mmol) versetzt und über Nacht gerührt. Zur Vervollständigung der Reaktion wurde die Lösung auf 5 ml eingeengt, mit der gleichen Menge Natriumborhydrid versetzt und über Nacht gerührt. Nach Einengen wurde der Rückstand mit 1K-KHSO₄-Lösung versetzt, mit Essigester extrahiert. Die organische Phase wurde getrocknet, eingeengt und über HPLC (Licroprep RP18, 35-80% Acetonitril/Wasser/0,05% Trifluoressigsäure) gereinigt, um 72 mg eines weißen Feststoffs zu ergeben (18% der Theorie).

### Beispiel CXI

### 2,2-Dicyclopentyl-2-[4-(2-butyl-4-chlor-5-formyl-imidazol-1-yl-methyl)-phenyl]-acetonitril

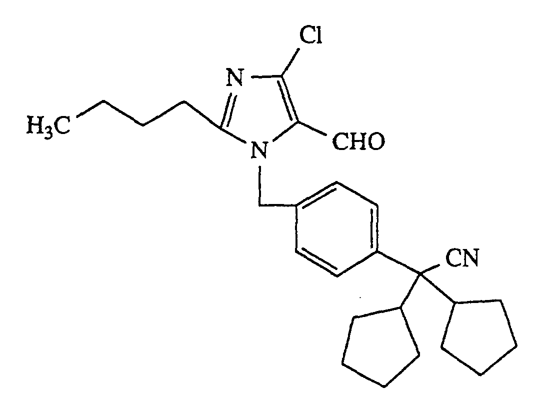

Alkylierung mit Beispiel 27 (0,93 g; 2,7 mmol) analog Beispiel 26 und Kieselgelchromatographie (Toluol/Essigester Stufengradient) ergaben 1,3 g eines gelben Öls [54% der Theorie; R_{f} 0,57(Toluol:Essigester = 5:1)].

### Beispiel CXII

### 2,2-Dicyclopentyl-2-[4-(2-butyl-4-chlor-5-hydroxymethyl-imidazol-1-yl-methyl)-phenyl]-acetonitril

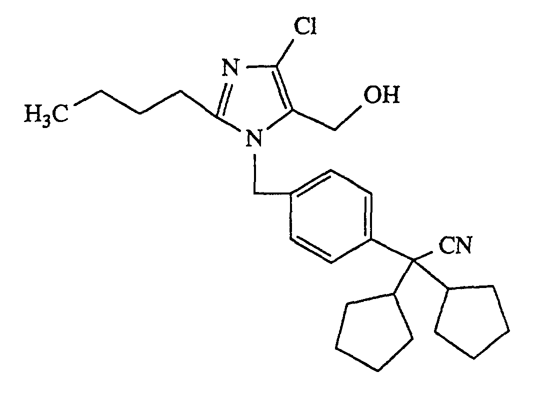

Eine Lösung von Beispiel CXI (100 mg; 0,22 mmol) in Toluol (4 ml) wurde bei 10°C unter Argon tropfenweise mit 1M-Diisobutylaluminiumhydrid/Tetrahydrofuran (0,48 ml) versetzt und bei Raumtemperatur über Nacht gerührt. Nach Zugabe von Wasser (0,5 ml) wurde die Emulsion in intensiv gerührte 5%ige Schwefelsäure gegeben, auf pH 7 gestellt und mit Ether extrahiert. Trocknen und Einengen der organischen Phase, gefolgt von Kieselgelchromatographie (Methylenchlorid:Methanol = 20:1) lieferte 58 mg eines Öls [59% der Theorie; R_{f} 0,44 (Methylenchlorid:Methanol = 20:1)].

### Beispiel CXIII

### 2-{4-[2-Butyl-4-chlor-5-(1-hydroxy)-ethyl-imidazol-1-yl-methyl]-phenyl}-2-cyclopentyl-essigsäure-tert.butylester

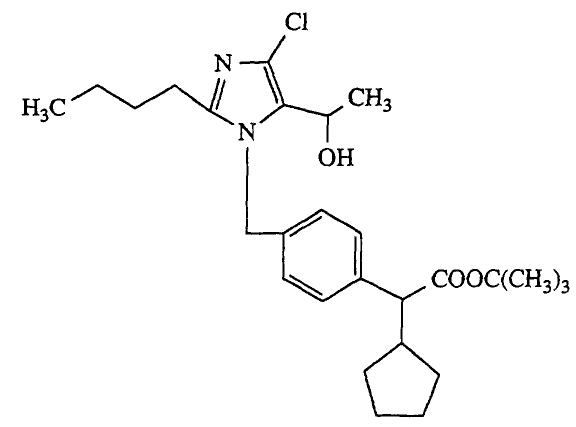

Unter Argon wurde ein Lösung von Beispiel I (230 mg; 0,50 mmol) in Tetrahydrofuran (5 ml) bei -78°C mit 3M-MeMgCl/THF (0,18 ml; 0,55 mmol) versetzt und über Nacht bei Raumtemperatur gerührt. Nach Einengen wurde der Rückstand in Essigester aufgenommen, zweimal mit 5%-NaCO₃-Lösung, dann mit gesättigter Kochsalzlösung gewaschen, getrocknet und eingeengt, um 206 mg eines Öls zu erhalten [93,9% der Theorie; R_{f} 0,10 (Methylenchlorid:Essigester = 10:1)].

### Beispiel CXIV

### 2-{4-[2-Butyl-4-chlor-5-(1-hydroxy)-ethyl-imidazol-1-yl-methyl]-phenyl}-2-cyclopentyl-essigsäure

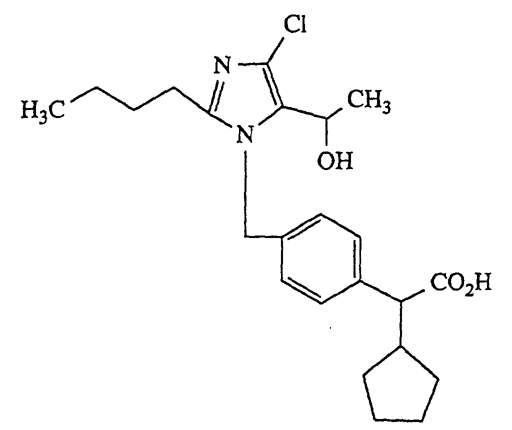

Eine Lösung von Beispiel CXII (356 mg; 0,38 mmol) in Dichlormethan (10 ml) und Trifluoressigsäure (10 ml) wurde 1,5 h bei Raumtemperatur gehalten, dann eingeengt. Der Rückstand wurde mit Wasser versetzt, mit gesättigter Natriumhydrogencarbonat-Lösung auf pH 3 gestellt und mit Ether extrahiert. Trocknen, Einengen und Kiselgelchromatographie (Methylenchlorid:Methanol = 20:1) lieferte 97 mg einer farblosen, amorphen Substanz [29% der Theorie; R_{f} 0,33 (Methylenchlorid:Methanol:Essigsäure = 20:1:0,1)].

### Beispiel CXV

### N-4-Tolylsulfonyl-2-{4-[2-butyl-4-chlor-5-(1-hydroxy)-ethyl-imidazol-1-yl-methyl]-phenyl}-2-cyclopentyl-essigsäureamid

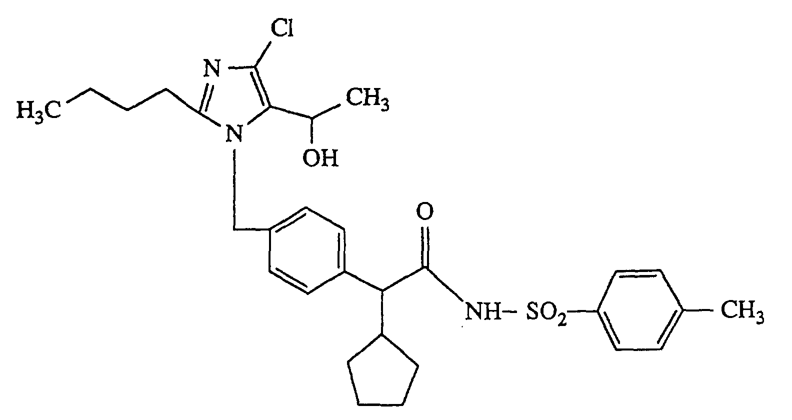

Eine Lösung von Beispiel III (100 mg; 0,18 mmol) in THF (5 ml) wurde bei 0°C unter Argon mit 80% Natriumhydrid/Paraffinöl-Dispersion (6,0 mg; 0,2 mmol) versetzt, 15 min bei Raumtemperatur gerührt, dann bei 0°C mit 3M-MeMgCl/THF (0,1 ml) versetzt und 40 h bei Raumtemperatur gerührt. Die Reaktionslösung wurde mit 1M-KHSO₄-Lösung auf pH 3 angesäuert und dreimal mit Essigester extrahiert. Die organischen Phasen wurden mit gesättigter Kochsalzlösung gewaschen, getrocknet und eingeengt. Kieselgelchromatographie lieferte 36 mg einer teils festen Substanz [36% der Theorie; R_{f} 0,25 (Methylenchlorid:Methanol:konz.Ammoniak-wasser= 10:1:0,1)].

### Beispiel CXVI

### 2-{4-[2-Butyl-4-Chlor-5-(1-hydroxy)-propyl-imidazol-1-yl-methyl]-phenyl}-2-cyclopentyl-essigsäure

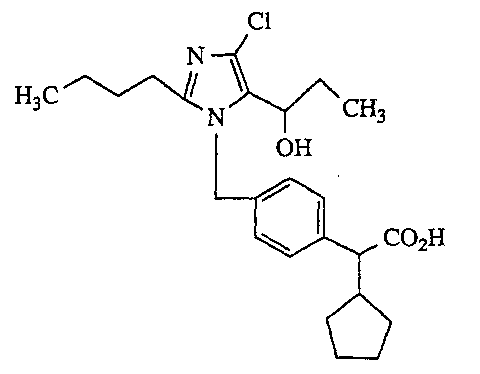

Eine Lösung von Beispiel II (1,13 g; 1,2 mmol) in THF (10 ml) wurde unter Argon bei -20°C 3M-Ethylmagnesiumbromid/Ether (2,1 ml) zugetropft (bis nach DC kein Startmaterial mehr vorhanden). Nach Zugabe von 1M-KHSO₄-Lösung wurde mit Essigester extrahiert. Die organische Phase wurde getrocknet, eingeengt, mit HPLC (Licrosorp RP18, 40-90% Acetonitril/Wasser/0,05% Trifluoressigsäure) gereinigt, um 189 mg eines weißen Feststoffs zu ergeben [36% der Theorie; R_{f} 0,50 (Methylenchlorid:Essigester:Essigsäure = 5:2:0,1)].

### Beispiel CXVII

### 2-{4-[2-Butyl-4-chlor-5-(1-propen-1-yl)-imidazol-1-yl-methyl]-phenyl}-2-cyclopentyl-essigsäure

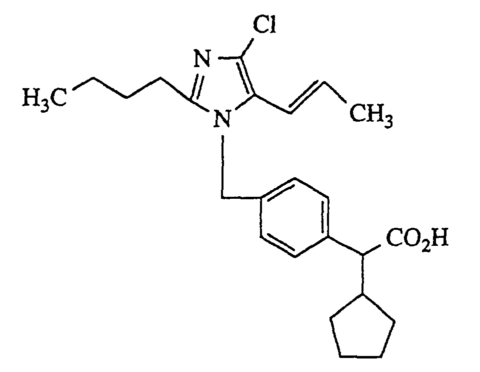

Die HPLC-Reinigung von Beispiel CXVI lieferte als unpolareres Nebenprodukt 19 mg eines weißen Feststoffs (3,8% der Theorie).

### Beispiel CXVIII

### 2-{4-[2-Butyl-4-chlor-5-(1-hydroxy)-isobutyl-imidazol-1-yl-methyl]-phenyl}-2-cyclopentyl-essigsäure

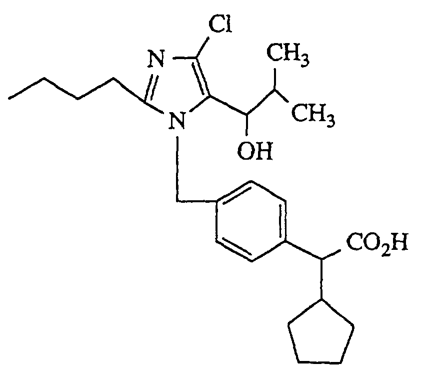

Analog zu Beispiel CXVII wurden aus Beispiel II (1,13 g roh; 1,2 mmol) mit Isopropylmagnesiumchlorid 113 mg eines weißen Feststoffs erhalten (21% der Theorie; R_{f} 0,50).

### Beispiel CXIX

### 2-{4-[2-Butyl-4-chlor-5-(1-hydroxy)-neopentyl-imidazol-1-yl-methyl]-phenyl}-2-cyclopentyl-essigsäure

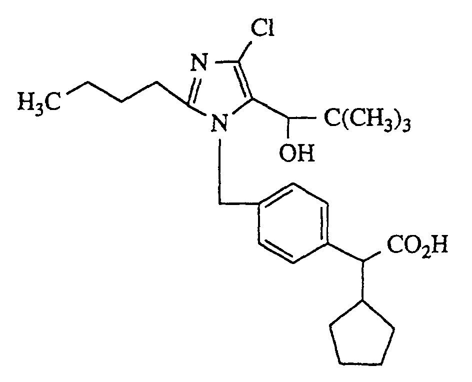

Analog zu Beispiel CXVII wurden aus Beispiel II (1,13 g roh; 1,2 mmol) mit tert.-Butylmagnesiumchlorid 68 mg eines weißen Feststoffs erhalten (12% der Theorie; R_{f} 0,50).

### Beispiel CXX

### 2- {4-[2-(1-Brom-butyl)-4-chlor-5-formyl-imidazol-1-yl-methyl]-phenyl}-2-cyclo-pentyl-essigsäure-tert.-butylester

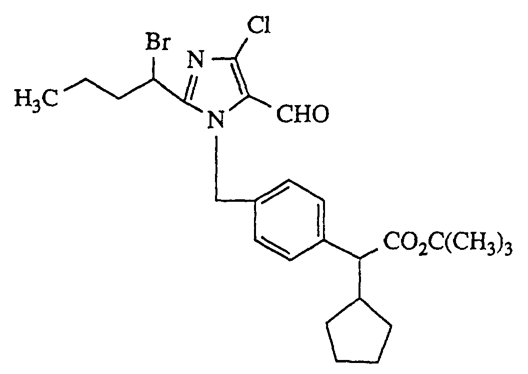

Analog zu Beispiel 24 wurden aus Beispiel I (15 g; 33 mmol) nach Kieselgelchromatographie (Hexan:Essigester = 5:1) 7,7 g eines gelben Öls erhalten [43% der Theorie; R_{f} 0,46 (Methylenchlorid)].

### Beispiel CXXI

### 2- {4-[2-(1-Buten-1-yl]-4-chlor-5-formyl-imidazol-1-yl-methyl]-phenyl}-2-cyclo-pentyl-essigsäure-tert.-butylester

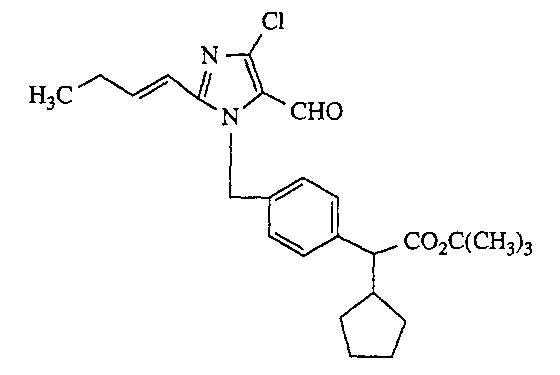

Eine Lösung von Beispiel CXX (7,7 g; 14 mmol) in THF (250 ml) wurde mit DBU (5,3 ml; 35 mmol) versetzt und 3d stehen gelassen. Nach Zugabe von Wasser und Extraktion mit Ether wurde die organische Phase mit verdünnter Salzsäure gewaschen, getrocknet und eingeengt. Kieselgelchromatographie des Rückstands (Methylenchlorid) ergab 4,4 g eines Öls (67% der Theorie; R_{f} 0,2).

### Beispiel CXXII

### 2-{4-[2-(1-Buten-1-yl)-4-chlor-5-formyl-imidazol-1-yl-methyl]-phenyl}-2-cyclo-pentyl-essigsäure

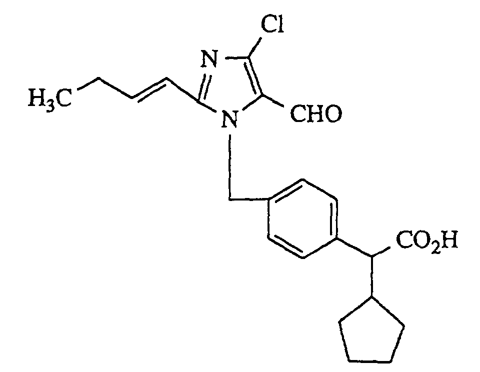

Esterspaltung von Beispiel CXXI (4,6 g; 9,6 mmol) analog Beispiel CXIII ergab 3,5 g eines gelben Öls [91% der Theorie; R_{f} 0,40 (Methylenchlorid:Methanol = 20:1)].

### Beispiel CXXIII

### 2-{4-[2-(1-Buten-1-yl)-4-chlor-5-hydroxymethyl-imidazol-1-yl-methyl]-phenyl}-2-cyclopentyl-essigsäure

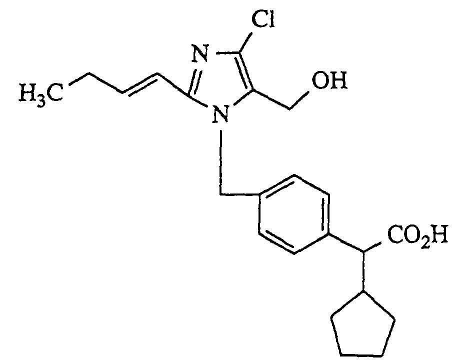

Reduktion von Beispiel CXXI (68 mg; 0,17 mmol) analog Beispiel CIX und Kieselgelchromatographie (Methylenchlorid:Methanol = 20:1) ergaben 30 mg eines weißen Feststoffs (44% der Theorie; R_{f} 0,18).

### Beispiel CXXIV

### N-4-Tolylsulfonsäure-2- {4-[2-(1-buten-1-yl)-4-chlor-5-formyl-imidazol-1-yl-methyl]-phenyl}-2-cyclopentyl-essigsäureamid

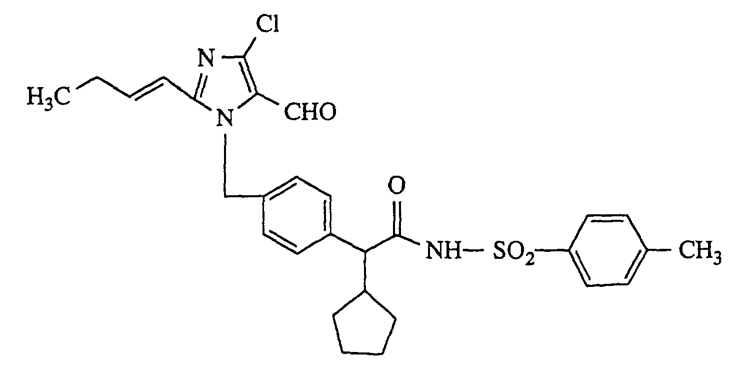

Eine Lösung von Beispiel CXXIII (1,5 g; 3,7 mmol) wurde mit einigen Tropfen DBU und Tosylisocyanat (0,62 ml; 4,1 mmol) versetzt und über Nacht unter Rückfluß gekocht. Einengen und Kieselgelchromatographie (Methylenchlorid:Methanol:konz.Ammoniakwasser = 20:1:0,1) ergaben 1,5 g eines fahlgelben Feststoffs [72% der Theorie; Fp 99°C; R_{f} 0,39 (Methylenchlorid:Methanol:konz.Ammoniakwasser = 10:1:0,1)].

### Beispiel CXXV

### 2-[4-(2-Butyl-5-formyl-4-jod-imidazol-1-yl-methyl)-phenyl]-2-cyclopentyl-essigsäure-tert.butylester

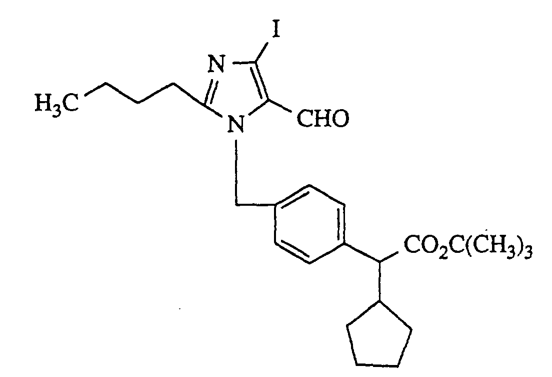

Kopplung von Beispiel 29 (9,3 g; 33 mmol) mit Beispiel 3 (11,8 g; 33 mmol) analog Beispiel 26 ergab nach Kieselgelchromatographie (Petrolether:Methylenchlorid = 1:1) 12,7 g eines gelben Feststoffs [70% der Theorie; Fp 95-7°C; R_{f} 0,18 (Methylenchlorid)].

### Beispiel CXXVI

### 2-[4-(2-Butyl-5-formyl-4-jod-imidazol-1-yl-methyl)-phenyl]-2-cyclopentyl-essigsäure

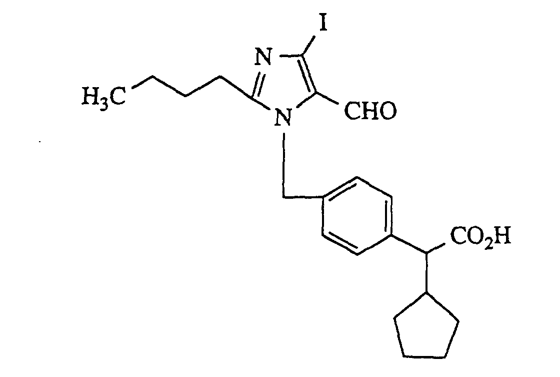

Esterspaltung von Beispiel CXXV (3,4 g; 6,2 mmol) analog Beispiel CXXIV ergab 3,15 g eines gelben Harzes [Theorie: 3,05 g; R_{f} 0,21 (Methylenchlorid:Methanol:konz.Ammoniakwasser= 10:1:0,1)].

### Beispiel CXXVII

### N-4-Tolylsulfonsäure-2-[4-(2-butyl-5-formyl-4-jod-imidazol-1-yl-methyl)-phenyl]-2-cyclopentyl-essigsäureamid

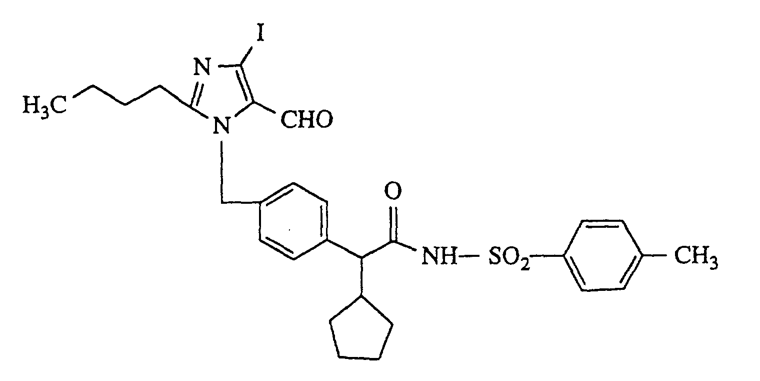

Reaktion von Beispiel CXXVI (2,55 g; 5,16 mmol) analog Beispiel CXXIII ergab nach Kieselgelchromatographie (Methylenchlorid:Methanol:konz.Ammoniakwasser = 20:1:0,1) 2,32 g eines festen Schaums [69% der Theorie; R_{f} 0,40 (Methylenchlorid:Methanol:konz.Ammoniakwasser = 10:1:0,1)].

### Beispiel CXXVIII

### N-4-Tolylsulfonsäure-2-[4-butyl-5-hydroxymethyl-4-jod-imidazol-1-yl-methyl)-phenyl]-2-cyclopentyl-essigsäureamid

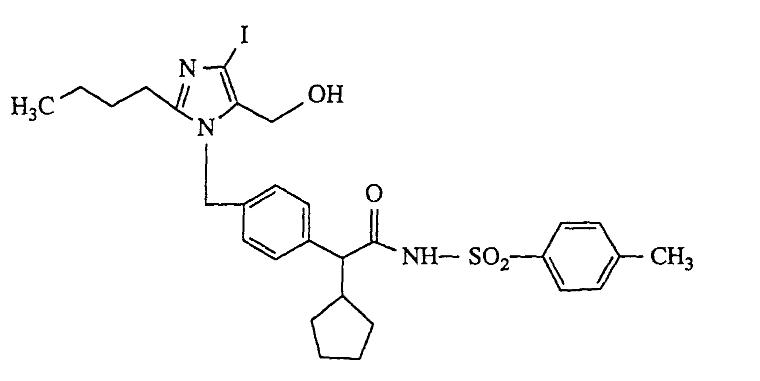

Reduktion von Beispiel CXXVII (1,3 g; 2,0 mmol) analog Beispiel CIX ergab nach Kieselgelchromatographie (Methylenchlorid:Methanol = 20:1) 1,1 g eines weißen Feststoffs (85% der Theorie; R_{f} 0,19).

### Beispiel CXXIX

### 2-[4-(2-Butyl-5-hydroxymethyl-4-jod-imidazol-1-yl-methyl)-phenyl]-2-cyclopentyl-essigsäure-tert.butylester

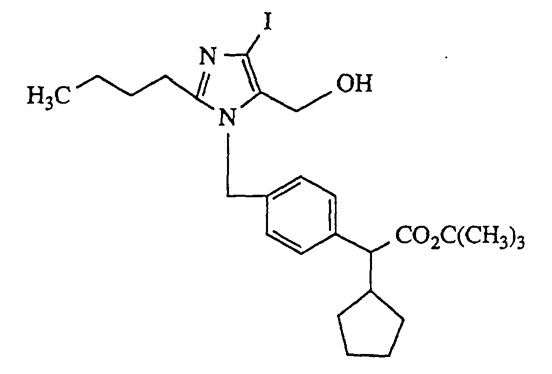

Reduktion von Beispiel CXXV (5,4 g; 9,8 mmol) analog Beispiel CIX ergab nach Kieselgelchromatographie (Methylenchlorid:Methanol = 20:1) 4,3 g eines Öls (80% der Theorie; R_{f} 0,34).

### Beispiel CXXX

### 2-{4-{2-Butyl-5-[(2-methoxy)ethoxy-methoxy-methyl]-4-jod-imidazol-1-yl-methyl}-phenyl}-2-cyclopentyl-essigsäure-tert.butylester

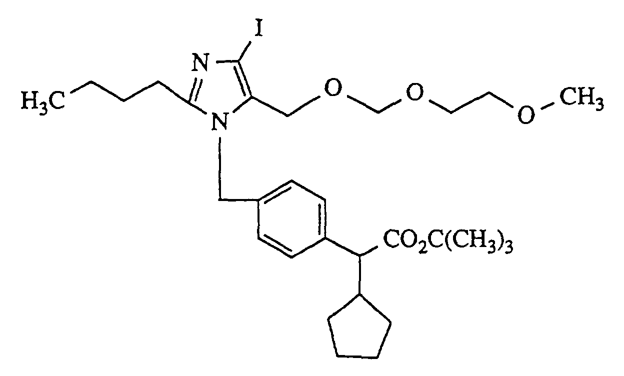

Unter Argon wurde zu einer Suspension von 80%-Natriumhydrid/Paraffinöl (4,71 mg; 15,7 mmol) in THF (10 ml) bei -5°C eine Lösung von Beispiel CXXIX (3,94 g; 7,13 mmol) in THF (100 ml) zugetropft. Nach 15 min Rühren bei 0°C wurde bei -5°C 2-Methoxy-ethoxy-methylchlorid (1,8 ml; 15,7 mmol) zugetropft. Nach 2h bei Raumtemperatur und 2h bei 50°C wurde das Reaktionsgemisch eingeengt, zwischen Ether und Wasser verteilt und mit NaHCO₃ auf pH 8-9 eingestellt. Die wäßrige Phase wurde viermal mit Ether extrahiert, die vereinten organischen Phasen wurden mit gesättigter Kochsalzlösung gewaschen, getrocknet und eingeengt. Kieselgelchromatographie (Methylenchlorid:Methanol-Stufengradient) lieferte 2,15 g eines Öls [47% der Theorie; R_{f} 0,54 (Methylenchlorid:Methanol = 20:1)].

### Beispiel CXXXI

### 2-{4-{2-Butyl-5-[(2-methoxy)ethoxy-methoxy-methyl]-trifluormethyl-imidazol-1-yl-methyl}-phenyl}-2-cyclopentyl-essigsäure-tert.butylester

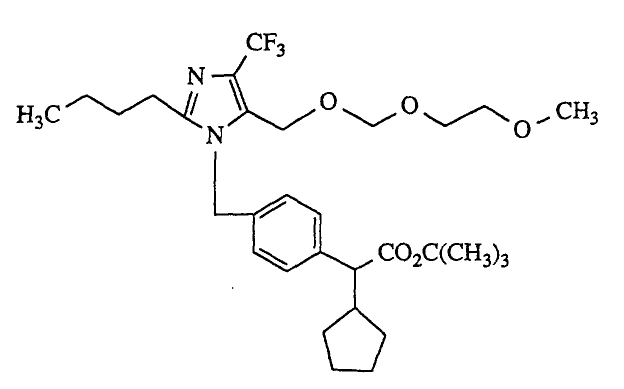

Unter Argon wurde zu einer Suspension von Cadmiumpulver (22,4 g; 0,200 g atom) in DMF (50 ml) Dibromdifluormethan (8,6 ml; 94 mmol; Trockeneiskühler) langsam zugetropft. Nach 2h Rühren wurde die Suspension unter Argon durch eine Schlenck-Fritte filtriert, um eine braune, ca. 1,6M-Bistrifluormethylcadmium/DMF-Stammlösung zu ergeben.

Unter Argon wurde eine Lösung von 1,6M-Bistrifluormethylcadmium/DMF (27 ml) und HMPT (36 ml) mit Kupfer(I)-bromid (3,78 g; 26,4 mmol) und Beispiel CLXXX (4,64 g; 7,2 mmol) versetzt. Nach 8h Rühren bei 75°C wurden Wasser (100 ml) und Methylenchlorid (500 ml) zugegeben. Der Niederschlag wurde abfiltriert, die organische Phase abgetrennt und eingeengt. Der Rückstand wurde in Essigester (500 ml) und Petrolether (500 ml) gelöst, fünfmal mit Wasser gewaschen, getrocknet und eingeengt. Kieselgelchromatographie (Methylenchlorid/Methanol-Stufengradient) ergaben 3,75 g eines Öls (89% der Theorie; R_{f} 0,45 (Hexan:Essigester = 2:1)].

### Beispiel CXXXII

### 2-{4-(2-Butyl-5-[(2-methoxy)ethoxy-methoxy-methyl]-4-trifluonnethyl-imidazol-1-yl-methyl}-phenyl}-2-cyclopentyl-essigsäure

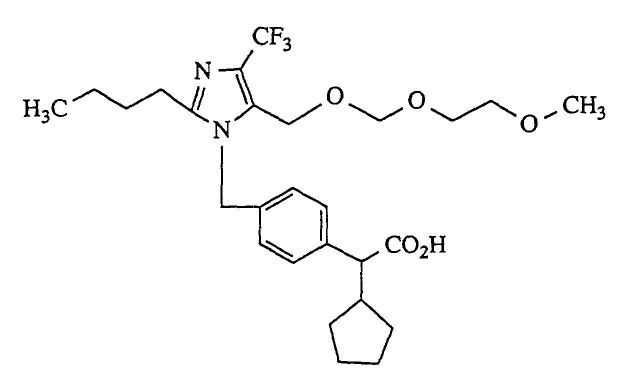

Eine Lösung von Beispiel CXXXI (3,73 g; 6,48 mmol) in Methylenchlorid (40 ml) wurde bei 0°C mit Trifluoressigsäure (40 ml) versetzt und 3h bei Raumtemperatur gerührt. Nach Einengen wurde der Rückstand in 100 ml Essigester aufgenommen und mit 5%-NaHCO₃-Lösung auf pH 7 eingestellt. Die wäßrige Phase wurde viermal mit Essigester extrahiert, die vereinten organischen Phasen wurden mit gesättigter Kochsalzlösung gewaschen, getrocknet und eingeengt, um 2,65 g eines Öls zu ergeben. Alkalischstellen der vereinten wäßrigen Phasen mit Na₂CO₃-Lösung und Essigester-Extraktion lieferten weitere 0,32 g [87% der Theorie; R_{f} 0,40 (Methylenchlorid:Methanol = 20:1)].

### Beispiel CXXXIII

### 2-[4-(2-Butyl-5-hydroxymethyl-4-trifluormethyl -imidazol-1-yl-methyl)-phenyl]-2-cyclopentyl-essigsäure

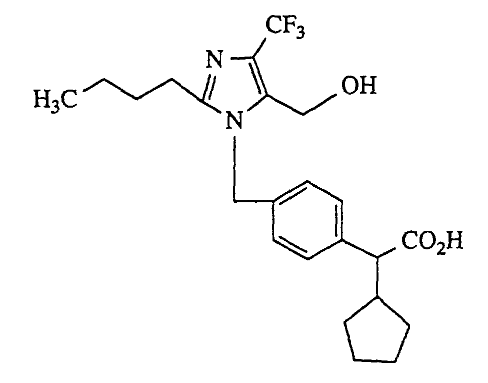

Eine Lösung von Beispiel CXXXII (160 mg; 0,304 mmol) in Dioxan (5 ml) wurde mit 4N-HCl/Dioxan (5 ml) und Wasser (10 ml) versetzt und 1h gerührt. Nach Einengen wurde der pH mit 5%-NaHCO₃-Lösung auf 7 eingestellt, und die Lösung wurde mit Essigester fünfmal extrahiert. Die vereinten organischen Phasen wurden mit gesättigter Kochsalzlösung gewaschen, getrocknet und eingeengt, um 141 mg eines Öls zu ergeben [Theorie: 133 mg; R_{f} 0,24 (Methylenchlorid:Methanol = 20:1)].

### Beispiel CXXXIV

### N-4-Tolylsulfonsäure-2-{4-{2-butyl-5-[(2-methoxy)ethoxy-methoxy-methyl]-4-trifluormethyl-imidazol-1-yl-methyl}-phenyl}-2-cyclopentyl-essigsäureamid

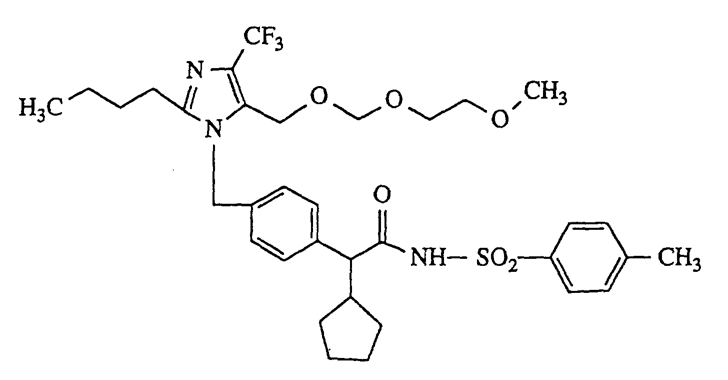

Beispiel CXXXII (650 mg; 1,23 mmol) wurde analog Beispiel CXXVII umgesetzt. Kieselgelchromatographie (Hexan/Essigester 4:1 bis 2:1, dann Methylenchlorid/Methanol 50:1 bis 12,5:1) ergab 410 mg eines Öls [49% der Theorie; R_{f} 0,35 (Hexan:Essigester = 1:1)].

### Beispiel CXXXV

### N-4-Tolylsulfonsäure-2-[4-(2-butyl-5-hydroxymethyl-4-trifluormethyl-imidazol-1-yl-methyl)-phenyl]-2-cyclopentyl-essigsäureamid

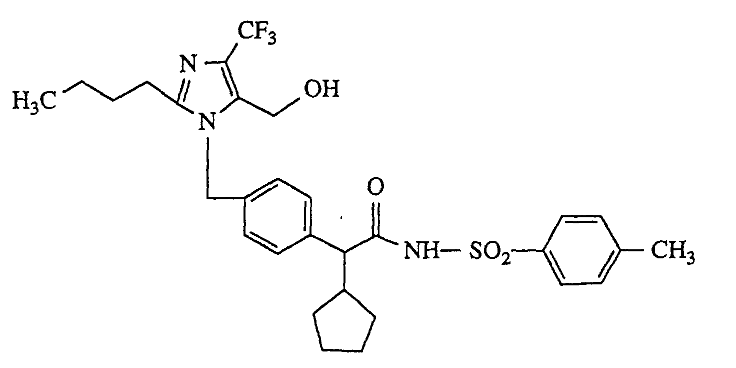

Beispiel CXXXIV (200 mg; 0,294 mmol) wurde analog Beispiel CXXXIII entschützt, um 160 mg eines Öls zu ergeben [92% der Theorie; R_{f} 0,41 (Methylenchlorid:Methanol = 20:1)].

### Beispiel CXXXVI

### 2-[4-(5-Acetoxymethyl-2-butyl-4-jod-imidazol-1-yl-methyl)-phenyl]-2-cyclopentyl-essigsäure-tert.butylester

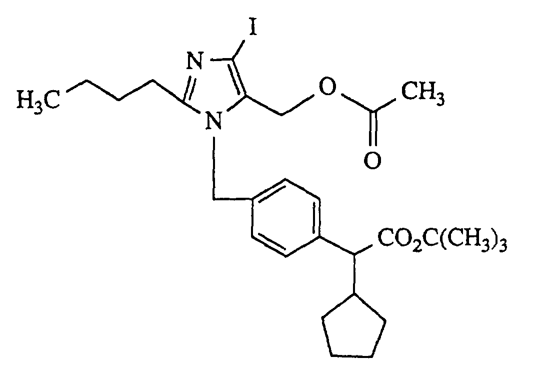

Eine Lösung von Beispiel CXXIX (9,5 g; 17 mmol) in Pyridin (20 ml) und Acetanhydrid (20 ml) wurde mit 4-DMAP (0,21 g; 1,7 mmol) versetzt und über Nacht bei Raumtemperatur, dann 2h bei 50°C gerührt. Das Reaktionsgemisch wurde eingeengt, mit 1M-KHSO₄-Lösung auf pH 3 gestellt und mit Essigester extrahiert. Trocknen und Einengen der organischen Phasen und Kieselgelchromatographie (Methylenchlorid) lieferten 9,2 g eines gelben Öls (93% der Theorie; R_{f} 0,34).

### Beispiel CXXXVII

### 2-[4-(5-Acetoxymethyl-2-butyl-4-perfluorbutyl-imidazol-1-yl-methyl)-phenyl]-2-cyclopentyl-essigsäure-tert.butylester

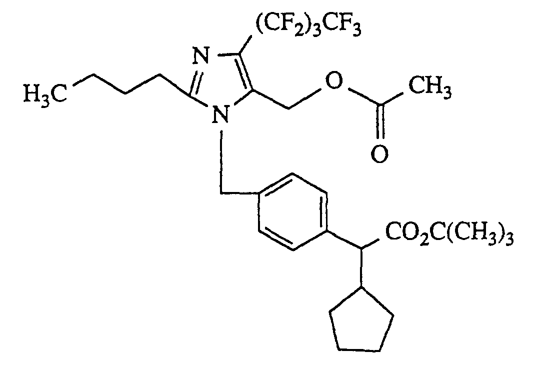

Unter Argon wurde zu einer Suspension von Cadmiumpulver (12,3 g; 0,110 g atom) in DMF (100 ml) Perfluorbutyljodid (12,9 ml; 75 mmol; Trockeneiskühler) langsam zugetropft. Nach 2h Rühren bei Raumtemperatur und 1h bei 35°C wurde die Suspension unter Argon durch eine Schlenck-Fritte filtriert.

Unter Argon wurden Bisperfluorbutylcadmium/DMF-Lösung (15 ml) und HMPT (7 ml) mit Kupfer(I)-bromid (1,5 g; 11 mmol) und einer Lösung von Beispiel CXXXVI (886 mg; 1,49 mmol) in DMF (10 ml) versetzt. Nach 6h Rühren bei 75°C wurden Wasser (100 ml) und Methylenchlorid (500 ml) zugegeben. Der Niederschlag wurde abfiltriert, die organische Phase abgetrennt und eingeengt. Der Rückstand wurde in Essigester/Petrolether (1:1) gelöst, fünfmal mit Wasser gewaschen, getrocknet und eingeengt. Kieselgelchromatographie (Methylenchlorid:Methanol = 1:1) ergaben 235 mg eines Öls (23% der Theorie; R_{f} 0,48 (Hexan:Essigester = 3:1)].

### Beispiel CXXXVIII

### 2-[4-(5-Acetoxymethyl-2-butyl-4-perfluorbutyl-imidazol-1-yl-methyl)-phenyl]-2-cyclopentyl-essigsäure

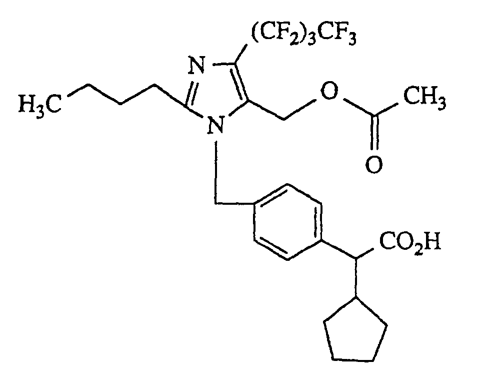

Esterspaltung von Beispiel CXXXVII analog Beispiel CXXXII führte zu 172 mg eines Öls [96% der Theorie; R_{f} 0,34 (Methylenchlorid:Methanol = 20:1)].

### Beispiel CXXXIX

### N-4-Tolylsulfonsäure-2-{4-[2-(1-buten-1-yl)-4-chlor-5-hydroxymethyl-imidazol-1-yl-methyl]-phenyl}-2-cyclopentyl-essigsäureamid

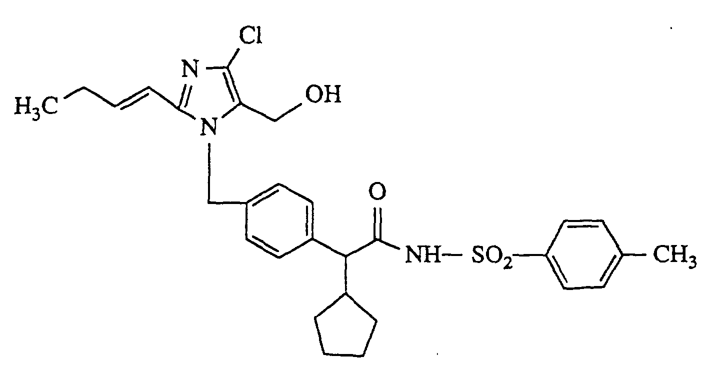

Reduktion von Beispiel CXXIV (0,50 g; 0,90 mmol) analog Beispiel CX und Kieselgelchromatographie (Methylenchlorid:Methanol = 20:1) ergaben 322 mg eines weißen Feststoffs (64% der Theorie; Fp 116-8°C; R_{f} 0,25).

Die in den Tabellen XII bis XV aufgeführten Verbindungen werden in Analogie zu den dort angegebenen Vorschriften hergestellt.

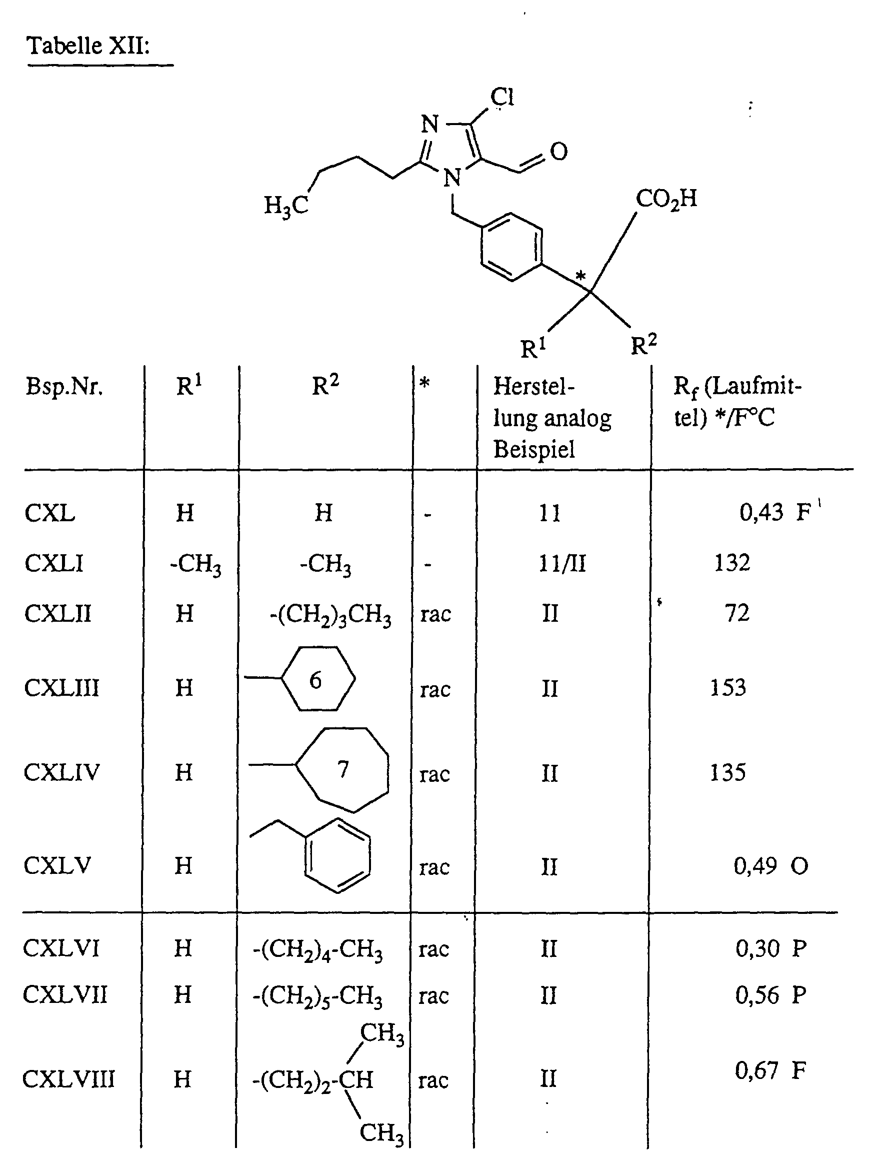

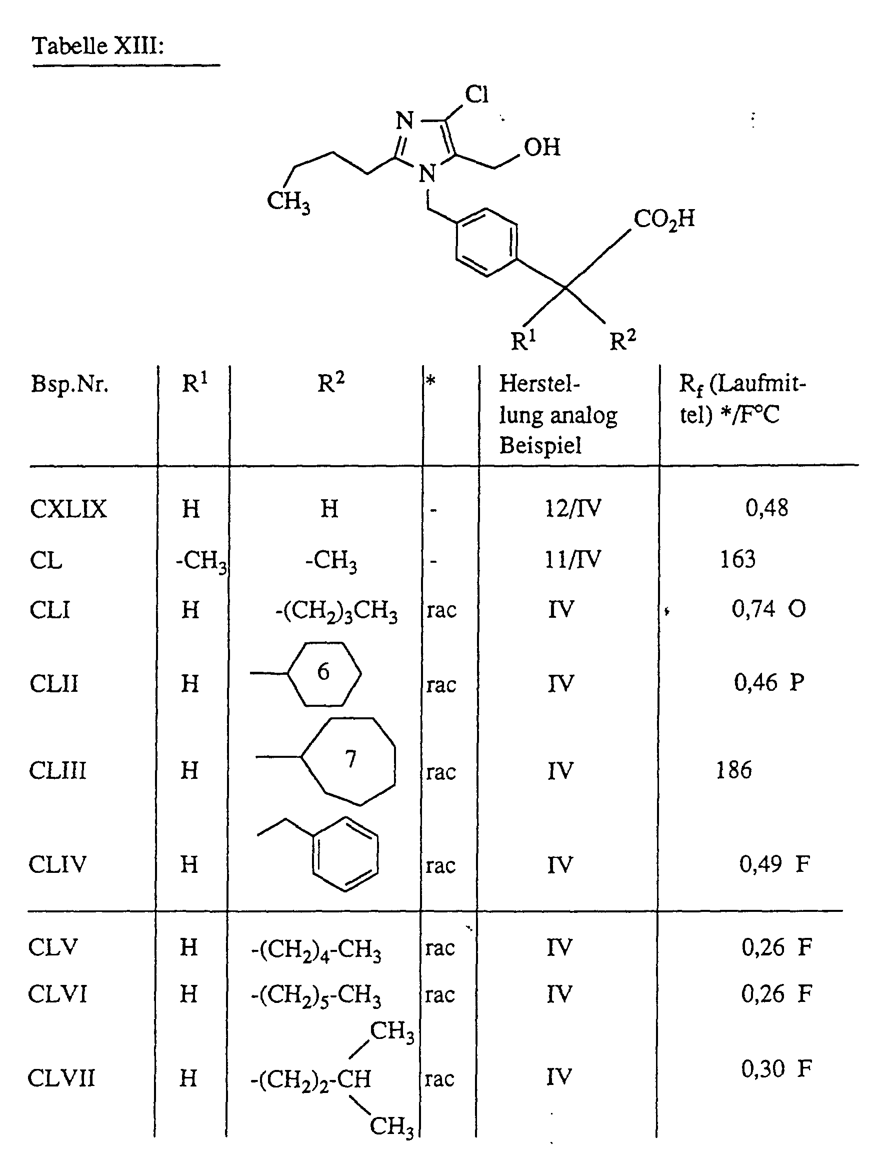

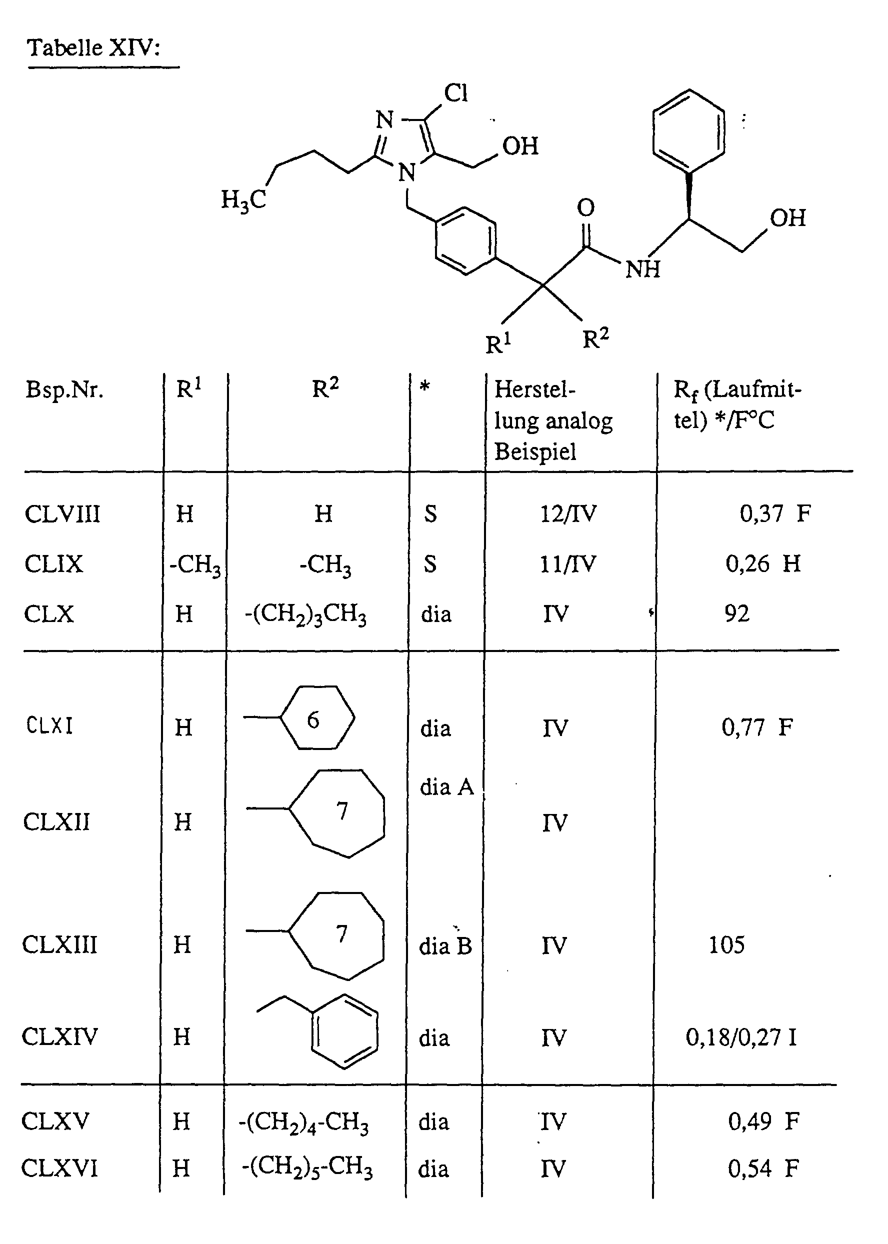

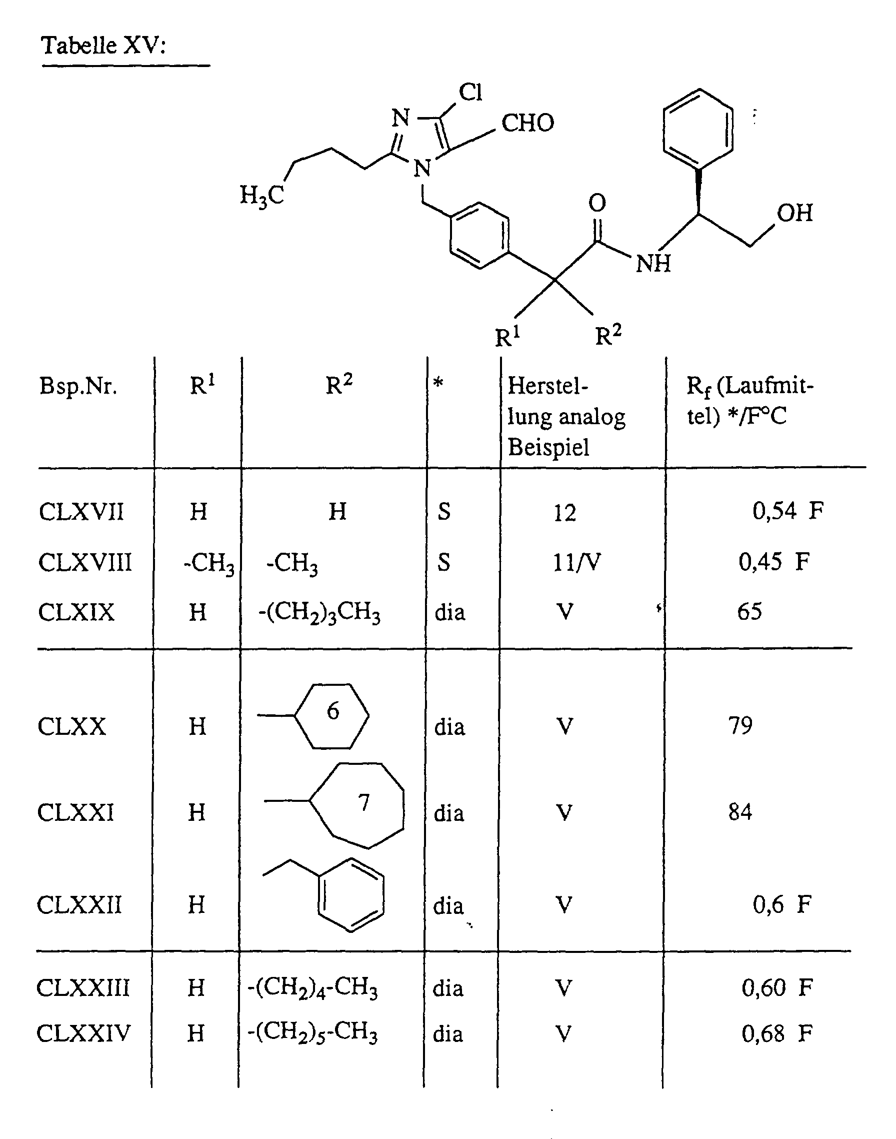

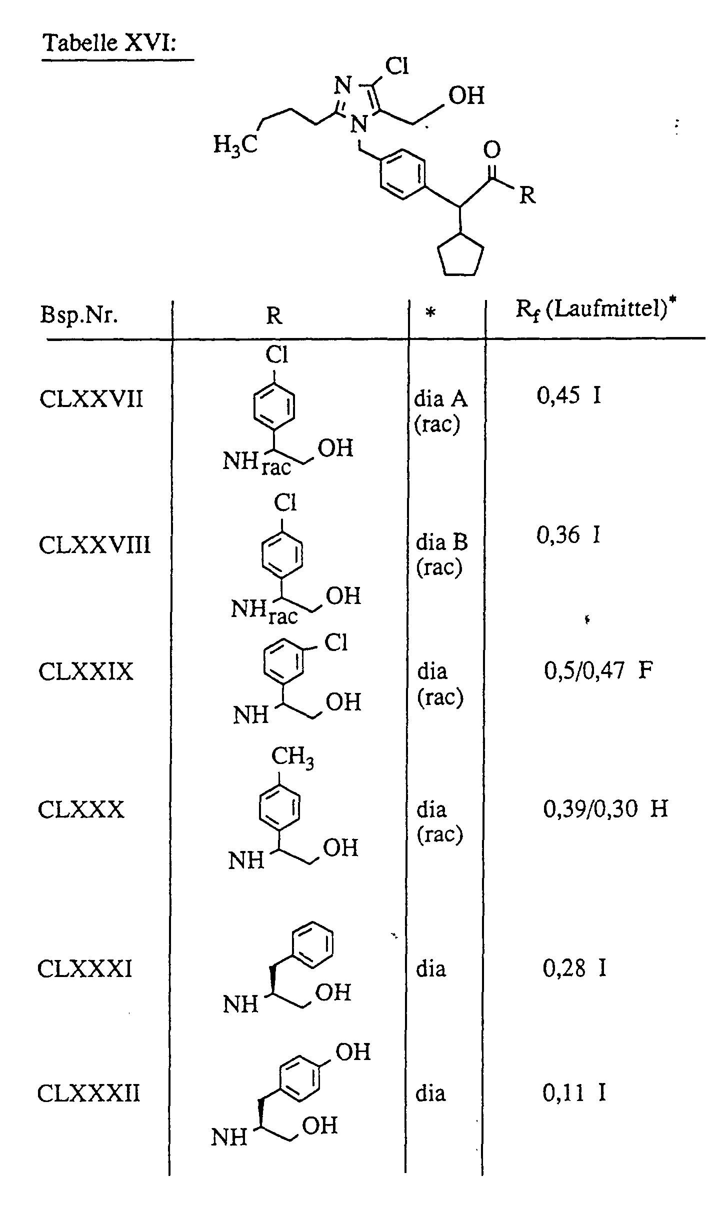

### Beispiel CLXXV

### 2 [4-(2-Butyl-4-chlor-5-carboxy-imidazol-1-yl-methyl)phenyl]2-cyclopentylessigsäure-N(2-acetoxy-1,S-phenyl-ethyl)amid

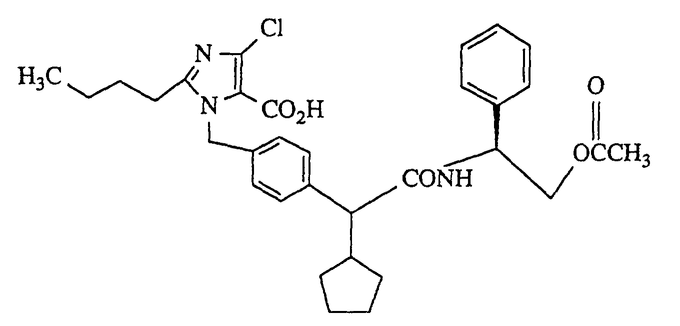

564 mg 2[4-(2-Butyl-4-chlor-5-formyl-imidazol-1-yl-methyl)phenyl]2-cyclopentylessigsäure-N(2-acetoxy-1,S-phenyl-ethyl)amid werden in 6 ml tert.Butanol gelöst, mit 4 ml 1,25 M wäßriger NaH₂PO₄-Lösung (pH 7) und anschließend mit 6 ml 1 M KMnO₄-Lösung (wäßrig) versetzt. Nach 10 minütigem Rühren werden im Eisbad 6 ml gesättigte Na₂SO₃-Lösung zugegeben, und die Lösung wird mit 1 M HCl auf pH 3 gebracht. Nach dreifacher Extraktion mit Essigsäureethylester wird die organische Phase über Na₂SO₄ getrocknet, filtriert und eingeengt.
Ausbeute: 340 mg (63 % der Theorie).

### Beispiel CLXXVI

### 2[4-(2-Butyl-4-chlor-5-carboxy-imidazol-1-yl-methyl)-phenyl]2-cyclopentylessigsäure-N(2-hydroxy-1,S-phenyl-ethyl)amid

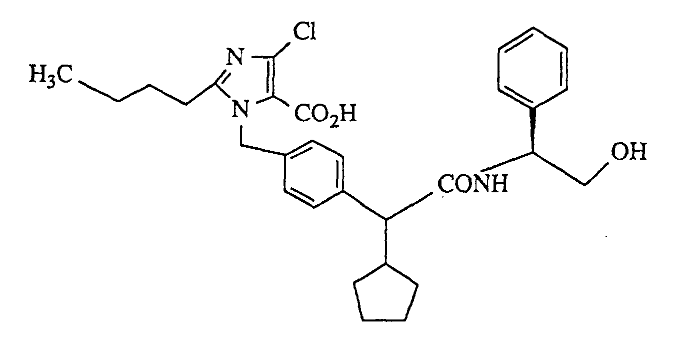

200 mg 2-[4-(2-Butyl-4-chlor-5-carboxy-imidazol-1-yl-methyl)phenyl]2-cyclopentyl-essigsäure-N(2-acetoxy-1,S-phenyl-ethyl)arnid werden in 1,2 ml Dioxan/0,8 ml H₂O gelöst und mit 44 mg LiOH in 0,3 ml gelöst versetzt. Nach Rühren über Nacht wird mit Ether gewaschen, auf pH 4 gebracht (1 N HOAc) und mit Essigester 3 x extrahiert. Die vereinigten Essigesterphasen werden über Na₂SO₄ getrocknet, filtriert und eingeengt.
Ausbeute: 179 mg (95 % der Theorie).

Die in Tabelle XVI aufgeführten Beispiele werden in Analogie zur Vorschrift des Beispiels II hergestellt.

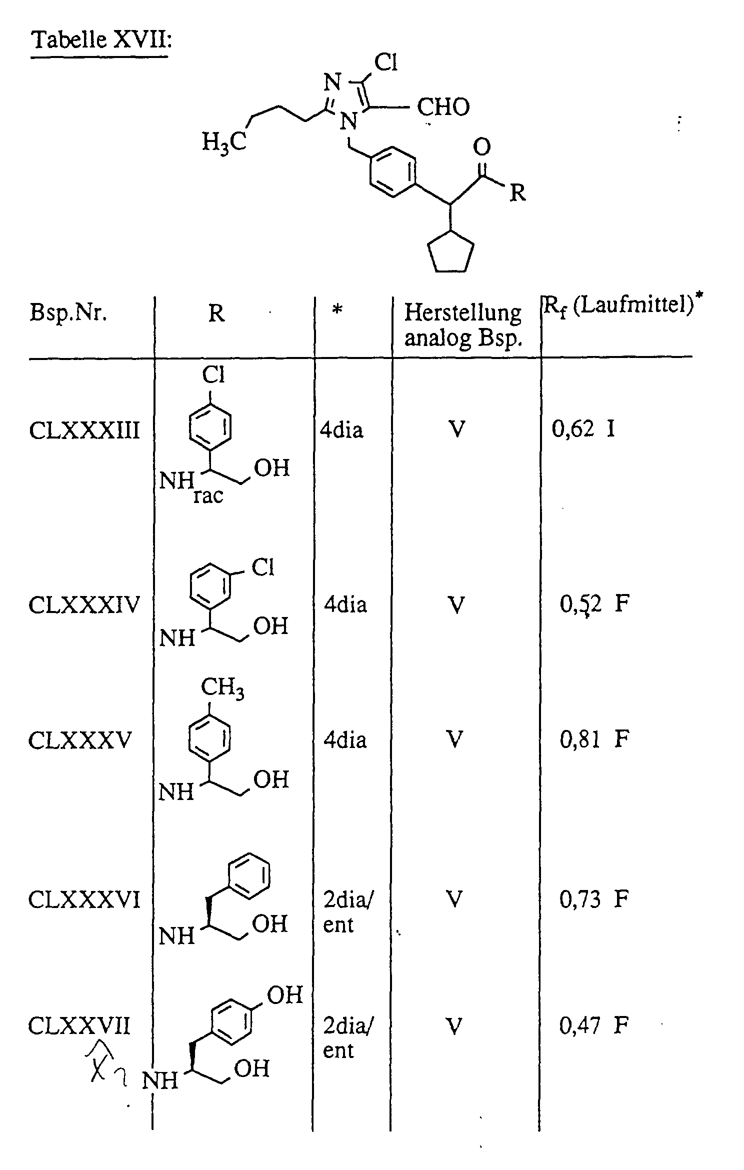

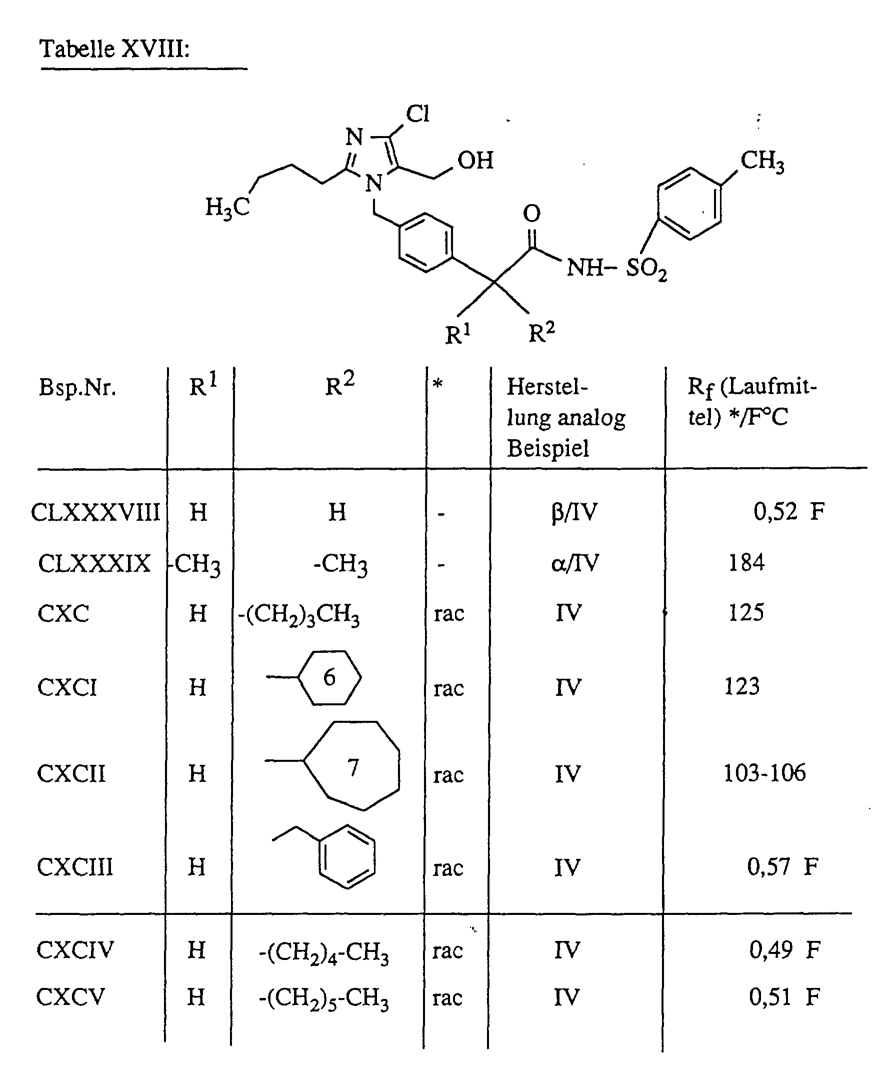

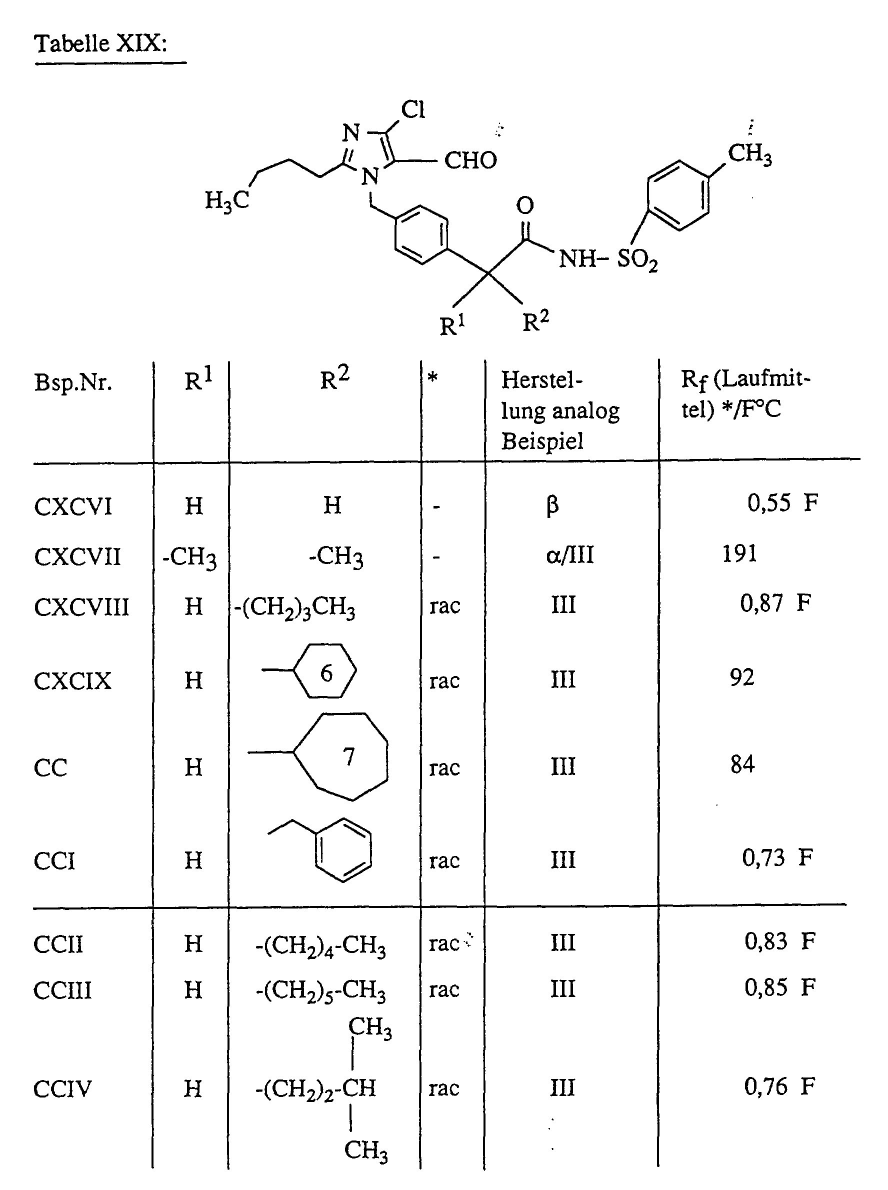

In Analogie zur Vorschrift des Beispiels CLXXV werden die in Tabelle XX aufgeführten Verbindungen hergestellt:

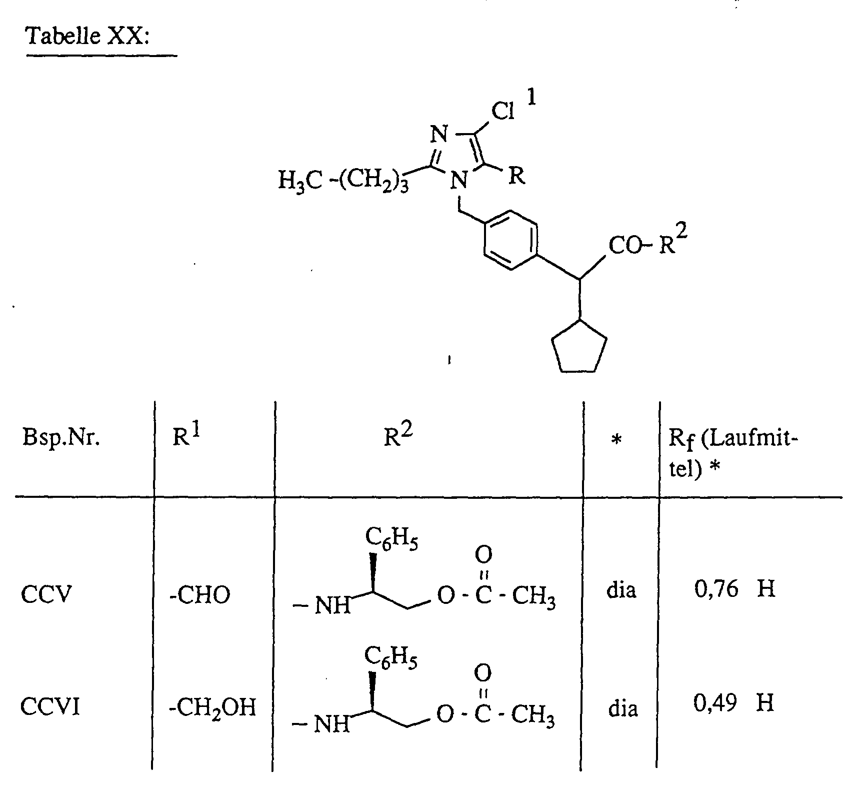

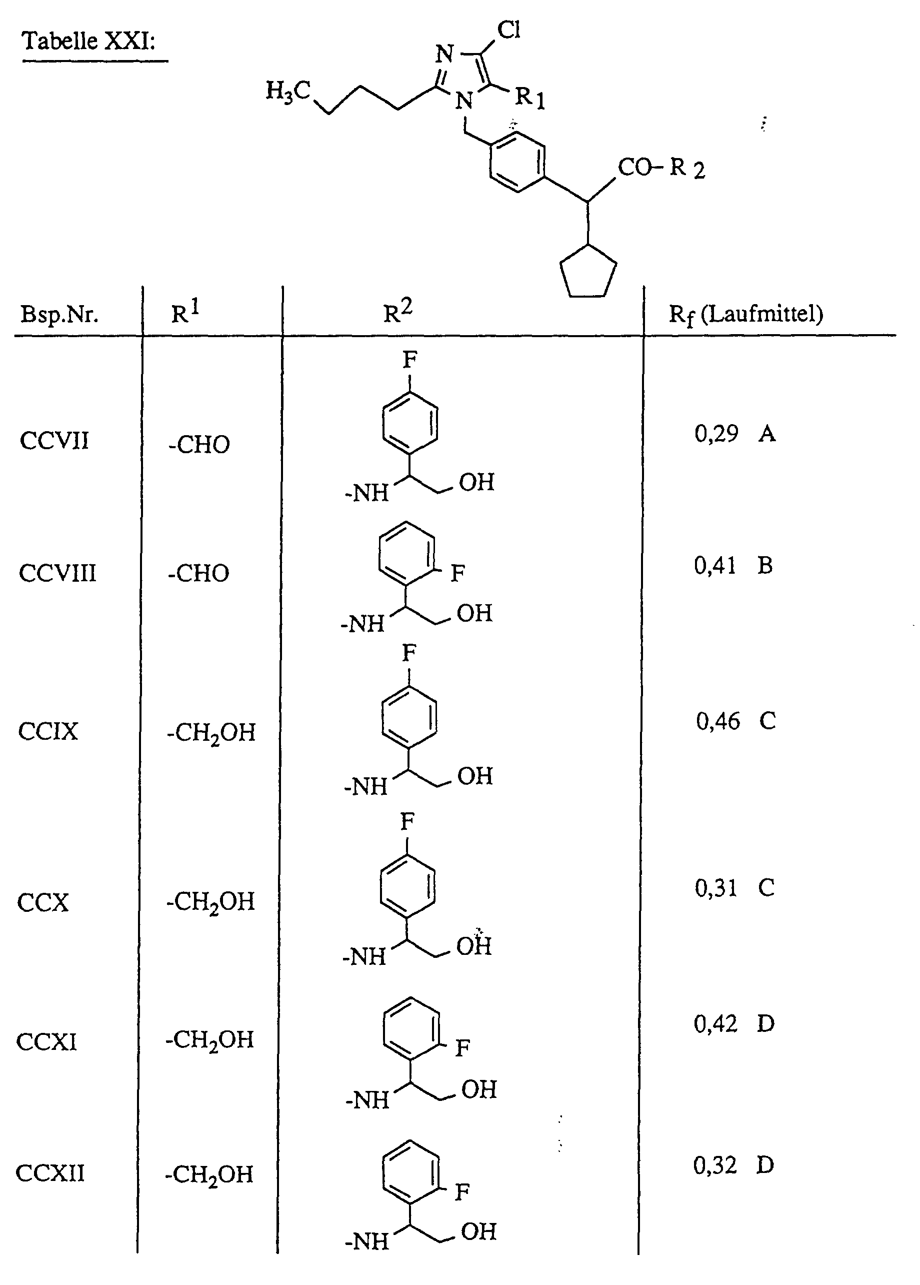

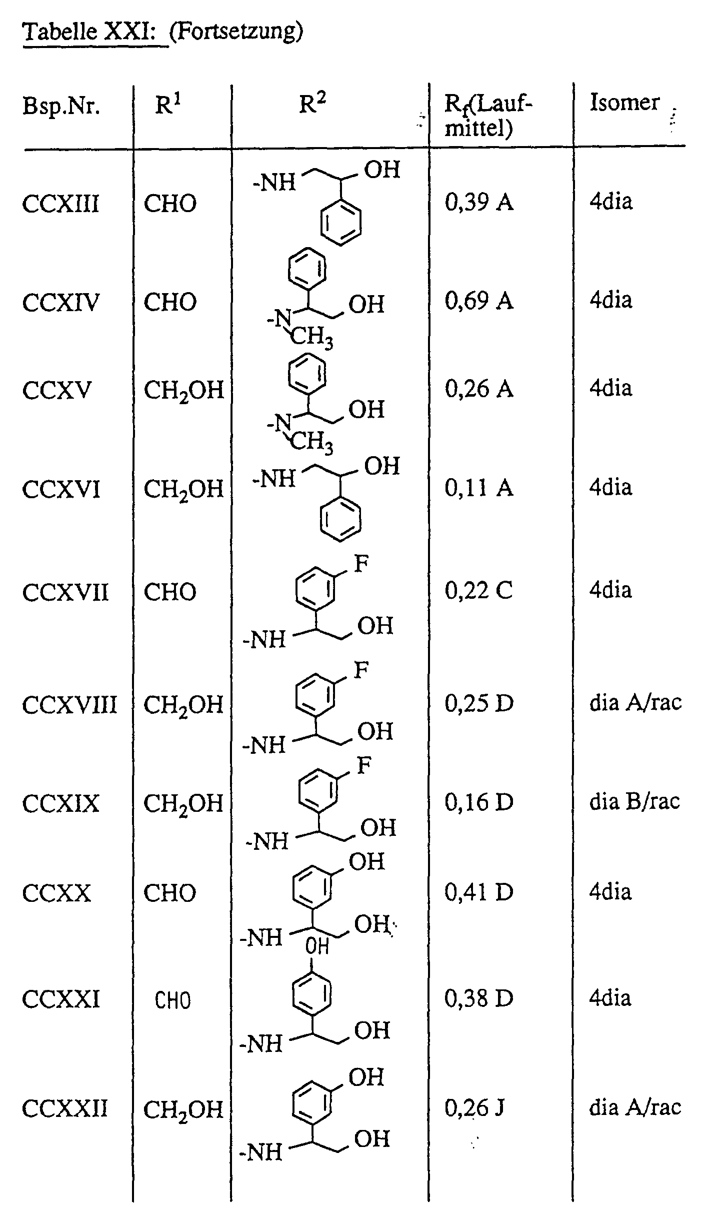

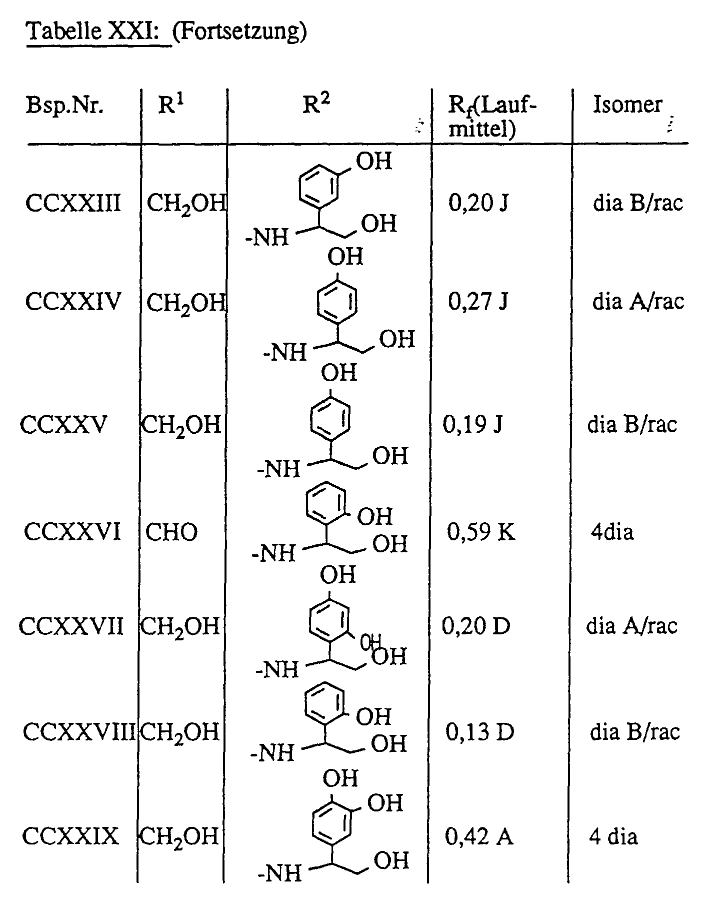

Die in Tabellen XXII bis XXXI aufgeführten Verbindungen werden in Analogie zu den dort genannten Vorschriften hergestellt.

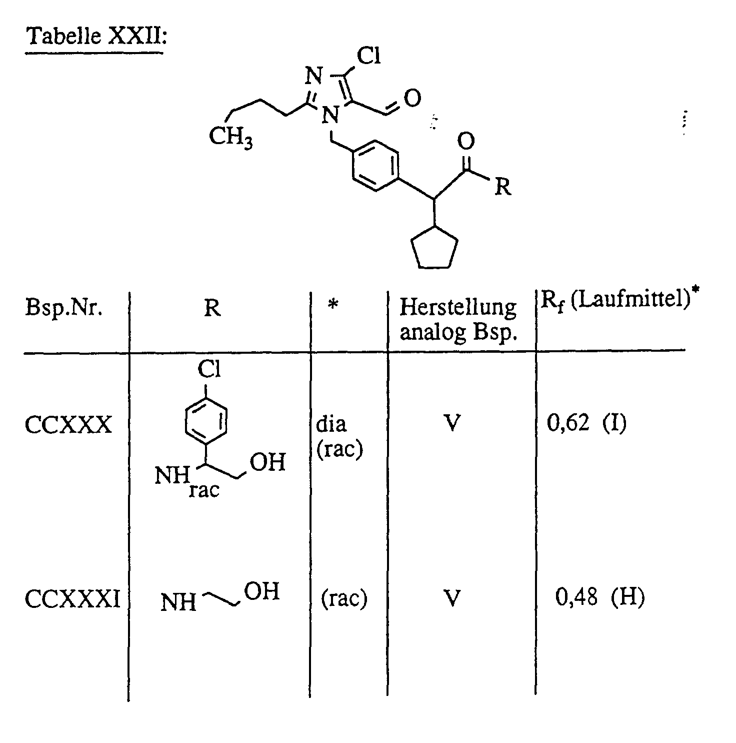

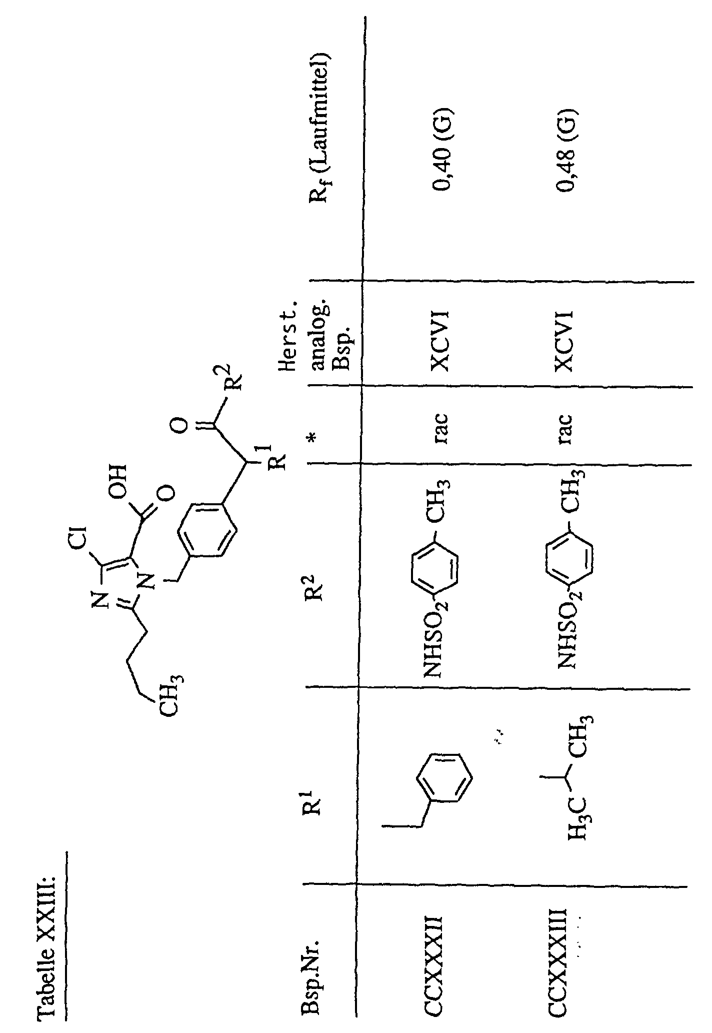

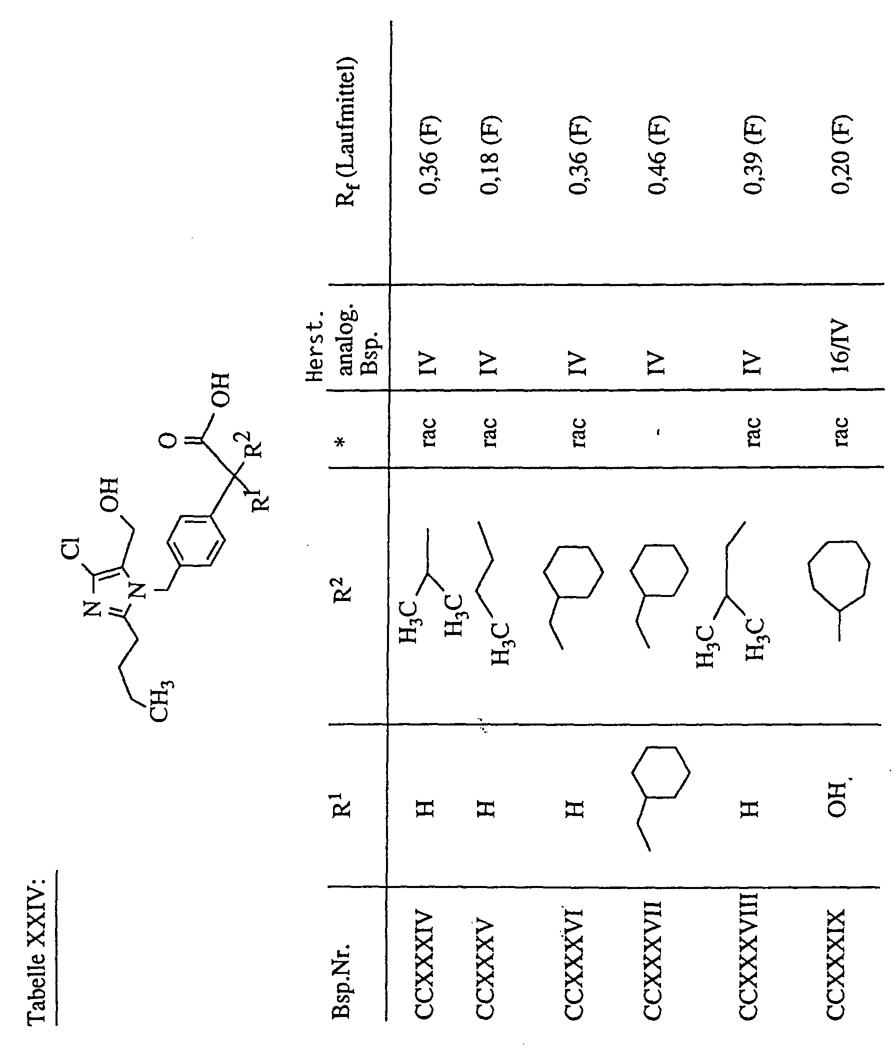

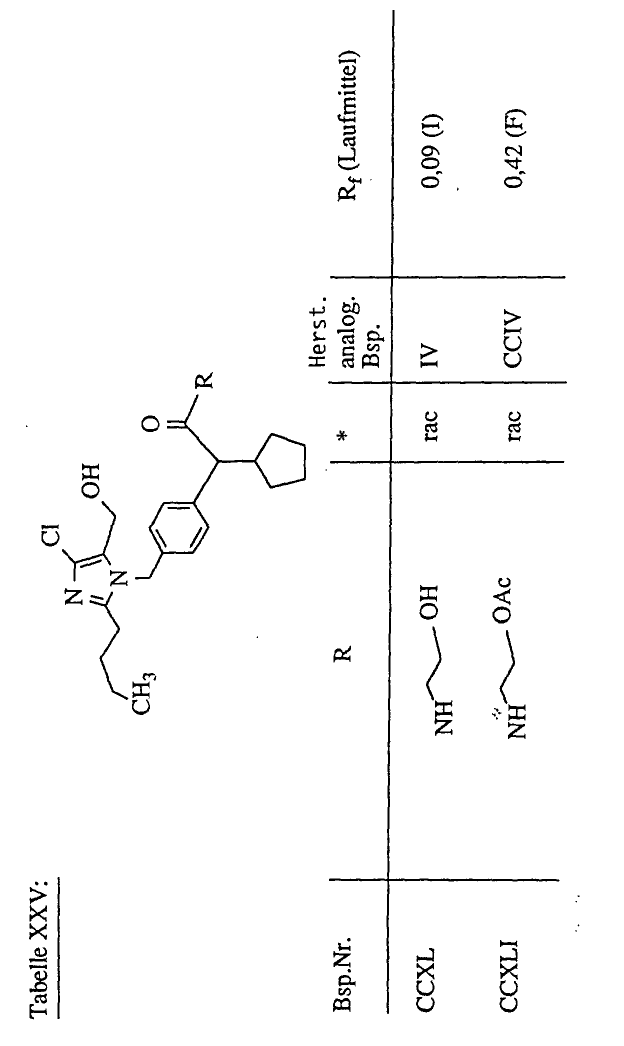

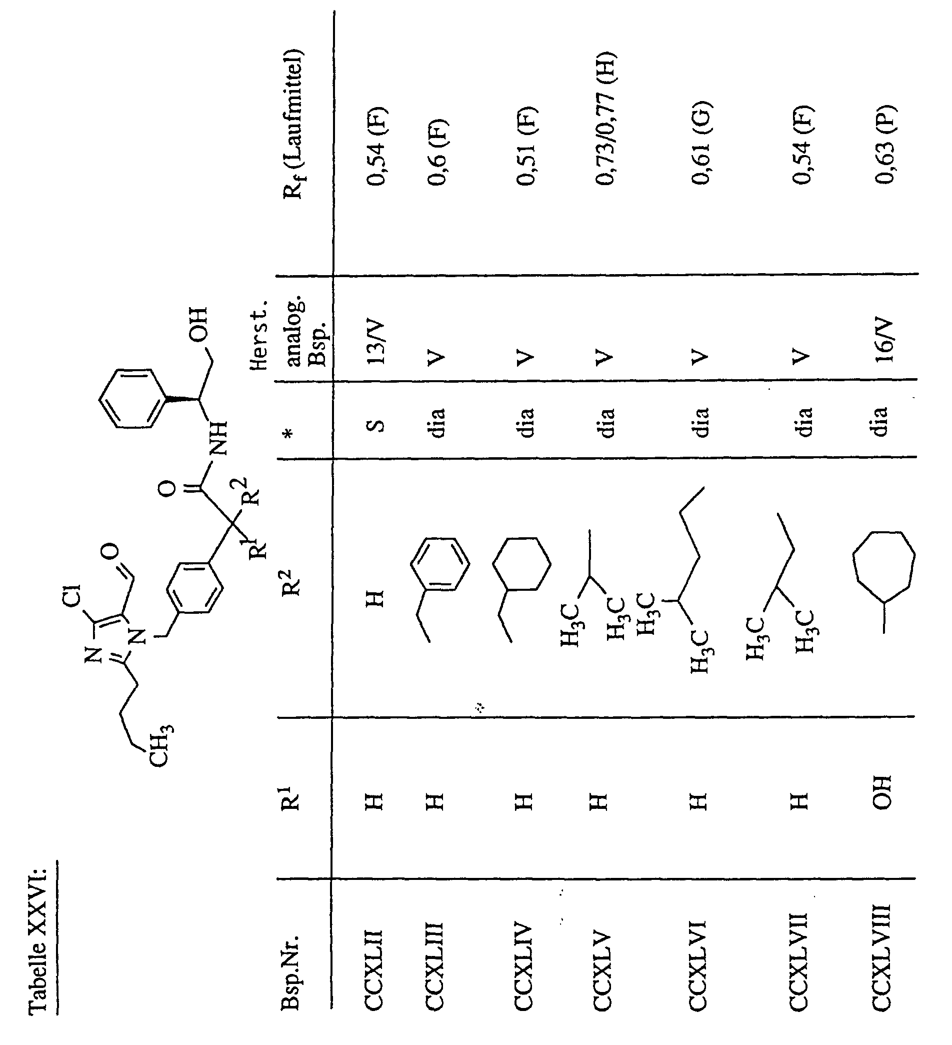

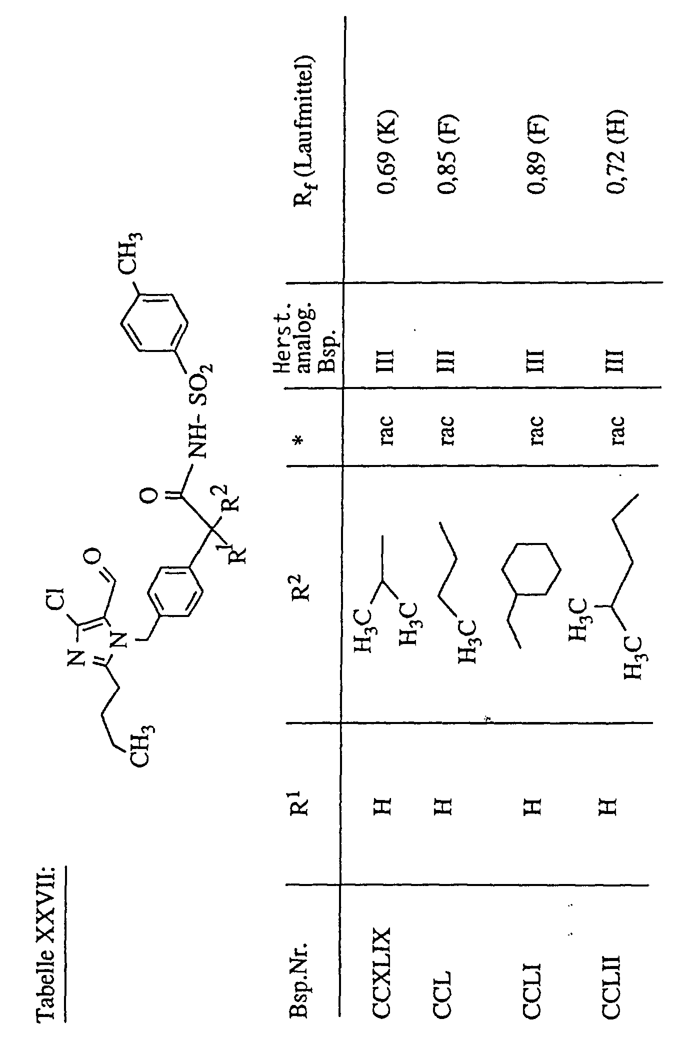

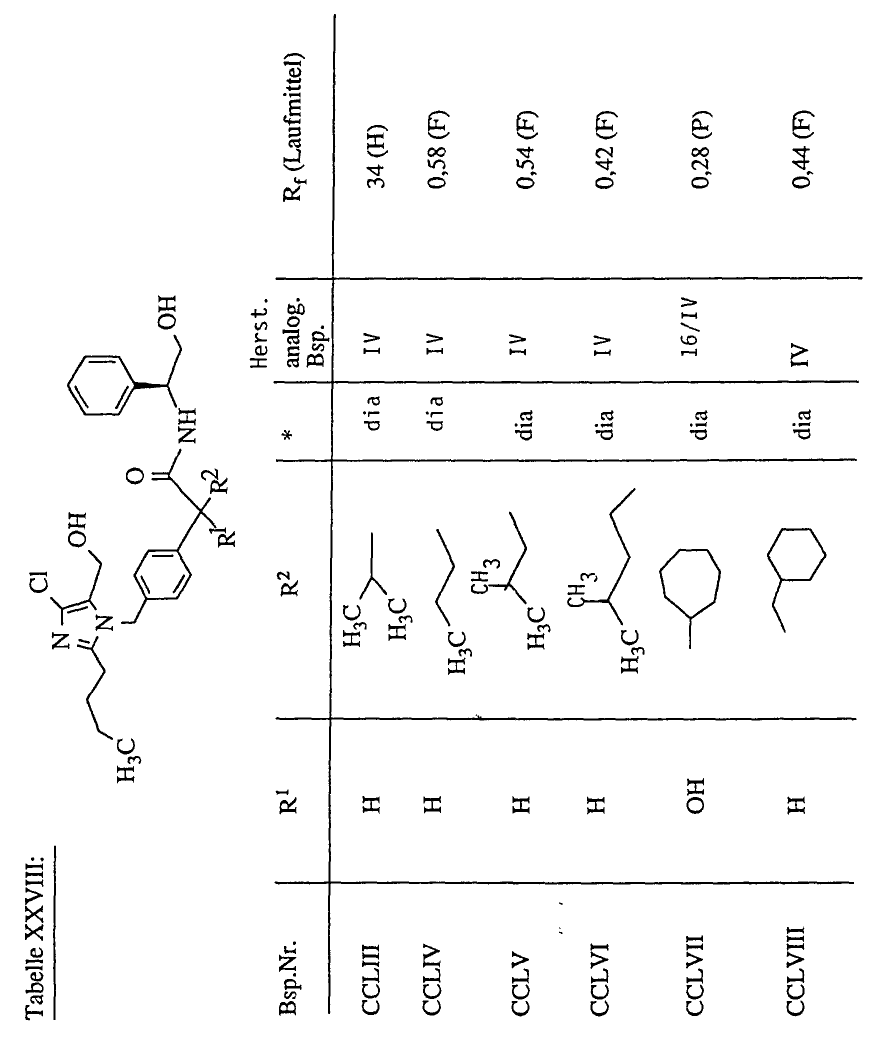

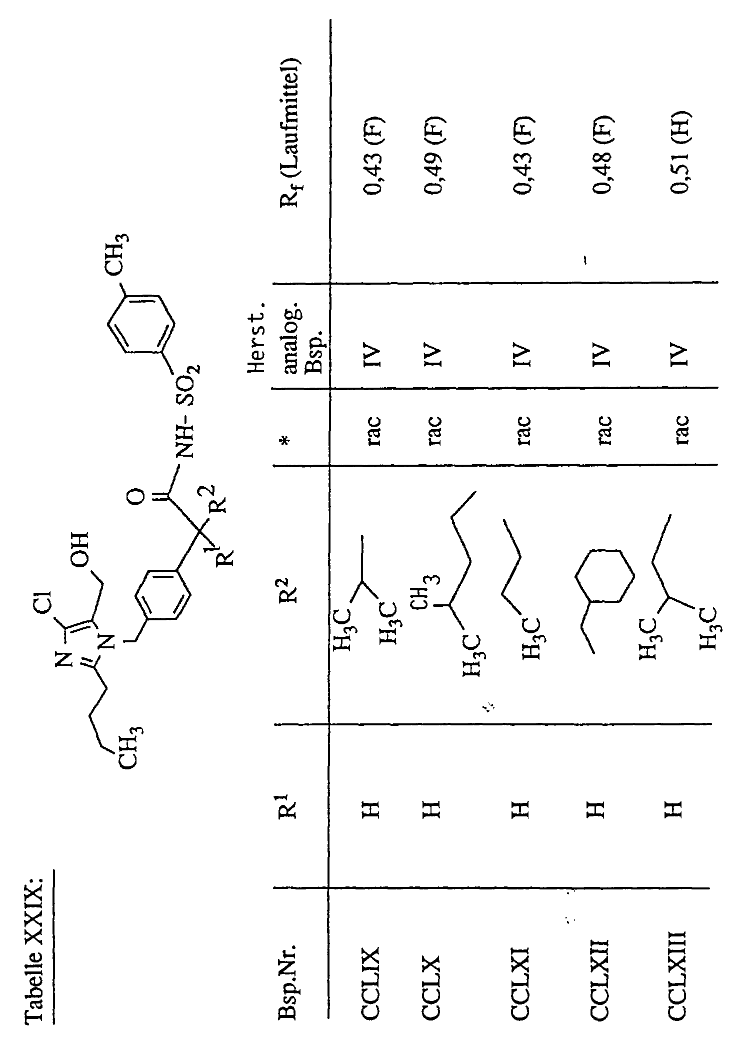

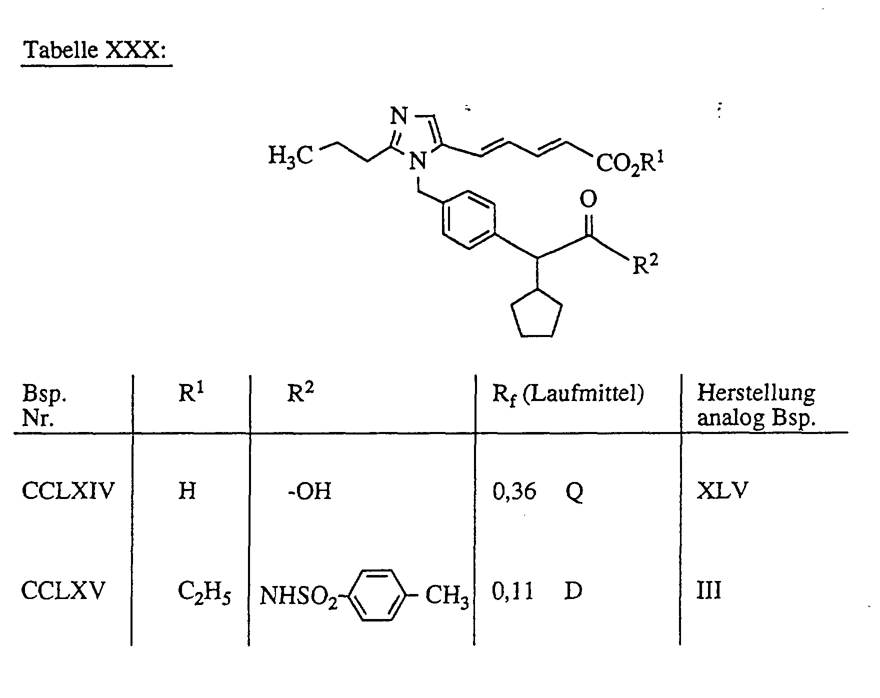

### Beispiel CCLXVI

### N-4-tolylsulfonsäure-2-[4-(2-butyl-5-hydroxymethyl-4-pentafluorethylimidazol-1-yl-methyl)-phenyl]-2-cyclopentyl-essigsäureamid

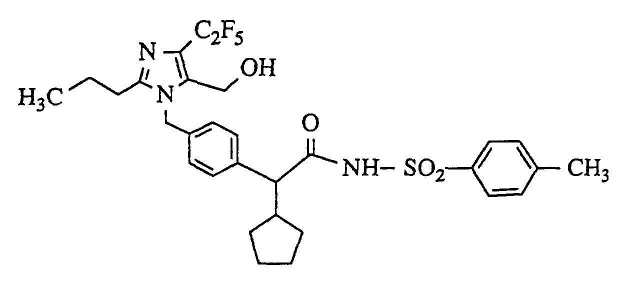

280 mg 0,409 mmol der Verbindung aus Beispiel 4 werden 3 Tage in 60 ml einer gesättigten methanolischen Ammoniaklösung stehen gelassen. Dann wurde eingeengt und zwischen einer 5 %igen wäßrigen Natriumbicarbonatlösung und Ethylacetat verteilt. Die wäßrige Phase wurde mit wäßriger Kaliumhydrogensulphatlösung auf pH 3 gestellt und anschließend mit Ethylacetat extrahiert. Trocknen und Einengen der organischen Phase ergaben 255 mg [97 % der Theorie, R_{f} = 0,27 (C)].

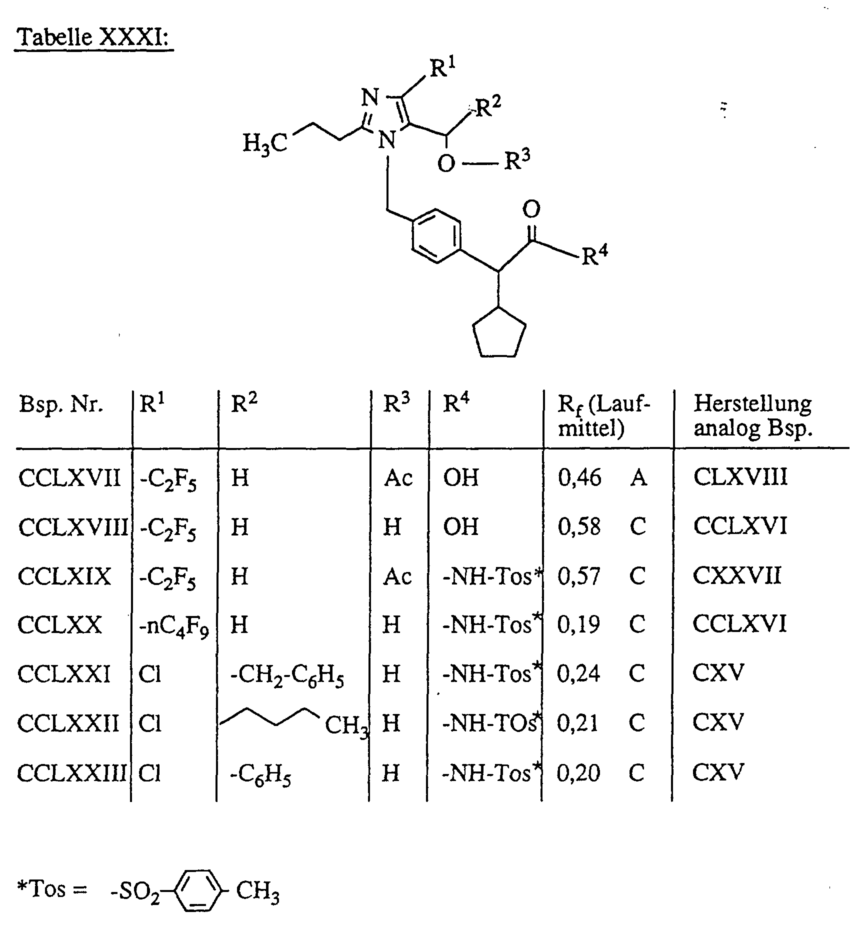

## Patentansprüche

1. Heterocyclisch substituierte Phenylessigsäurederivate der Formel 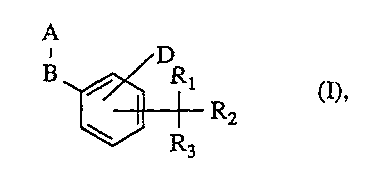 in welcher
A für einen über ein Stickstoffatom gebundenen heterocyclischen Rest der Formel 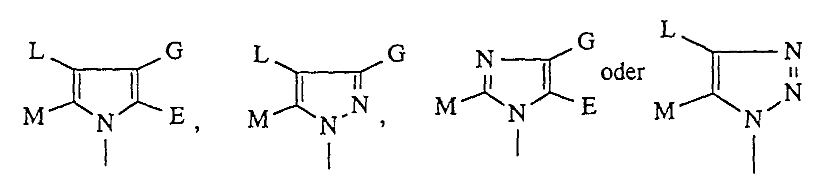 steht,
worin
E, G, L und M gleich oder verschieden sind und Wasserstoff, Cyclopropyl, Cyclopentyl, Tetrazolyl, Fluor, Chlor, Iod, Perfluoralkyl mit bis zu 4 Kohlenstoffatomen oder eine Gruppe der Formel 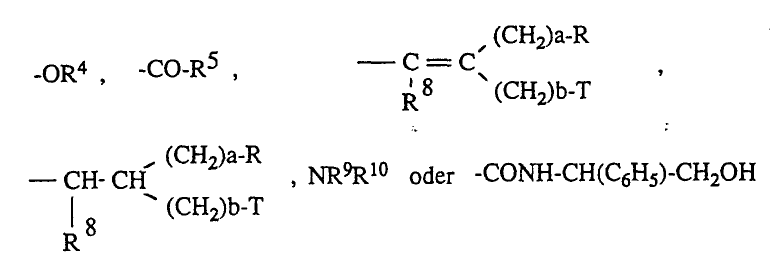 bedeuten,
worin
R⁴ Wasserstoff, geradkettiges oder verzweigtes Alkyl oder Acyl mit jeweils bis zu 6 Kohlenstoffatomen oder Phenyl bedeutet, das seinerseits durch Fluor, Chlor, Brom, Hydroxy, Nitro oder durch geradkettiges oder verzweigtes Alkyl oder Alkoxy mit jeweils bis zu 6 Kohlenstoffatomen substituiert ist, oder Methoxyethoxymethyl bedeutet,
R⁵ Wasserstoff, Hydroxy oder geradkettiges oder verzweigtes Alkyl oder Alkoxy mit jeweils bis zu 6 Kohlenstoffatomen oder eine Gruppe der Formel -NR⁹R¹⁰ oder -NR⁹-SO₂R¹¹ bedeutet,
worin
R⁹ und R¹⁰ gleich oder verschieden sind und Wasserstoff, geradkettiges oder verzweigtes Alkyl oder Acyl mit jeweils bis zu 6 Kohlenstoffatomen oder Phenyl bedeuten, das gegebenenfalls durch Nitro, Trifluormethyl, Fluor, Chlor, Brom oder durch geradkettiges oder verzweigtes Alkyl mit bis zu 4 Kohlenstoffatomen substituiert ist,
R¹¹ geradkettiges oder verzweigtes Alkyl mit bis zu 6 Kohlenstoffatomen, Benzyl, 2-Phenylvinyl oder Phenyl bedeutet, das gegebenenfalls durch Fluor, Chlor, Brom, Trifluormethyl oder durch geradkettiges oder verzweigtes Alkyl oder Alkoxy mit jeweils bis zu 4 Kohlenstoffatomen substituiert ist,
R⁸ Wasserstoff, Hydroxy, Acetyloxy, geradkettiges oder verzweigtes Alkyl mit bis zu 6 Kohlenstoffatomen oder Phenyl bedeutet,
a und b gleich oder verschieden sind und eine Zahl 0, 1, 2 oder 3 bedeuten,
R Wasserstoff oder Phenyl, Thienyl oder Furyl bedeutet, die gegebenenfalls bis zu 2fach gleich oder verschieden durch Carboxy, geradkettiges oder verzweigtes Alkyl, Alkoxy oder Alkoxycarbonyl mit jeweils bis zu 6 Kohlenstoffatomen, Nitro, Hydroxy, Fluor, Chlor, Brom, Trifluormethyl oder Trifluormethoxy substituiert sind,
T Wasserstoff oder eine Gruppe der Formel -OR^{4'} oder -CO-R^{5'} bedeutet,
worin
R^{4'} und R^{5'} die oben angegebene Bedeutung von R⁴ und R⁵ haben und mit dieser gleich oder verschieden ist,
oder
E, G, L und M geradkettiges oder verzweigtes Alkyl, Alkenyl oder Alkadienyl mit jeweils bis zu 8 Kohlenstoffatomen bedeuten, die gegebenenfalls bis zu 2fach durch Fluor, Brom, Chlor, Phenyl, Tetrazolyl, Cyclopropyl, Cyclopentyl, Cyclohexyl oder durch eine der Gruppen 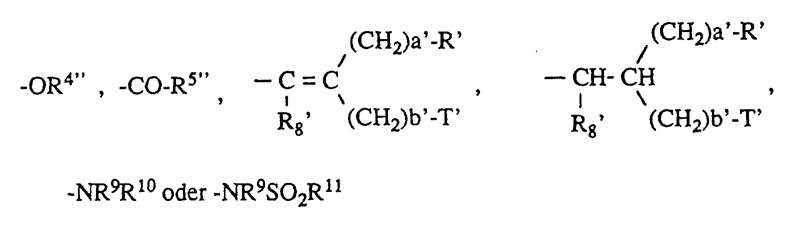 substituiert sind,
worin
R^{4"}, R^{5"}, R^{8'}, a', b', R' und T' die oben angegebene Bedeutung von R⁴, R⁵, R⁸, a, b, R und T haben und mit dieser gleich oder verschieden sind,
und
R⁹, R¹⁰ und R¹¹ die oben angegebene Bedeutung haben
oder
jeweils E und G, G und L oder L und M gemeinsam mit dem Heterocyclus einen an diesen ankondensierten Cyclopentyl-, Cyclopentenyl-, Cyclohexyl-, Cyclohexenyl-, Phenyl-, Pyridyl-, Thienyl- oder Pyrimidinyl-Ring bilden, wobei diese Ringe gegebenenfalls bis zu 2fach gleich oder verschieden durch Fluor, Chlor, Brom oder durch eine Gruppe der Formel -V-W oder W substituiert sind,
worin
V geradkettiges oder verzweigtes Alkylen mit bis zu 4 Kohlenstoffatomen bedeutet,
W Wasserstoff oder eine Gruppe der Formel -OR^{4"},-CO-R^{5"} oder -NR^{9'}R^{10'} bedeutet,
worin
R^{4"} und R^{5"} die oben angegebene Bedeutung haben,
und
R^{9'} und R^{10'} die oben angegebene Bedeutung von R⁹ und R¹⁰ haben und mit dieser gleich oder verschieden sind,
B für unverzweigtes Alkyl, Alkenyl oder Alkinyl mit jeweils bis zu 3 Kohlenstoffatomen steht,
D für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
R¹ und R² gleich oder verschieden sind und für Wasserstoff, Hydroxy oder für geradkettiges oder verzweigtes Alkyl mit bis zu 8 Kohlenstoffatomen stehen, die gegebenenfalls durch Fluor, Chlor, Brom, Cyclopropyl, Cyclopentyl, Cyclohexyl oder Phenyl substituiert ist, das seinerseits durch Fluor, Chlor, Brom oder durch geradkettiges oder verzweigtes Alkyl oder Alkoxy mit jeweils bis zu 6 Kohlenstoffatomen substituiert sein kann, für Cyclobutyl, Cyclopentyl, Cyclohexyl, Cycloheptyl, Cyclooctyl oder Phenyl stehen, die gegebenenfalls durch Fluor, Chlor, Brom oder durch geradkettiges oder verzweigtes Alkyl oder Alkoxy mit jeweils bis zu 4 Kohlenstoffatomen substituiert sind,
oder
R¹ und R² gemeinsam einen Cyclopropyl-, Cyclopentyl- oder Cyclohexyl-ring bilden, der gegebenenfalls durch geradkettiges oder verzweigtes Alkyl oder Alkoxy mit jeweils bis zu 4 Kohlenstoffatomen, Phenyl, Fluor oder Chlor substituiert ist,
R³ für eine Gruppe der Formel
-CO-NR¹⁹R²⁰ steht,
worin
R¹⁹ und R²⁰ gleich oder verschieden sind und die oben angegebene Bedeutung von R⁹ und R¹⁰ haben und mit dieser gleich oder verschieden sind oder
R¹⁹ die oben angegebene Bedeutung hat
und
R²⁰ eine Gruppe der Formel 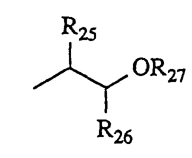 bedeutet,
worin
R²⁵ und R²⁶ gleich oder verschieden sind und Wasserstoff, Phenyl, Benzyl, Pyridyl oder Thienyl bedeuten, die ihrerseits einfach oder zweifach durch Carboxy, Fluor, Chlor, Brom oder durch geradkettiges oder verzweigtes Alkyl, Alkoxy oder Alkoxycarbonyl mit jeweils bis zu 6 Kohlenstoffatomen substituiert sein können,
R²⁷ Wasserstoff oder geradkettiges oder verzweigtes Alkyl oder Acyl mit jeweils bis zu 6 Kohlenstoffatomen bedeutet,
und deren Salze.

2. Verfahren zur Herstellung von heterocyclisch substituierten Phenylessigsäurederivaten nach Anspruch 1, **dadurch gekennzeichnet**, daß man Verbindungen der allgemeinen Formel (II) 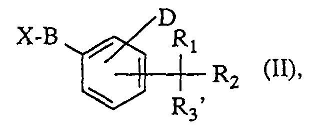 in welcher
B, D, R¹ und R² die in Anspruch 1 angegebene Bedeutung haben,
X für eine typische Abgangsgruppe wie beispielsweise Chlor, Brom, Iod, Tosylat oder Mesylat, vorzugsweise für Brom steht,
und
R^{3'} für Alkoxycarbonyl mit bis zu 6 Kohlenstoffatomen oder Cyano steht,
mit Verbindungen der allgemeinen Formel (III)
A-H (III),
worin
A die in Anspruch 1 angegebene Bedeutung hat,
in inerten Lösemitteln, gegebenenfalls in Anwesenheit einer Base und gegebenenfalls unter Schutzgasatmosphäre zu Verbindungen der allgemeinen Formel (Ia) 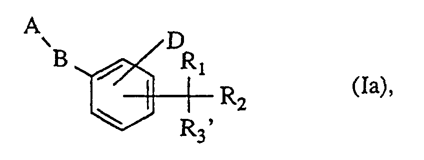 in welcher
A, B, D, R¹, R² und R^{3'} die in Anspruch 1 angegebene Bedeutung haben,
umsetzt,
die Ester- oder Cyanogruppe nach üblicher Methode verseift,
zur Herstellung der Amide (R³ = -CO-NR¹⁹R²⁰) entweder direkt die Ester oder die Säuren nach üblicher Aktivierung, gegebenenfalls in Anwesenheit einer Base, eines Hilfs- und/oder eines Dehydrationsmittels, mit Aminen der allgemeinen Formel (IV)
HNR¹⁹R²⁰ (IV),
in welcher
R¹⁹ und R²⁰ die oben angegebenen Bedeutung haben, umsetzt,
und gegebenenfalls sowohl den Substituenten D als auch die Substituenten E, G, M und L auf jeder Stufe, vorzugsweise über die Säurestufe (R³ = COOH) nach üblicher Methode wie beispielsweise Reduktion, Halogenierung, Dehydrohalogenierung, Oxidation, Alkylierung oder Hydrolyse einführt oder in andere funktionelle Gruppen überführt,
gegebenenfalls die Isomeren trennt, und im Fall der Herstellung der Salze mit einer entsprechenden Base oder Säure umsetzt.

3. Arzneimittel enthaltend mindestens ein heterocyclisch substituiertes Phenylessigsäurederivat nach Anspruch 1.

4. Verfahren zur Herstellung von Arzneimitteln nach Anspruch 5, **dadurch gekennzeichnet**, daß man die heterocyclisch substituierten Phenylessigsäurederivate gegebenenfalls mit Hilfe von üblichen Hilfs- und Trägerstoffen in eine geeignete Applikationsform bringt.

5. Verwendung von heterocyclisch substituierten Phenylessigsäurederivaten nach Anspruch 1 zur Herstellung von Arzneimitteln.

6. Verwendung von heterocyclisch substituierten Phenylessigsäurederivaten nach Anspruch 1 zur Herstellung von Arzneimitteln zur Behandlung von artieller Hypertonie, Atherosklerose, coronaren Herzerkrankungen, Herzinsuffizienz, Störungen der Himleistung, ischämischen Gehimerkrankungen peripheren Durchblutungsstörungen, Funktionsstörungen der Niere und nebennierebronchospastisch und vaskulär bedingten Erkrankungen der Atemwege, Natriumretention und Ödemen.

7. Heterocyclisch substituierte Phenylessigsäurederivate nach Anspruch 1 zur Behandlung von Krankheiten.
